# EUROPEAN PATENT SPECIFICATION

(11) **EP 1 232 260 B1**
(45) Date of publication and mention of the grant of the patent: **19.09.2007**
(21) Application number: 00972654.8
(22) Date of filing: 28.07.2000
(51) Int. Cl.: C12N 15/12, C07K 14/705, C12N 5/10, C07K 16/28, C12N 15/00, A01K 67/027, C12N 15/11

(54) **POLYMORPHISM IN THE HUMAN MDR-1 GENE AND APPLICATIONS THEREOF**
POLYMORPHISMUS IM HUMANEN MDR-1 GEN UND DESSEN VERWENDUNG
POLYMORPHISME DU GENE MDR-1 HUMAIN ET SON UTILISATION

(30) Priority: 30.07.1999 EP 99114938; 22.02.2000 EP 00103361
(43) Date of publication of application: 21.08.2002
(73) Proprietor: EPIDAUROS AG Biotechnologie Aktiengesellschaft, 82347 Bernried (DE)
(72) Inventor: BRINKMANN, Ulrich, 82347 Bernried (DE); HOFFMEYER, Sven, 82390 Eberfing (DE); EICHELBAUM, Michel, 71711 Morr (DE); ROOTS, Ivar, 10117 Berlin (DE)
(74) Representative: Vossius & Partner
(86) International application number: PCT/EP2000/007314
(87) International publication number: WO 2001/009183

(56) References cited:
- US-A- 5 206 352
- US-A- 5 399 483
- US-A- 5 849 998
- US-A- 5 928 637
- MICKLEY LA, LEE JS, WENG Z, ZHAN Z, ALVAREZ M, WILSON W, BATES SE, FOJO T: "Genetic polymorphism in MDR-1: a tool for examining allelic expression in normal cells, unselected and drug-selected cell lines, and human tumors." BLOOD, vol. 91, no. 5, 1 March 1998 (1998-03-01), pages 1749-1756, XP000874392 cited in the application
- SHOSHANI T, ZHANG S, DEY S, PASTAN I, GOTTESMAN MM.: "Analysis of random recombinationbetwenn human MDR1 and mouse Mdr1a cDNA ina pHaMDR-dihydrofolate reductase bicistronic expression system" MOL PHARMACOL 1998 OCT;54(4):623-30, XP001032582
- GÜREL S, YERCI O, FILIZ G, DOLAR E, YILMAZLAR T, NAK SG, GULTEN M, ZORLUOGLU A, MEMIK F.: "High expression of multidrug resistance-1 (MDR-1) and its relationship with multiple prognostic factors in gastric carcinomas in patients in Turkey." J INT MED RES 1999 MAR-APR;27(2):79-84, XP001030014
- HAFKEMEYER P, DEY S, AMBUDKAR SV, HRYCYNA CA, PASTAN I, GOTTESMAN MM.: "Contribution to substrate specificity and transport of nonconserved residues in transmembrane domain 12 of human P-glycoprotein" BIOCHEMISTRY 1998 NOV 17;37(46):16400-9, XP001032503
- CHEN,G. ET AL.: "Multidrug-resistant human sarcoma cells with a mutant P-glycoprotein, altered phenotype, and resistance to cyclosporins" J. BIOL. CHEM, vol. 272, no. 9, 1997, pages 5974-5982, XP002130474
- KIM RB, FROMM MF, WANDEL C, LEAKE B, WOOD AJ, RODEN DM, WILKINSON GR.: "The drug transporter P-glycoprotein limits oral absorption and brain entry of HIV-1 protease inhibitors." J CLIN INVEST. 1998 JAN 15;101(2):289-94., XP002114521
- CHEN C.J., CLARK D., UEDA K., PASTAN I., GOTTESMAN M.M., RONINSON I.B.: "Genomic organization of the human multidrug resistance (MDR1) gene and origin of P-glycoproteins" J. BIOL. CHEM. 265(1):506-514(1990)., XP000857468
- HOFFMEYER S ET AL: "FUNCTIONAL POLYMORPHISMS OF THE HUMAN MULTIDRUG-RESISTANCE GENE: MULTIPLE SEQUENCE VARIATIONS AND CORRELATION OF ONE ALLELE WITH P-GLYCOPROTEIN EXPRESSION AND ACTIVITY IN VIVO" PROCEEDINGS OF THE NATIONAL ACADEMY OF SCIENCES OF USA, NATIONAL ACADEMY OF SCIENCE. WASHINGTON, US, vol. 97, no. 7, 28 March 2000 (2000-03-28), pages 3473-3478, XP000996164 ISSN: 0027-8424
- CASCORBI I, GERLOFF T, JOHNE A, MEISEL C, HOFFMEYER S, SCHWAB M, SCHAEFFELER E, EICHELBAUM M, BRINKMANN U, ROOTS I.: "Frequency of single nucleotide polymorphisms in the P-glycoprotein drug transporter MDR1 gene in white subjects." CLIN PHARMACOL THER. 2001 MAR;69(3):169-74., XP001030016
- KUBBA V ET AL: "Single nucleotide polymorphism in MDR1 gene among patients with acute leukemia: correlation of drug induced apoptosis to MDR1 genotype" BLOOD, W.B.SAUNDERS COMPAGNY, ORLANDO, FL, US, vol. 96, no. 11 Part 2, 16 November 2000 (2000-11-16), page 180b XP002175220 ISSN: 0006-4971
- AMEYAW M-M ET AL: "MDR1 PHARMACOGENETICS: FREQUENCY OF THE C3435T MUTATION IN EXON 26 IS SIGNIFICANTLY INFLUENCED BY ETHNICITY" PHARMACOGENETICS, CHAPMAN & HALL, LONDON, GB, vol. 11, no. 3, April 2001 (2001-04), pages 217-221, XP001033432 ISSN: 0960-314X

### SEQUENCE LISTING

<110> EPIDAUROS Biotechnologie Aktiengesellschaft
<120> Polymorphisms in the human MDR-1 gene and their use in diagnostic and therapeutic applications
<130> D 1875 PCT
<140>
   <141>
<160> 376
<170> Patent In Ver. 2.1
<210> 1
   <211> 20
   <212> DNA
   <213> Artificial Sequence
<220>
   <223> Description of Artificial Sequence: synthetic
<400> 1
   cccttaacta cgtcctgtag 20
<210> 2
   <211> 20
   <212> DNA
   <213> Artificial Sequence
<220>
   <223> Description of Artificial Sequence: synthetic
<400> 2
   gaggacttca cactatccac 20
<210> 3
   <211> 21
   <212> DNA
   <213> Artificial Sequence
<220>
   <223> Description of Artificial Sequence: synthetic
<400> 3
   tcttactgct ctctgggctt c 21
<210> 4
   <211> 20
   <212> DNA
   <213> Artificial Sequence
<220>
   <223> Description of Artificial Sequence: synthetic
<400> 4
   ctcagccaac aaacttctgc 20
<210> 5
   <211> 20
   <212> DNA
   <213> Artificial Sequence
<220>
   <223> Description of Artificial Sequence: synthetic
<400> 5
   cactcagtga taaccacgta 20
<210> 6
   <211> 20
   <212> DNA
   <213> Artificial Sequence
<220>
   <223> Description of Artificial Sequence: synthetic
<400> 6
   gcatctccat taacataccc 20
<210> 7
   <211> 20
   <212> DNA
   <213> Artificial Sequence
<220>
   <223> Description of Artificial Sequence: synthetic
<400> 7
   gggtgtcttg gactaggttg 20
<210> 8
   <211> 20
   <212> DNA
   <213> Artificial Sequence
<220>
   <223> Description of Artificial Sequence: synthetic
<400> 8
   tgcctcctac aggactaaac 20
<210> 9
   <211> 20
   <212> DNA
   <213> Artificial Sequence
<220>
   <223> Description of Artificial Sequence: synthetic
<400> 9
   cacacagtca gcagagaagt 20
<210> 10
   <211> 22
   <212> DNA
   <213> Artificial Sequence
<220>
   <223> Description of Artificial Sequence: synthetic
<400> 10
   actatcaaga gtattgttct cc 22
<210> 11
   <211> 20
   <212> DNA
   <213> Artificial Sequence
<220>
   <223> Description of Artificial Sequence: synthetic
<400> 11
   ggaatgagtg gtctctttgg 20
<210> 12
   <211> 20
   <212> DNA
   <213> Artificial Sequence
<220>
   <223> Description of Artificial Sequence: synthetic
<400> 12
   aaggcactgg gaacaaaagg 20
<210> 13
   <211> 21
   <212> DNA
   <213> Artificial Sequence
<220>
   <223> Description of Artificial Sequence: synthetic
<400> 13
   tcctagtaga aacttctacc c 21
<210> 14
   <211> 19
   <212> DNA
   <213> Artificial Sequence
<220>
   <223> Description of Artificial Sequence: synthetic
<400> 14
   ttccgtaggg tgagagcag 19
<210> 15
   <211> 21
   <212> DNA
   <213> Artificial Sequence
<220>
   <223> Description of Artificial Sequence: synthetic
<400> 15
   cagattttgc tctacacatg c 21
<210> 16
   <211> 23
   <212> DNA
   <213> Artificial Sequence
<220>
   <223> Description of Artificial Sequence: synthetic
<400> 16
   attagttatg ctgtaataca tcc 23
<210> 17
   <211> 22
   <212> DNA
   <213> Artificial Sequence
<220>
   <223> Description of Artificial Sequence: synthetic
<400> 17
   catgtatatc acaggactga ac 22
<210> 18
   <211> 22
   <212> DNA
   <213> Artificial Sequence
<220>
   <223> Description of Artificial Sequence: synthetic
<400> 18
   ctagtagtgc atatgtctgt ag 22
<210> 19
   <211> 21
   <212> DNA
   <213> Artificial Sequence
<220>
   <223> Description of Artificial Sequence: synthetic
<400> 19
   gtgacagaat gagaacctgt c 21
<210> 20
   <211> 21
   <212> DNA
   <213> Artificial Sequence
<220>
   <223> Description of Artificial Sequence: synthetic
<400> 20
   tggagagctg gataaagtga c 21
<210> 21
   <211> 23
   <212> DNA
   <213> Artificial Sequence
<220>
   <223> Description of Artificial Sequence: synthetic
<400> 21
   aaattgatct gttagaagcc aag 23
<210> 22
   <211> 23
   <212> DNA
   <213> Artificial Sequence
<220>
   <223> Description of Artificial Sequence: synthetic
<400> 22
   actaggttta aatatacatg cac 23
<210> 23
   <211> 21
   <212> DNA
   <213> Artificial Sequence
<220>
   <223> Description of Artificial Sequence: synthetic
<400> 23
   gaacagtcag ttcctatatc c 21
<210> 24
   <211> 21
   <212> DNA
   <213> Artificial Sequence
<220>
   <223> Description of Artificial Sequence: synthetic
<400> 24
   gggcaacatc agaaagatgt g 21
<210> 25
   <211> 21
   <212> DNA
   <213> Artificial Sequence
<220>
   <223> Description of Artificial Sequence: synthetic
<400> 25
   cacatctttc tgatgttgcc c 21
<210> 26
   <211> 19
   <212> DNA
   <213> Artificial Sequence
<220>
   <223> Description of Artificial Sequence: synthetic
<400> 26
   aagggcaaag ggcaaggac 19
<210> 27
   <211> 21
   <212> DNA
   <213> Artificial Sequence
<220>
   <223> Description of Artificial Sequence: synthetic
<400> 27
   tgggttttct gtggtagaaa t 21
<210> 28
   <211> 20
   <212> DNA
   <213> Artificial Sequence
<220>
   <223> Description of Artificial Sequence: synthetic
<400> 28
   gttggtttga actaagcctc 20
<210> 29
   <211> 21
   <212> DNA
   <213> Artificial Sequence
<220>
   <223> Description of Artificial Sequence: synthetic
<400> 29
   aaatttctct ctctttaggc c 21
<210> 30
   <211> 22
   <212> DNA
   <213> Artificial Sequence
<220>
   <223> Description of Artificial Sequence: synthetic
<400> 30
   ttctgaagtt aaactatacc tg 22
<210> 31
   <211> 23
   <212> DNA
   <213> Artificial Sequence
<220>
   <223> Description of Artificial Sequence: synthetic
<400> 31
   ttcttattta ttttagacag cag 23
<210> 32
   <211> 19
   <212> DNA
   <213> Artificial Sequence
<220>
   <223> Description of Artificial Sequence: synthetic
<400> 32
   gcatctccct tcataccag 19
<210> 33
   <211> 23
   <212> DNA
   <213> Artificial Sequence
<220>
   <223> Description of Artificial Sequence: synthetic
<400> 33
   cattgataag gaataaggat agg 23
<210> 34
   <211> 20
   <212> DNA
   <213> Artificial Sequence
<220>
   <223> Description of Artificial Sequence: synthetic
<400> 34
   cctatcacaa accagactgc 20
<210> 35
   <211> 21
   <212> DNA
   <213> Artificial Sequence
<220>
   <223> Description of Artificial Sequence: synthetic
<400> 35
   atttccagcg tactaaggct c 21
<210> 36
   <211> 21
   <212> DNA
   <213> Artificial Sequence
<220>
   <223> Description of Artificial Sequence: synthetic
<400> 36
   acttggctgt tagtagccat g 21
<210> 37
   <211> 21
   <212> DNA
   <213> Artificial Sequence
<220>
   <223> Description of Artificial Sequence: synthetic
<400> 37
   gtcacagaaa catagcaagc c 21
<210> 38
   <211> 21
   <212> DNA
   <213> Artificial Sequence
<220>
   <223> Description of Artificial Sequence: synthetic
<400> 38
   caaccaatat aggagctagg c 21
<210> 39
   <211> 22
   <212> DNA
   <213> Artificial Sequence
<220>
   <223> Description of Artificial Sequence: synthetic
<400> 39
   acatcctgag tactaaatgc ag 22
<210> 40
   <211> 23
   <212> DNA
   <213> Artificial Sequence
<220>
   <223> Description of Artificial Sequence: synthetic
<400> 40
   aaagcatgtg atatattcgt agg 23
<210> 41
   <211> 24
   <212> DNA
   <213> Artificial Sequence
<220>
   <223> Description of Artificial Sequence: synthetic
<400> 41
   tttctctaat ttgttttgtt ttgc 24
<210> 42
   <211> 21
   <212> DNA
   <213> Artificial Sequence
<220>
   <223> Description of Artificial Sequence: synthetic
<400> 42
   tttagtttga ctcaccttcc c 21
<210> 43
   <211> 21
   <212> DNA
   <213> Artificial Sequence
<220>
   <223> Description of Artificial Sequence: synthetic
<400> 43
   ccttccagat ccataactca g 21
<210> 44
   <211> 22
   <212> DNA
   <213> Artificial Sequence
<220>
   <223> Description of Artificial Sequence: synthetic
<400> 44
   taccttctag ccaaagtaat cc 22
<210> 45
   <211> 22
   <212> DNA
   <213> Artificial Sequence
<220>
   <223> Description of Artificial Sequence: synthetic
<400> 45
   aagtgtaaag tgaggaccaa ac 22
<210> 46
   <211> 21
   <212> DNA
   <213> Artificial Sequence
<220>
   <223> Description of Artificial Sequence: synthetic
<400> 46
   ttcatgaggc ttcacagtag g 21
<210> 47
   <211> 22
   <212> DNA
   <213> Artificial Sequence
<220>
   <223> Description of Artificial Sequence: synthetic
<400> 47
   tcagctttct agcattgtga tg 22
<210> 48
   <211> 23
   <212> DNA
   <213> Artificial Sequence
<220>
   <223> Description of Artificial Sequence: synthetic
<400> 48
   ttgaaaggaa tctatgatct agg 23
<210> 49
   <211> 21
   <212> DNA
   <213> Artificial Sequence
<220>
   <223> Description of Artificial Sequence: synthetic
<400> 49
   ttcttctcat tgcagaacac a 21
<210> 50
   <211> 20
   <212> DNA
   <213> Artificial Sequence
<220>
   <223> Description of Artificial Sequence: synthetic
<400> 50
   tgtgaaagtg tgctcaccac 20
<210> 51
   <211> 22
   <212> DNA
   <213> Artificial Sequence
<220>
   <223> Description of Artificial Sequence: synthetic
<400> 51
   gatctgtgaa ctcttgtttt ca 22
<210> 52
   <211> 21
   <212> DNA
   <213> Artificial Sequence
<220>
   <223> Description of Artificial Sequence: synthetic
<400> 52
   gaagagagac ttacattagg c 21
<210> 53
   <211> 22
   <212> DNA
   <213> Artificial Sequence
<220>
   <223> Description of Artificial Sequence: synthetic
<400> 53
   catgaaccat tcttagcttc tg 22
<210> 54
   <211> 22
   <212> DNA
   <213> Artificial Sequence
<220>
   <223> Description of Artificial Sequence: synthetic
<400> 54
   ttgtgtcatc tttactctcc tg 22
<210> 55
   <211> 23
   <212> DNA
   <213> Artificial Sequence
<220>
   <223> Description of Artificial Sequence: synthetic
<400> 55
   atgtgattat ggaataggtt gtc 23
<210> 56
   <211> 23
   <212> DNA
   <213> Artificial Sequence
<220>
   <223> Description of Artificial Sequence: synthetic
<400> 56
   tatttaacat ctcatacagt cag 23
<210> 57
   <211> 21
   <212> DNA
   <213> Artificial Sequence
<220>
   <223> Description of Artificial Sequence: synthetic
<400> 57
   ccaaggactg ttgaaagtag c 21
<210> 58
   <211> 21
   <212> DNA
   <213> Artificial Sequence
<220>
   <223> Description of Artificial Sequence: synthetic
<400> 58
   ttgcatatgc aagtgtacag c 21
<210> 59
   <211> 20
   <212> DNA
   <213> Artificial Sequence
<220>
   <223> Description of Artificial Sequence: synthetic
<400> 59
   cacagggttg ttgttaagcc 20
<210> 60
   <211> 21
   <212> DNA
   <213> Artificial Sequence
<220>
   <223> Description of Artificial Sequence: synthetic
<400> 60
   tctgaggatg tttccacttt c 21
<210> 61
   <211> 23
   <212> DNA
   <213> Artificial Sequence
<220>
   <223> Description of Artificial Sequence: synthetic
<400> 61
   ttatggcttt gaagtatgag tta 23
<210> 62
   <211> 21
   <212> DNA
   <213> Artificial Sequence
<220>
   <223> Description of Artificial Sequence: synthetic
<400> 62
   gcatgcttga cagtttctga g 21
<210> 63
   <211> 21
   <212> DNA
   <213> Artificial Sequence
<220>
   <223> Description of Artificial Sequence: synthetic
<400> 63
   ctcagaaact gtcaagcatg c 21
<210> 64
   <211> 19
   <212> DNA
   <213> Artificial Sequence
<220>
   <223> Description of Artificial Sequence: synthetic
<400> 64
   ttggaacggc caccaagac 19
<210> 65
   <211> 19
   <212> DNA
   <213> Artificial Sequence
<220>
   <223> Description of Artificial Sequence: synthetic
<400> 65
   cccttctaac catggccag 19
<210> 66
   <211> 19
   <212> DNA
   <213> Artificial Sequence
<220>
   <223> Description of Artificial Sequence: synthetic
<400> 66
   gtgcctcctg tcaatggtg 19
<210> 67
   <211> 19
   <212> DNA
   <213> Artificial Sequence
<220>
   <223> Description of Artificial Sequence: synthetic
<400> 67
   ctactgaaac cgcagcatg 19
<210> 68
   <211> 19
   <212> DNA
   <213> Artificial Sequence
<220>
   <223> Description of Artificial Sequence: synthetic
<400> 68
   ttggagacag tgactcacc 19
<210> 69
   <211> 21
   <212> DNA
   <213> Artificial Sequence
<220>
   <223> Description of Artificial Sequence: synthetic
<400> 69
   gctcattcga gtagcggctc t 21
<210> 70
   <211> 21
   <212> DNA
   <213> Artificial Sequence
<220>
   <223> Description of Artificial Sequence: synthetic
<220>
   <223> y=t or c
<400> 70
   ctcattcgag yagcggctct t 21
<210> 71
   <211> 20
   <212> DNA
   <213> Artificial Sequence
<220>
   <223> Description of Artificial Sequence: synthetic
<400> 71
   agagccgcta ctcgaatgag 20
<210> 72
   <211> 20
   <212> DNA
   <213> Artificial Sequence
<220>
   <223> Description of Artificial Sequence: synthetic
<220>
   <223> r=a or g
<400> 72
   agagccgctr ctcgaatgag 20
<210> 73
   <211> 21
   <212> DNA
   <213> Artificial Sequence
<220>
   <223> Description of Artificial Sequence: synthetic
<400> 73
   ctcattcgag cagcggctct t 21
<210> 74
   <211> 20
   <212> DNA
   <213> Artificial Sequence
<220>
   <223> Description of Artificial Sequence: synthetic
<400> 74
   agagccgctg ctcgaatgag 20
<210> 75
   <211> 23
   <212> DNA
   <213> Artificial Sequence
<220>
   <223> Description of Artificial Sequence: synthetic
<400> 75
   cttcaggtcg ggatggatct tga 23
<210> 76
   <211> 23
   <212> DNA
   <213> Artificial Sequence
<220>
   <223> Description of Artificial Sequence: synthetic
<220>
   <223> r=a or g
<400> 76
   cttcaggtcg gratggatct tga 23
<210> 77
   <211> 20
   <212> DNA
   <213> Artificial Sequence
<220>
   <223> Description of Artificial Sequence: synthetic
<400> 77
   caagatccat cccgacctga 20
<210> 78
   <211> 20
   <212> DNA
   <213> Artificial Sequence
<220>
   <223> Description of Artificial Sequence: synthetic
<220>
   <223> y=c or t
<400> 78
   caagatccat yccgacctga 20
<210> 79
   <211> 23
   <212> DNA
   <213> Artificial Sequence
<220>
   <223> Description of Artificial Sequence: synthetic
<400> 79
   cttcaggtcg gaatggatct tga 23
<210> 80
   <211> 20
   <212> DNA
   <213> Artificial Sequence
<220>
   <223> Description of Artificial Sequence: synthetic
<400> 80
   caagatccat tccgacctga 20
<210> 81
   <211> 20
   <212> DNA
   <213> Artificial Sequence
<220>
   <223> Description of Artificial Sequence: synthetic
<400> 81
   aaactgaaca ataaaaggta 20
<210> 82
   <211> 20
   <212> DNA
   <213> Artificial Sequence
<220>
   <223> Description of Artificial Sequence: synthetic
<220>
   <223> r=a or g
<400> 82
   aaactgaacr ataaaaggta 20
<210> 83
   <211> 22
   <212> DNA
   <213> Artificial Sequence
<220>
   <223> Description of Artificial Sequence: synthetic
<400> 83
   taccttttat tgttcagttt aa 22
<210> 84
   <211> 22
   <212> DNA
   <213> Artificial Sequence
<220>
   <223> Description of Artificial Sequence: synthetic
<220>
   <223> y=t or c
<400> 84
   taccttttat ygttcagttt aa 22
<210> 85
   <211> 20
   <212> DNA
   <213> Artificial Sequence
<220>
   <223> Description of Artificial Sequence: synthetic
<220>
   <221> CDS
   <222> (1).. (18)
<400> 85
   aaa ctg aac gat aaa agt gc                                    20
   Lys Leu Asn Asp Lys Ser
     1               5
<210> 86
   <211> 22
   <212> DNA
   <213> Artificial Sequence
<220>
   <223> Description of Artificial Sequence: synthetic
<400> 86
   taccttttat cgttcagttt aa 22
<210> 87
   <211> 22
   <212> DNA
   <213> Artificial Sequence
<220>
   <223> Description of Artificial Sequence: synthetic
<400> 87
   gacataaatg gtatgtttgt tt 22
<210> 88
   <211> 21
   <212> DNA
   <213> Artificial Sequence
<220>
   <223> Description of Artificial Sequence: synthetic
<220>
   <223> k=g or t
<400> 88
   agacataaat gktatgtttg t 21
<210> 89
   <211> 22
   <212> DNA
   <213> Artificial Sequence
<220>
   <223> Description of Artificial Sequence: synthetic
<400> 89
   aacaaacata ccatttatgt ct 22
<210> 90
   <211> 21
   <212> DNA
   <213> Artificial Sequence
<220>
   <223> Description of Artificial Sequence: synthetic
<220>
   <223> m=a or c
<400> 90
   aacaaacata mcatttatgt c 21
<210> 91
   <211> 21
   <212> DNA
   <213> Artificial Sequence
<220>
   <223> Description of Artificial Sequence: synthetic
<400> 91
   agacataaat gttatgtttg t 21
<210> 92
   <211> 21
   <212> DNA
   <213> Artificial Sequence
<220>
   <223> Description of Artificial Sequence: synthetic
<400> 92
   aacaaacata acatttatgt c 21
<210> 93
   <211> 21
   <212> DNA
   <213> Artificial Sequence
<220>
   <223> Description of Artificial Sequence: synthetic
<400> 93
   gatacagggt tcttcatgaa t 21
<210> 94
   <211> 21
   <212> DNA
   <213> Artificial Sequence
<220>
   <223> Description of Artificial Sequence: synthetic
<220>
   <223> y=c or t
<400> 94
   gatacagggt ycttcatgaa t 21
<210> 95
   <211> 21
   <212> DNA
   <213> Artificial Sequence
<220>
   <223> Description of Artificial Sequence: synthetic
<400> 95
   attcatgaag aaccctgtat c 21
<210> 96
   <211> 21
   <212> DNA
   <213> Artificial Sequence
<220>
   <223> Description of Artificial Sequence: synthetic
<220>
   <223> r=a or g
<400> 96
   attcatgaag raccctgtat c 21
<210> 97
   <211> 21
   <212> DNA
   <213> Artificial Sequence
<220>
   <223> Description of Artificial Sequence: synthetic
<220>
   <221> CDS
   <222> (1)..(21)
<400> 97
   gat aca ggg tcc ttc atg aat                                     21
   Asp Thr Gly Ser Phe Met Asn
     1               5
<210> 98
   <211> 21
   <212> DNA
   <213> Artificial Sequence
<220>
   <223> Description of Artificial Sequence: synthetic
<400> 98
   attcatgaag gaccctgtat c 21
<210> 99
   <211> 23
   <212> DNA
   <213> Artificial Sequence
<220>
   <223> Description of Artificial Sequence: synthetic
<400> 99
   taagcagcaa caatgtcgtg tgc 23
<210> 100
   <211> 23
   <212> DNA
   <213> Artificial Sequence
<220>
   <223> Description of Artificial Sequence: synthetic
<220>
   <223> y=c or t
<400> 100
   taagcagcaa yaatgtcgtg tgc 23
<210> 101
   <211> 19
   <212> DNA
   <213> Artificial Sequence
<220>
   <223> Description of Artificial Sequence: synthetic
<400> 101
   agcagcaata atgtcgtgt 19
<210> 102
   <211> 19
   <212> DNA
   <213> Artificial Sequence
<220>
   <223> Description of Artificial Sequence: synthetic
<400> 102
   ttcacttcag ttacccatc 19
<210> 103
   <211> 18
   <212> DNA
   <213> Artificial Sequence
<220>
   <223> Description of Artificial Sequence: synthetic
<220>
   <223> r=g or a
<400> 103
   tcacttcart tacccatc 18
<210> 104
   <211> 18
   <212> DNA
   <213> Artificial Sequence
<220>
   <223> Description of Artificial Sequence: synthetic
<400> 104
   atgggtaact gaagtgaa 18
<210> 105
   <211> 18
   <212> DNA
   <213> Artificial Sequence
<220>
   <223> Description of Artificial Sequence: synthetic
<220>
   <223> y=c or t
<400> 105
   atgggtaayt gaagtgaa 18
<210> 106
   <211> 18
   <212> DNA
   <213> Artificial Sequence
<220>
   <223> Description of Artificial Sequence: synthetic
<220>
   <221> CDS
   <222> (2)..(16)
<400> 106
   t cac ttc aat tac cca tc                                         18
     His Phe Asn Tyr Pro
       1               5
<210> 107
   <211> 18
   <212> DNA
   <213> Artificial Sequence
<220>
   <223> Description of Artificial Sequence: synthetic
<400> 107
   atgggtaatt gaagtgaa 18
<210> 108
   <211> 20
   <212> DNA
   <213> Artificial Sequence
<220>
   <223> Description of Artificial Sequence: synthetic
<400> 108
   cttgaagggc ctgaacctga 20
<210> 109
   <211> 20
   <212> DNA
   <213> Artificial Sequence
<220>
   <223> Description of Artificial Sequence: synthetic
<220>
   <223> y=c or t
<400> 109
   tcttgaaggg yctgaacctg 20
<210> 110
   <211> 20
   <212> DNA
   <213> Artificial Sequence
<220>
   <223> Description of Artificial Sequence: synthetic
<400> 110
   caggttcagg cccttcaaga 20
<210> 111
   <211> 21
   <212> DNA
   <213> Artificial Sequence
<220>
   <223> Description of Artificial Sequence: synthetic
<220>
   <223> r=g or a
<400> 111
   tcaggttcag rcccttcaag a 21
<210> 112
   <211> 18
   <212> DNA
   <213> Artificial Sequence
<220>
   <223> Description of Artificial Sequence: synthetic
<400> 112
   cttgaagggt ctgaacct 18
<210> 113
   <211> 17
   <212> DNA
   <213> Artificial Sequence
<220>
   <223> Description of Artificial Sequence: synthetic
<400> 113
   ggttcagacc cttcaag 17
<210> 114
   <211> 17
   <212> DNA
   <213> Artificial Sequence
<220>
   <223> Description of Artificial Sequence: synthetic
<400> 114
   tcagcagtca cattgca 17
<210> 115
   <211> 17
   <212> DNA
   <213> Artificial Sequence
<220>
   <223> Description of Artificial Sequence: synthetic
<220>
   <223> y=c or t
<400> 115
   cagcagtyac attgcac 17
<210> 116
   <211> 17
   <212> DNA
   <213> Artificial Sequence
<220>
   <223> Description of Artificial Sequence: synthetic
<400> 116
   cagcagttac attgcac 17
<210> 117
   <211> 20
   <212> DNA
   <213> Artificial Sequence
<220>
   <223> Description of Artificial Sequence: synthetic:
<400> 117
   caaaaattag taaaggaata 20
<210> 118
   <211> 20
   <212> DNA
   <213> Artificial Sequence
<220>
   <223> Description of Artificial Sequence: synthetic
<220>
   <223> w=a or t
<400> 118
   acaaaaattw gtaaaggaat 20,
<210> 119
   <211> 20
   <212> DNA
   <213> Artificial Sequence
<220>
   <223> Description of Artificial Sequence: synthetic
<400> 119
   caaaaatttg taaaggaata 20
<210> 120
   <211> 16
   <212> DNA
   <213> Artificial Sequence
<220>
   <223> Description of Artificial Sequence: synthetic
<400> 120
   gaagagatcg tgaggg 16
<210> 121
   <211> 17
   <212> DNA
   <213> Artificial Sequence
<220>
   <223> Description of Artificial Sequence: synthetic
<220>
   <223> y=c or t
<400> 121
   gaagagatyg tgagggc 17
<210> 122
   <211> 17
   <212> DNA
   <213> Artificial Sequence
<220>
   <223> Description of Artificial Sequence: synthetic
<400> 122
   aagagattgt gagggca 17
<210> 123
   <211> 19
   <212> DNA
   <213> Artificial Sequence
<220>
   <223> Description of Artificial Sequence: synthetic
<400> 123
   ggtttctctt caggtcggg 19
<210> 124
   <211> 20
   <212> DNA
   <213> Artificial Sequence
<220>
   <223> Description of Artificial Sequence: synthetic
<400> 124
   cggtttctct tcaggtcgga 20
<210> 125
   <211> 20
   <212> DNA
   <213> Artificial Sequence
<220>
   <223> Description of Artificial Sequence: synthetic
<400> 125
   ctcagccaac aaacttctgc 20
<210> 126
   <211> 24
   <212> DNA
   <213> Artificial Sequence
<220>
   <223> Description of Artificial Sequence: synthetic
<400> 126
   gaaacaagct agttaccttt tatt 24
<210> 127
   <211> 23
   <212> DNA
   <213> Artificial Sequence
<220>
   <223> Description of Artificial Sequence: synthetic
<400> 127
   aaacaagcta gttacctttt atc 23
<210> 128
   <211> 21
   <212> DNA
   <213> Artificial Sequence
<220>
   <223> Description of Artificial Sequence: synthetic
<400> 128
   tcttactgct ctctgggctt c 21
<210> 129
   <211> 22
   <212> DNA
   <213> Artificial Sequence
<220>
   <223> Description of Artificial Sequence: synthetic
<400> 129
   gaccaccaca aaacaaacat ac 22
<210> 130
   <211> 22
   <212> DNA
   <213> Artificial Sequence
<220>
   <223> Description of Artificial Sequence: synthetic
<400> 130
   gaccaccaca aaacaaacat aa 22
<210> 131
   <211> 20
   <212> DNA
   <213> Artificial Sequence
<220>
   <223> Description of Artificial Sequence: synthetic
<400> 131
   cacacagtca gcagagaagt 20
<210> 132
   <211> 21
   <212> DNA
   <213> Artificial Sequence
<220>
   <223> Description of Artificial Sequence: synthetic
<400> 132
   cttcctccag attcatgaag a 21
<210> 133
   <211> 21
   <212> DNA
   <213> Artificial Sequence
<220>
   <223> Description of Artificial Sequence: synthetic
<400> 133
   cttcctccag attcatgaag g 21
<210> 134
   <211> 20
   <212> DNA
   <213> Artificial Sequence
<220>
   <223> Description of Artificial Sequence: synthetic
<400> 134
   cacacagtca gcagagaagt 20
<210> 135
   <211> 21
   <212> DNA
   <213> Artificial Sequence
<220>
   <223> Description of Artificial Sequence: synthetic
<400> 135
   aaaaggatgc acacgacatt g 21
<210> 136
   <211> 22
   <212> DNA
   <213> Artificial Sequence
<220>
   <223> Description of Artificial Sequence: synthetic
<400> 136
   caaaaggatg cacacgacat ta 22
<210> 137
   <211> 20
   <212> DNA
   <213> Artificial Sequence
<220>
   <223> Description of Artificial Sequence: synthetic
<400> 137
   ggaatgagtg gtctctttgg 20
<210> 138
   <211> 23
   <212> DNA
   <213> Artificial Sequence
<220>
   <223> Description of Artificial Sequence: synthetic
<400> 138
   gaattcagaa atgttcactt cag 23
<210> 139
   <211> 23
   <212> DNA
   <213> Artificial Sequence
<220>
   <223> Description of Artificial Sequence: synthetic
<400> 139
   gaattcagaa atgttcactt caa 23
<210> 140
   <211> 23
   <212> DNA
   <213> Artificial Sequence
<220>
   <223> Description of Artificial Sequence: synthetic
<400> 140
   actaggttta aatatacatg cac 23
<210> 141
   <211> 20
   <212> DNA
   <213> Artificial Sequence
<220>
   <223> Description of Artificial Sequence: synthetic
<400> 141
   cctggtagat cttgaagggc 20
<210> 142
   <211> 20
   <212> DNA
   <213> Artificial Sequence
<220>
   <223> Description of Artificial Sequence: synthetic
<400> 142
   cctggtagat cttgaagggt 20
<210> 143
   <211> 21
   <212> DNA
   <213> Artificial Sequence
<220>
   <223> Description of Artificial Sequence: synthetic
<400> 143
   gggcaacatc agaaagatgt g 21
<210> 144
   <211> 23
   <212> DNA
   <213> Artificial Sequence
<220>
   <223> Description of Artificial Sequence: synthetic
<400> 144
   aggataggat atattccttt act 23
<210> 145
   <211> 23
   <212> DNA
   <213> Artificial Sequence
<220>
   <223> Description of Artificial Sequence: synthetic
<400> 145
   aggataggat atattccttt aca 23
<210> 146
   <211> 19
   <212> DNA
   <213> Artificial Sequence
<220>
   <223> Description of Artificial Sequence: synthetic
<400> 146
   tgggcatcac acttacccc 19
<210> 147
   <211> 19
   <212> DNA
   <213> Artificial Sequence
<220>
   <223> Description of Artificial Sequence: synthetic
<400> 147
   ctcctttgct gccctcacg 19
<210> 148
   <211> 19
   <212> DNA
   <213> Artificial Sequence
<220>
   <223> Description of Artificial Sequence: synthetic
<400> 148
   ctcctttgct gccctcaca 19
<210> 149
   <211> 22
   <212> DNA
   <213> Artificial Sequence
<220>
   <223> Description of Artificial Sequence: synthetic
<400> 149
   gatctgtgaa ctcttgtttt ca 22
<210> 150
   <211> 19
   <212> DNA
   <213> Artificial Sequence
<220>
   <223> Description of Artificial Sequence: synthetic
<400> 150
   gactaaagag acataaatg 19
<210> 151
   <211> 19
   <212> DNA
   <213> Artificial Sequence
<220>
   <223> Description of Artificial Sequence: synthetic
<220>
   <223> s=g or c
<400> 151
   gactaaagas acataaatg 19
<210> 152
   <211> 19
   <212> DNA
   <213> Artificial Sequence
<220>
   <223> Description of Artificial Sequence: synthetic
<400> 152
   catttatgtc tctttagtc 19
<210> 153
   <211> 19
   <212> DNA
   <213> Artificial Sequence
<220>
   <223> Description of Artificial Sequence: synthetic
<220>
   <223> s=c or g
<400> 153
   catttatgts tctttagtc 19
<210> 154
   <211> 19
   <212> DNA
   <213> Artificial Sequence
<220>
   <223> Description of Artificial Sequence: synthetic
<400> 154
   gactaaagac acataaatg 19
<210> 155
   <211> 19
   <212> DNA
   <213> Artificial Sequence
<220>
   <223> Description of Artificial Sequence: synthetic
<400> 155
   catttatgtg tctttagtc 19
<210> 156
   <211> 19
   <212> DNA
   <213> Artificial Sequence
<220>
   <223> Description of Artificial Sequence: synthetic
<400> 156
   atcattaaat gaaatgagt 19
<210> 157
   <211> 19
   <212> DNA
   <213> Artificial Sequence
<220>
   <223> Description of Artificial Sequence: synthetic
<220>
   <223> y=t or c
<400> 157
   atcattaaay gaaatgagt 19
<210> 158
   <211> 19
   <212> DNA
   <213> Artificial Sequence
<220>
   <223> Description of Artificial Sequence: synthetic
<400> 158
   actcatttca tttaatgat 19
<210> 159
   <211> 19
   <212> DNA
   <213> Artificial Sequence
<220>
   <223> Description of Artificial Sequence: synthetic
<220>
   <223> r=a or g
<400> 159
   actcatttcr tttaatgat 19
<210> 160
   <211> 19
   <212> DNA
   <213> Artificial Sequence
<220>
   <223> Description of Artificial Sequence: synthetic
<400> 160
   atcattaaac gaaatgagt 19
<210> 161
   <211> 19
   <212> DNA
   <213> Artificial Sequence
<220>
   <223> Description of Artificial Sequence: synthetic
<400> 161
   actcatttcg tttaatgat 19
<210> 162
   <211> 19
   <212> DNA
   <213> Artificial Sequence
<220>
   <223> Description of Artificial Sequence: synthetic
<400> 162
   caacaatgtc gtgtgcatc 19
<210> 163
   <211> 19
   <212> DNA
   <213> Artificial Sequence
<220>
   <223> Description of Artificial Sequence: synthetic
<220>
   <223> y=c or t
<400> 163
   caacaatgty gtgtgcatc 19
<210> 164
   <211> 19
   <212> DNA
   <213> Artificial Sequence
<220>
   <223> Description of Artificial Sequence: synthetic
<400> 164
   gatgcacacg acattgttg 19
<210> 165
   <211> 19
   <212> DNA
   <213> Artificial Sequence
<220>
   <223> Description of Artificial Sequence: synthetic
<220>
   <223> r=g or a
<400> 165
   gatgcacacr acattgttg 19
<210> 166
   <211> 19
   <212> DNA
   <213> Artificial Sequence
<220>
   <223> Description of Artificial Sequence: synthetic
<400> 166
   caacaatgtt gtgtgcatc 19
<210> 167
   <211> 19
   <212> DNA
   <213> Artificial Sequence
<220>
   <223> Description of Artificial Sequence: synthetic
<400> 167
   gatgcacaca acattgttg 19
<210> 168
   <211> 19
   <212> DNA
   <213> Artificial Sequence
<220>
   <223> Description of Artificial Sequence: synthetic
<400> 168
   ggcttgaaga tgtaagaat 19
<210> 169
   <211> 19
   <212> DNA
   <213> Artificial Sequence
<220>
   <223> Description of Artificial Sequence: synthetic
<220>
   <223> r=a or g
<400> 169
   ggcttgaagr tgtaagaat 19
<210> 170
   <211> 19
   <212> DNA
   <213> Artificial Sequence
<220>
   <223> Description of Artificial Sequence: synthetic
<400> 170
   attcttacat cttcaagcc 19
<210> 171
   <211> 19
   <212> DNA
   <213> Artificial Sequence
<220>
   <223> Description of Artificial Sequence: synthetic
<220>
   <223> y=t or c
<400> 171
   attcttacay cttcaagcc 19
<210> 172
   <211> 19
   <212> DNA
   <213> Artificial Sequence
<220>
   <223> Description of Artificial Sequence: synthetic
<400> 172
   ggcttgaagg tgtaagaat 19
<210> 173
   <211> 19
   <212> DNA
   <213> Artificial Sequence
<220>
   <223> Description of Artificial Sequence: synthetic
<400> 173
   attcttacac cttcaagcc 19
<210> 174
   <211> 19
   <212> DNA
   <213> Artificial Sequence
<220>
   <223> Description of Artificial Sequence: synthetic
<400> 174
   gaacattgcc tatggagac 19
<210> 175
   <211> 19
   <212> DNA
   <213> Artificial Sequence
<220>
   <223> Description of Artificial Sequence: synthetic
<220>
   <223> y=c or t
<400> 175
   gaacattgcy tatggagac 19
<210> 176
   <211> 19
   <212> DNA
   <213> Artificial Sequence
<220>
   <223> Description of Artificial Sequence: synthetic
<400> 176
   gtctccatag gcaatgttc 19
<210> 177
   <211> 19
   <212> DNA
   <213> Artificial Sequence
<220>
   <223> Description of Artificial Sequence: synthetic
<220>
   <223> r=g or a
<400> 177
   gtctccatar gcaatgttc 19
<210> 178
   <211> 19
   <212> DNA
   <213> Artificial Sequence
<220>
   <223> Description of Artificial Sequence: synthetic
<400> 178
   gaacattgct tatggagac 19
<210> 179
   <211> 19
   <212> DNA
   <213> Artificial Sequence
<220>
   <223> Description of Artificial Sequence: synthetic
<400> 179
   gtctccataa gcaatgttc 19
<210> 180
   <211> 19
   <212> DNA
   <213> Artificial Sequence
<220>
   <223> Description of Artificial Sequence: synthetic
<400> 180
   aacttactta tatctttga 19
<210> 181
   <211> 19
   <212> DNA
   <213> Artificial Sequence
<220>
   <223> Description of Artificial Sequence: synthetic
<220>
   <223> r=a or g
<400> 181
   aacttacttr tatctttga 19
<210> 182
   <211> 19
   <212> DNA
   <213> Artificial Sequence
<220>
   <223> Description of Artificial Sequence: synthetic
<400> 182
   tcaaagatat aagtaagtt 19
<210> 183
   <211> 19
   <212> DNA
   <213> Artificial Sequence
<220>
   <223> Description of Artificial Sequence: synthetic
<220>
   <223> y=t or c
<400> 183
   tcaaagatay aagtaagtt 19
<210> 184
   <211> 19
   <212> DNA
   <213> Artificial Sequence
<220>
   <223> Description of Artificial Sequence: synthetic
<400> 184
   aacttacttg tatctttga 19
<210> 185
   <211> 19
   <212> DNA
   <213> Artificial Sequence
<220>
   <223> Description of Artificial Sequence: synthetic
<400> 185
   tcaaagatac aagtaagtt 19
<210> 186
   <211> 19
   <212> DNA
   <213> Artificial Sequence
<220>
   <223> Description of Artificial Sequence: synthetic
<400> 186
   agaaatagta taatcaaca 19
<210> 187
   <211> 19
   <212> DNA
   <213> Artificial Sequence
<220>
   <223> Description of Artificial Sequence: synthetic
<220>
   <223> w=a or t
<400> 187
   agaaatagtw taatcaaca 19
<210> 188
   <211> 19
   <212> DNA
   <213> Artificial Sequence
<220>
   <223> Description of Artificial Sequence: synthetic
<400> 188
   tgttgattat actatttct 19
<210> 189
   <211> 19
   <212> DNA
   <213> Artificial Sequence
<220>
   <223> Description of Artificial Sequence: synthetic
<220>
   <223> w=t or a
<400> 189
   tgttgattaw actatttct 19
<210> 190
   <211> 19
   <212> DNA
   <213> Artificial Sequence
<220>
   <223> Description of Artificial Sequence: synthetic
<400> 190
   agaaatagtt taatcaaca 19
<210> 191
   <211> 19
   <212> DNA
   <213> Artificial Sequence
<220>
   <223> Description of Artificial Sequence: synthetic
<400> 191
   tgttgattaa actatttct 19
<210> 192
   <211> 19
   <212> DNA
   <213> Artificial Sequence
<220>
   <223> Description of Artificial Sequence: synthetic
<400> 192
   tagggagggt ttaaggcca 19
<210> 193
   <211> 19
   <212> DNA
   <213> Artificial Sequence
<220>
   <223> Description of Artificial Sequence: synthetic
<220>
   <223> y=t or c
<400> 193
   tagggagggy ttaaggcca 19
<210> 194
   <211> 19
   <212> DNA
   <213> Artificial Sequence
<220>
   <223> Description of Artificial Sequence: synthetic
<400> 194
   tggccttaaa ccctcccta 19
<210> 195
   <211> 19
   <212> DNA
   <213> Artificial Sequence
<220>
   <223> Description of Artificial Sequence: synthetic
<220>
   <223> r=a or g
<400> 195
   tggccttaar ccctcccta 19
<210> 196
   <211> 19
   <212> DNA
   <213> Artificial Sequence
<220>
   <223> Description of Artificial Sequence: synthetic
<400> 196
   tagggagggc ttaaggcca 19
<210> 197
   <211> 19
   <212> DNA
   <213> Artificial Sequence
<220>
   <223> Description of Artificial Sequence: synthetic
<400> 197
   tggccttaag ccctcccta 19
<210> 198
   <211> 19
   <212> DNA
   <213> Artificial Sequence
<220>
   <223> Description of Artificial Sequence: synthetic
<400> 198
   gaaaggtgag ataaagcaa 19
<210> 199
   <211> 19
   <212> DNA
   <213> Artificial Sequence
<220>
   <223> Description of Artificial Sequence: synthetic
<220>
   <223> r=g or a
<400> 199
   gaaaggtgar ataaagcaa 19
<210> 200
   <211> 19
   <212> DNA
   <213> Artificial Sequence
<220>
   <223> Description of Artificial Sequence: synthetic
<400> 200
   ttgctttatc tcacctttc 19
<210> 201
   <211> 19
   <212> DNA
   <213> Artificial Sequence
<220>
   <223> Description of Artificial Sequence: synthetic
<220>
   <223> y=c or t
<400> 201
   ttgctttaty tcacctttc 19
<210> 202
   <211> 19
   <212> DNA
   <213> Artificial Sequence
<220>
   <223> Description of Artificial Sequence: synthetic
<400> 202
   gaaaggtgaa ataaagcaa 19
<210> 203
   <211> 19
   <212> DNA
   <213> Artificial Sequence
<220>
   <223> Description of Artificial Sequence: synthetic
<400> 203
   ttgctttatt tcacctttc 19
<210> 204
   <211> 19
   <212> DNA
   <213> Artificial Sequence
<220>
   <223> Description of Artificial Sequence: synthetic
<400> 204
   catttacccc agatggacc 19
<210> 205
   <211> 19
   <212> DNA
   <213> Artificial Sequence
<220>
   <223> Description of Artificial Sequence: synthetic
<220>
   <223> y=c or t
<400> 205
   catttacccy agatggacc 19
<210> 206
   <211> 19
   <212> DNA
   <213> Artificial Sequence
<220>
   <223> Description of Artificial Sequence: synthetic
<400> 206
   ggtccatctg gggtaaatg 19
<210> 207
   <211> 19
   <212> DNA
   <213> Artificial Sequence
<220>
   <223> Description of Artificial Sequence: synthetic
<220>
   <223> r=g or a
<400> 207
   ggtccatctr gggtaaatg 19
<210> 208
   <211> 19
   <212> DNA
   <213> Artificial Sequence
<220>
   <223> Description of Artificial Sequence: synthetic
<400> 208
   catttaccct agatggacc 19
<210> 209
   <211> 19
   <212> DNA
   <213> Artificial Sequence
<220>
   <223> Description of Artificial Sequence: synthetic
<400> 209
   ggtccatcta gggtaaatg 19
<210> 210
   <211> 19
   <212> DNA
   <213> Artificial Sequence
<220>
   <223> Description of Artificial Sequence: synthetic
<400> 210
   gaggcgggcg gatcacgag 19
<210> 211
   <211> 19
   <212> DNA
   <213> Artificial Sequence
<220>
   <223> Description of Artificial Sequence: synthetic
<220>
   <223> r=g or a
<400> 211
   gaggcgggcr gatcacgag 19
<210> 212
   <211> 19
   <212> DNA
   <213> Artificial Sequence
<220>
   <223> Description of Artificial Sequence: synthetic
<400> 212
   ctcgtgatcc gcccgcctc 19
<210> 213
   <211> 19
   <212> DNA
   <213> Artificial Sequence
<220>
   <223> Description of Artificial Sequence: synthetic
<220>
   <223> y=c or t
<400> 213
   ctcgtgatcy gcccgcctc 19
<210> 214
   <211> 19
   <212> DNA
   <213> Artificial Sequence
<220>
   <223> Description of Artificial Sequence: synthetic
<400> 214
   gaggcgggca gatcacgag 19
<210> 215
   <211> 19
   <212> DNA
   <213> Artificial Sequence
<220>
   <223> Description of Artificial Sequence: synthetic
<400> 215
   ctcgtgatct gcccgcctc 19
<210> 216
   <211> 19
   <212> DNA
   <213> Artificial Sequence
<220>
   <223> Description of Artificial Sequence: synthetic
<400> 216
   ggagaatggt gtgaacccg 19
<210> 217
   <211> 19
   <212> DNA
   <213> Artificial Sequence
<220>
   <223> Description of Artificial Sequence: synthetic
<220>
   <223> y=t or c
<400> 217
   ggagaatggy gtgaacccg 19
<210> 218
   <211> 19
   <212> DNA
   <213> Artificial Sequence
<220>
   <223> Description of Artificial Sequence: synthetic
<400> 218
   cgggttcaca ccattctcc
<210> 219
   <211> 19
   <212> DNA
   <213> Artificial Sequence
<220>
   <223> Description of Artificial Sequence: synthetic
<220>
   <223> r=a or g
<400> 219
   cgggttcacr ccattctcc
<210> 220
   <211> 19
   <212> DNA
   <213> Artificial Sequence
<220>
   <223> Description of Artificial Sequence: synthetic
<400> 220
   ggagaatggc gtgaacccg
<210> 221
   <211> 19
   <212> DNA
   <213> Artificial Sequence
<220>
   <223> Description of Artificial Sequence: synthetic
<400> 221
   cgggttcacg ccattctcc
<210> 222
   <211> 19
   <212> DNA
   <213> Artificial Sequence
<220>
   <223> Description of Artificial Sequence: synthetic
<400> 222
   atatggaagg aaattacaa
<210> 223
   <211> 19
   <212> DNA
   <213> Artificial Sequence
<220>
   <223> Description of Artificial Sequence: synthetic
<220>
   <223> r=g or a
<400> 223
   atatggaagr aaattacaa 19
<210> 224
   <211> 19
   <212> DNA
   <213> Artificial Sequence
<220>
   <223> Description of Artificial Sequence: synthetic
<400> 224
   ttgtaatttc cttccatat 19
<210> 225
   <211> 19
   <212> DNA
   <213> Artificial Sequence
<220>
   <223> Description of Artificial Sequence: synthetic
<220>
   <223> y=c or t
<400> 225
   ttgtaattty cttccatat 19
<210> 226
   <211> 19
   <212> DNA
   <213> Artificial Sequence
<220>
   <223> Description of Artificial Sequence: synthetic
<400> 226
   atatggaaga aaattacaa 19
<210> 227
   <211> 19
   <212> DNA
   <213> Artificial Sequence
<220>
   <223> Description of Artificial Sequence: synthetic
<400> 227
   ttgtaatttt cttccatat 19
<210> 228
   <211> 19
   <212> DNA
   <213> Artificial Sequence
<220>
   <223> Description of Artificial Sequence: synthetic
<400> 228
   aacacgggca ttgatctga 19
<210> 229
   <211> 19
   <212> DNA
   <213> Artificial Sequence
<220>
   <223> Description of Artificial Sequence: synthetic
<220>
   <223> r=a or g
<400> 229
   aacacgggcr ttgatctga 19
<210> 230
   <211> 19
   <212> DNA
   <213> Artificial Sequence
<220>
   <223> Description of Artificial Sequence: synthetic
<400> 230
   tcagatcaat gcccgtgtt 19
<210> 231
   <211> 19
   <212> DNA
   <213> Artificial Sequence
<220>
   <223> Description of Artificial Sequence: synthetic
<220>
   <223> y=t or c
<400> 231
   tcagatcaay gcccgtgtt 19
<210> 232
   <211> 19
   <212> DNA
   <213> Artificial Sequence
<220>
   <223> Description of Artificial Sequence: synthetic
<400> 232
   aacacgggcg ttgatctga 19
<210> 233
   <211> 19
   <212> DNA
   <213> Artificial Sequence
<220>
   <223> Description of Artificial Sequence: synthetic
<400> 233
   tcagatcaac gcccgtgtt 19
<210> 234
   <211> 19
   <212> DNA
   <213> Artificial Sequence
<220>
   <223> Description of Artificial Sequence: synthetic
<400> 234
   tgtattaaat gcgaatccc 19
<210> 235
   <211> 19
   <212> DNA
   <213> Artificial Sequence
<220>
   <223> Description of Artificial Sequence: synthetic
<220>
   <223> y=t or c
<400> 235
   tgtattaaay gcgaatccc 19
<210> 236
   <211> 19
   <212> DNA
   <213> Artificial Sequence
<220>
   <223> Description of Artificial Sequence: synthetic
<400> 236
   gggattcgca tttaataca 19
<210> 237
   <211> 19
   <212> DNA
   <213> Artificial Sequence
<220>
   <223> Description of Artificial Sequence: synthetic
<220>
   <223> r=a or g
<400> 237
   gggattcgcr tttaataca 19
<210> 238
   <211> 19
   <212> DNA
   <213> Artificial Sequence
<220>
   <223> Description of Artificial Sequence: synthetic
<400> 238
   tgtattaaac gcgaatccc 19
<210> 239
   <211> 19
   <212> DNA
   <213> Artificial Sequence
<220>
   <223> Description of Artificial Sequence: synthetic
<400> 239
   gggattcgcg tttaataca 19
<210> 240
   <211> 19
   <212> DNA
   <213> Artificial Sequence
<220>
   <223> Description of Artificial Sequence: synthetic
<400> 240
   ttgaaagacg tgtctacat 19
<210> 241
   <211> 19
   <212> DNA
   <213> Artificial Sequence
<220>
   <223> Description of Artificial Sequence: synthetic
<220>
   <223> r=g or a
<400> 241
   ttgaaagacr tgtctacat 19
<210> 242
   <211> 19
   <212> DNA
   <213> Artificial Sequence
<220>
   <223> Description of Artificial Sequence: synthetic
<400> 242
   atgtagacac gtctttcaa 19
<210> 243
   <211> 19
   <212> DNA
   <213> Artificial Sequence
<220>
   <223> Description of Artificial Sequence: synthetic
<220>
   <223> y=c or t
<400> 243
   atgtagacay gtctttcaa 19
<210> 244
   <211> 19
   <212> DNA
   <213> Artificial Sequence
<220>
   <223> Description of Artificial Sequence: synthetic
<400> 244
   ttgaaagaca tgtctacat 19
<210> 245
   <211> 19
   <212> DNA
   <213> Artificial Sequence
<220>
   <223> Description of Artificial Sequence: synthetic
<400> 245
   atgtagacat gtctttcaa 19
<210> 246
   <211> 19
   <212> DNA
   <213> Artificial Sequence
<220>
   <223> Description of Artificial Sequence: synthetic
<400> 246
   cgtgtctaca taagttgaa 19
<210> 247
   <211> 19
   <212> DNA
   <213> Artificial Sequence
<220>
   <223> Description of Artificial Sequence: synthetic
<220>
   <223> w=a or t
<400> 247
   cgtgtctacw taagttgaa 19
<210> 248
   <211> 19
   <212> DNA
   <213> Artificial Sequence
<220>
   <223> Description of Artificial Sequence: synthetic
<400> 248
   ttcaacttat gtagacacg 19
<210> 249
   <211> 19
   <212> DNA
   <213> Artificial Sequence
<220>
   <223> Description of Artificial Sequence: synthetic
<220>
   <223> w=t or a
<400> 249
   ttcaacttaw gtagacacg 19
<210> 250
   <211> 19
   <212> DNA
   <213> Artificial Sequence
<220>
   <223> Description of Artificial Sequence: synthetic
<400> 250
   cgtgtctact taagttgaa 19
<210> 251
   <211> 19
   <212> DNA
   <213> Artificial Sequence
<220>
   <223> Description of Artificial Sequence: synthetic
<400> 251
   ttcaacttaa gtagacacg 19
<210> 252
   <211> 19
   <212> DNA
   <213> Artificial Sequence
<220>
   <223> Description of Artificial Sequence: synthetic
<400> 252
   atgtccccaa tgattcagc 19
<210> 253
   <211> 19
   <212> DNA
   <213> Artificial Sequence
<220>
   <223> Description of Artificial Sequence: synthetic
<220>
   <223> r=a or g
<400> 253
   atgtccccar tgattcagc 19
<210> 254
   <211> 19
   <212> DNA
   <213> Artificial Sequence
<220>
   <223> Description of Artificial Sequence: synthetic
<400> 254
   gctgaatcat tggggacat 19
<210> 255
   <211> 19
   <212> DNA
   <213> Artificial Sequence
<220>
   <223> Description of Artificial Sequence: synthetic
<220>
   <223> y=t cr c
<400> 255
   gctgaatcay tggggacat 19
<210> 256
   <211> 19
   <212> DNA
   <213> Artificial Sequence
<220>
   <223> Description of Artificial Sequence: synthetic
<400> 256
   atgtccccag tgattcagc 19
<210> 257
   <211> 19
   <212> DNA
   <213> Artificial Sequence
<220>
   <223> Description of Artificial Sequence: synthetic
<400> 257
   gctgaatcac tggggacat 19
<210> 258
   <211> 19
   <212> DNA
   <213> Artificial Sequence
<220>
   <223> Description of Artificial Sequence: synthetic
<400> 258
   ccgggccggg agcagtcat 19
<210> 259
   <211> 19
   <212> DNA
   <213> Artificial Sequence
<220>
   <223> Description of Artificial Sequence: synthetic
<220>
   <223> r=g or a
<400> 259
   ccgggccggr agcagtcat 19
<210> 260
   <211> 19
   <212> DNA
   <213> Artificial Sequence
<220>
   <223> Description of Artificial Sequence: synthetic
<400> 260
   atgactgctc ccggcccgg 19
<210> 261
   <211> 19
   <212> DNA
   <213> Artificial Sequence
<220>
   <223> Description of Artificial Sequence: synthetic
<220>
   <223> y=c or t
<400> 261
   atgactgcty ccggcccgg 19
<210> 262
   <211> 19
   <212> DNA
   <213> Artificial Sequence
<220>
   <223> Description of Artificial Sequence: synthetic
<400> 262
   ccgggccgga agcagtcat 19
<210> 263
   <211> 19
   <212> DNA
   <213> Artificial Sequence
<220>
   <223> Description of Artificial Sequence: synthetic
<400> 263
   atgactgctt ccggcccgg 19
<210> 264
   <211> 19
   <212> DNA
   <213> Artificial Sequence
<220>
   <223> Description of Artificial Sequence: synthetic
<400> 264
   aaaatacttc ggaaatttg 19
<210> 265
   <211> 19
   <212> DNA
   <213> Artificial Sequence
<220>
   <223> Description of Artificial Sequence: synthetic
<220>
   <223> y=c or t
<400> 265
   aaaatactty ggaaatttg 19
<210> 266
   <211> 19
   <212> DNA
   <213> Artificial Sequence
<220>
   <223> Description of Artificial Sequence: synthetic
<400> 266
   caaatttccg aagtatttt 19
<210> 267
   <211> 19
   <212> DNA
   <213> Artificial Sequence
<220>
   <223> Description of Artificial Sequence: synthetic
<220>
   <223> r=g or a
<400> 267
   caaatttccr aagtatttt 19
<210> 268
   <211> 19
   <212> DNA
   <213> Artificial Sequence
<220>
   <223> Description of Artificial Sequence: synthetic
<400> 268
   aaaatacttt ggaaatttg 19
<210> 269
   <211> 19
   <212> DNA
   <213> Artificial Sequence
<220>
   <223> Description of Artificial Sequence: synthetic
<400> 269
   caaatttcca aagtatttt 19
<210> 270
   <211> 19
   <212> DNA
   <213> Artificial Sequence
<220>
   <223> Description of Artificial Sequence: synthetic
<400> 270
   gatacagggt tcttcatga 19
<210> 271
   <211> 19
   <212> DNA
   <213> Artificial Sequence
<220>
   <223> Description of Artificial Sequence: synthetic
<220>
   <223> y=t or c
<400> 271
   gatacagggy tcttcatga 19
<210> 272
   <211> 19
   <212> DNA
   <213> Artificial Sequence
<220>
   <223> Description of Artificial Sequence: synthetic
<400> 272
   tcatgaagaa ccctgtatc 19
<210> 273
   <211> 19
   <212> DNA
   <213> Artificial Sequence
<220>
   <223> Description of Artificial Sequence: synthetic
<220>
   <223> r=a or g
<400> 273
   tcatgaagar ccctgtatc 19
<210> 274
   <211> 19
   <212> DNA
   <213> Artificial Sequence: Synthetic DNA
<220>
   <221> CDS
   <222> (1)..(18)
<400> 274
   gat aca ggg ctc ttc atg a                                    19
   Asp Thr Gly Leu Phe Met
     1               5
<210> 275
   <211> 19
   <212> DNA
   <213> Artificial Sequence
<220>
   <223> Description of Artificial Sequence: synthetic
<400> 275
   tcatgaagag ccctgtatc 19
<210> 276
   <211> 19
   <212> DNA
   <213> Artificial Sequence
<220>
   <223> Description of Artificial Sequence: synthetic
<400> 276
   gtgcacgatg ttggggagc 19
<210> 277
   <211> 19
   <212> DNA
   <213> Artificial Sequence
<220>
   <223> Description of Artificial Sequence: synthetic
<220>
   <223> r=g or a
<400> 277
   gtgcacgatr ttggggagc 19
<210> 278
   <211> 19
   <212> DNA
   <213> Artificial Sequence
<220>
   <223> Description of Artificial Sequence: synthetic
<400> 278
   gctccccaac atcgtgcac 19
<210> 279
   <211> 19
   <212> DNA
   <213> Artificial Sequence
<220>
   <223> Description of Artificial Sequence: synthetic
<220>
   <223> y=c or t
<400> 279
   gctccccaay atcgtgcac 19
<210> 280
   <211> 19
   <212> DNA
   <213> Artificial Sequence
<220>
   <223> Description of Artificial Sequence: synthetic
<400> 280
   gtgcacgata ttggggagc 19
<210> 281
   <211> 19
   <212> DNA
   <213> Artificial Sequence
<220>
   <223> Description of Artificial Sequence: synthetic
<400> 281
   gctccccaat atcgtgcac 19
<210> 282
   <211> 19
   <212> DNA
   <213> Artificial Sequence
<220>
   <223> Description of Artificial Sequence: synthetic
<400> 282
   cattaaatga aggactggg 19
<210> 283
   <211> 19
   <212> DNA
   <213> Artificial Sequence
<220>
   <223> Description of Artificial Sequence: synthetic
<220>
   <223> r=a or g
<400> 283
   cattaaatgr aggactggg 19
<210> 284
   <211> 19
   <212> DNA
   <213> Artificial Sequence
<220>
   <223> Description of Artificial Sequence: synthetic
<400> 284
   cccagtcctt catttaatg 19
<210> 285
   <211> 19
   <212> DNA
   <213> Artificial Sequence
<220>
   <223> Description of Artificial Sequence: synthetic
<220>
   <223> y=t or c
<400> 285
   cccagtccty catttaatg 19
<210> 286
   <211> 19
   <212> DNA
   <213> Artificial Sequence
<220>
   <223> Description of Artificial Sequence: synthetic
<400> 286
   cattaaatgg aggactggg 19
<210> 287
   <211> 19
   <212> DNA
   <213> Artificial Sequence
<220>
   <223> Description of Artificial Sequence: synthetic
<400> 287
   cccagtcctc catttaatg 19
<210> 288
   <211> 19
   <212> DNA
   <213> Artificial Sequence
<220>
   <223> Description of Artificial Sequence: synthetic
<400> 288
   tcctctgaga atgtgcagt 19
<210> 289
   <211> 19
   <212> DNA
   <213> Artificial Sequence
<220>
   <223> Description of Artificial Sequence: synthetic
<220>
   <223> r=a or g
<400> 289
   tcctctgagr atgtgcagt 19
<210> 290
   <211> 19
   <212> DNA
   <213> Artificial Sequence
<220>
   <223> Description of Artificial Sequence: synthetic
<400> 290
   actgcacatt ctcagagga 19
<210> 291
   <211> 19
   <212> DNA
   <213> Artificial Sequence
<220>
   <223> Description of Artificial Sequence: synthetic
<220>
   <223> y=t or c
<400> 291
   actgcacaty ctcagagga 19
<210> 292
   <211> 19
   <212> DNA
   <213> Artificial Sequence
<220>
   <223> Description of Artificial Sequence: synthetic
<400> 292
   tcctctgagg atgtgcagt 19
<210> 293
   <211> 19
   <212> DNA
   <213> Artificial Sequence
<220>
   <223> Description of Artificial Sequence: synthetic
<400> 293
   actgcacatc ctcagagga 19
<210> 294
   <211> 19
   <212> DNA
   <213> Artificial Sequence
<220>
   <223> Description of Artificial Sequence: synthetic
<400> 294
   aaaattgctg tcactatct 19
<210> 295
   <211> 19
   <212> DNA
   <213> Artificial Sequence
<220>
   <223> Description of Artificial Sequence: synthetic
<220>
   <223> r=g or a
<400> 295
   aaaattgctr tcactatct 19
<210> 296
   <211> 19
   <212> DNA
   <213> Artificial Sequence
<220>
   <223> Description of Artificial Sequence: synthetic
<400> 296
   agatagtgac agcaatttt 19
<210> 297
   <211> 19
   <212> DNA
   <213> Artificial Sequence
<220>
   <223> Description of Artificial Sequence: synthetic
<220>
   <223> y=c or t
<400> 297
   agatagtgay agcaatttt 19
<210> 298
   <211> 19
   <212> DNA
   <213> Artificial Sequence
<220>
   <223> Description of Artificial Sequence: synthetic
<400> 298
   aaaattgcta tcactatct 19
<210> 299
   <211> 19
   <212> DNA
   <213> Artificial Sequence
<220>
   <223> Description of Artificial Sequence: synthetic
<400> 299
   agatagtgat agcaatttt 19
<210> 300
   <211> 19
   <212> DNA
   <213> Artificial Sequence
<220>
   <223> Description of Artificial Sequence: synthetic
<400> 300
   gagcacaaca gtccagctg 19
<210> 301
   <211> 19
   <212> DNA
   <213> Artificial Sequence
<220>
   <223> Description of Artificial Sequence: synthetic
<220>
   <223> r=a or g
<400> 301
   gagcacaacr gtccagctg 19
<210> 302
   <211> 19
   <212> DNA
   <213> Artificial Sequence
<220>
   <223> Description of Artificial Sequence: synthetic
<400> 302
   cagctggact gttgtgctc 19
<210> 303
   <211> 19
   <212> DNA
   <213> Artificial Sequence
<220>
   <223> Description of Artificial Sequence: synthetic
<220>
   <223> y=t or c
<400> 303
   cagctggacy gttgtgctc 19
<210> 304
   <211> 19
   <212> DNA
   <213> Artificial Sequence
<220>
   <223> Description of Artificial Sequence: synthetic
<400> 304
   gagcacaacg gtccagctg 19
<210> 305
   <211> 19
   <212> DNA
   <213> Artificial Sequence
<220>
   <223> Description of Artificial Sequence: synthetic
<400> 305
   cagctggacc gttgtgctc 19
<210> 306
   <211> 19
   <212> DNA
   <213> Artificial Sequence
<220>
   <223> Description of Artificial Sequence: synthetic
<400> 306
   tgggcagacg gtggccctg 19
<210> 307
   <211> 19
   <212> DNA
   <213> Artificial Sequence
<220>
   <223> Description of Artificial Sequence: synthetic
<220>
   <223> r=g or a
<400> 307
   tgggcagacr gtggccctg 19
<210> 308
   <211> 19
   <212> DNA
   <213> Artificial Sequence
<220>
   <223> Description of Artificial Sequence: synthetic
<400> 308
   cagggccacc gtctgccca 19
<210> 309
   <211> 19
   <212> DNA
   <213> Artificial Sequence
<220>
   <223> Description of Artificial Sequence: synthetic
<220>
   <223> y=c or t
<400> 309
   cagggccacy gtctgccca 19
<210> 310
   <211> 19
   <212> DNA
   <213> Artificial Sequence
<220>
   <223> Description of Artificial Sequence: synthetic
<400> 310
   tgggcagaca gtggccctg 19
<210> 311
   <211> 19
   <212> DNA
   <213> Artificial Sequence
<220>
   <223> Description of Artificial Sequence: synthetic
<400> 311
   cagggccact gtctgccca 19
<210> 312
   <211> 19
   <212> DNA
   <213> Artificial Sequence
<220>
   <223> Description of Artificial Sequence: synthetic
<400> 312
   ctcgccctgg tagatcttg 19
<210> 313
   <211> 19
   <212> DNA
   <213> Artificial Sequence
<220>
   <223> Description of Artificial Sequence: synthetic
<220>
   <223> r=g or a
<400> 313
   ctcgtcctgr tagatcttg 19
<210> 314
   <211> 19
   <212> DNA
   <213> Artificial Sequence
<220>
   <223> Description of Artificial Sequence: synthetic
<400> 314
   caagatctac caggacgag 19
<210> 315
   <211> 19
   <212> DNA
   <213> Artificial Sequence
<220>
   <223> Description of Artificial Sequence: synthetic
<220>
   <223> y=c or t
<400> 315
   caagatctay caggacgag 19
<210> 316
   <211> 19
   <212> DNA
   <213> Artificial Sequence
<220>
   <223> Description of Artificial Sequence: synthetic
<400> 316
   ctcgtcctga tagatcttg 19
<210> 317
   <211> 19
   <212> DNA
   <213> Artificial Sequence
<220>
   <223> description of Artificial Sequence: synthetic
<400> 317.
   caagatctat caggacgag 19
<210> 318
   <211> 19
   <212> DNA
   <213> Artificial Sequence
<220>
   <223> description of Artificial Sequence: synthetic
<400> 318
   gacctatgcg agctagacc 19
<210> 319
   <211> 19
   <212> DNA
   <213> Artificial Sequence
<220>
   <223> description of Artificial Sequence: synthetic
<22C>
   <223> r=g or a
<400> 319
   gacccatgcr agctagacc 19
<210> 320
   <211> 19
   <212> DNA
   <213> Artificial Sequence
<220>
   <223> Description of Artificial Sequence: synthetic
<400> 320
   ggtctagctc gcatgggtc 19
<210> 321
   <211> 19
   <212> DNA
   <213> Artificial Sequence
<220>
   <223> Description of Artificial Sequence: synthetic
<220>
   <223> y=c or t
<400> 321
   ggtctagcty gcatgggtc 19
<210> 322
   <211> 19
   <212> DNA
   <213> Artificial Sequence
<220>
   <223> Description of Artificial Sequence: synthetic
<400> 322
   gacccatgca agctagacc 19
<210> 323
   <211> 19
   <212> DNA
   <213> Artificial Sequence
<220>
   <223> Description of Artificial Sequence: synthetic
<400> 323
   ggtctagctt gcatgggtc 19
<210> 324
   <211> 19
   <212> DNA
   <213> Artificial Sequence
<220>
   <223> Description of Artificial Sequence: synthetic
<400> 324
   actttgtcta atctcctgc 19
<210> 325
   <211> 19
   <212> DNA
   <213> Artificial Sequence
<220>
   <223> Description of Artificial Sequence: synthetic
<220>
   <223> r=a or g
<400> 325
   actttgtctr atctcctgc 19
<210> 326
   <211> 19
   <212> DNA
   <213> Artificial Sequence
<220>
   <223> Description of Artificial Sequence: synthetic
<400> 326
   gcaggagatt agacaaagt 19
<210> 327
   <211> 19
   <212> DNA
   <213> Artificial Sequence
<220>
   <223> Description of Artificial Sequence: synthetic
<220>
   <223> y=t or c
<400> 327
   gcaggagaty agacaaagt 19
<210> 328
   <211> 19
   <212> DNA
   <213> Artificial Sequence
<220>
   <223> Description of Artificial Sequence: synthetic
<400> 328
   actttgtctg atctcctgc 19
<210> 329
   <211> 19
   <212> DNA
   <213> Artificial Sequence
<220>
   <223> Description of Artificial Sequence: synthetic
<400> 329
   gcaggagatc agacaaagt 19
<210> 330
   <211> 19
   <212> DNA
   <213> Artificial Sequence
<220>
   <223> Description of Artificial Sequence: synthetic
<400> 330
   aatcattttc tgtgccaca 19
<210> 331
   <211> 19
   <212> DNA
   <213> Artificial Sequence
<220>
   <223> Description of Artificial Sequence: synthetic
<220>
   <223> m=c or a
<400> 331
   aatcattttm tgtgccaca 19
<210> 332
   <211> 19
   <212> DNA
   <213> Artificial Sequence
<220>
   <223> Description of Artificial Sequence: synthetic
<400> 332
   tgtggcacag aaaatgatt 19
<210> 333
   <211> 19
   <212> DNA
   <213> Artificial Sequence
<220>
   <223> Description of Artificial Sequence: synthetic
<220>
   <223> k=g or t
<400> 333
   tgtggcacak aaaatgatt 19
<210> 334
   <211> 19
   <212> DNA
   <213> Artificial Sequence
<220>
   <223> Description of Artificial Sequence: synthetic
<400> 334
   aatcatttta tgtgccaca 19
<210> 335
   <211> 19
   <212> DNA
   <213> Artificial Sequence
<220>
   <223> Description of Artificial Sequence: synthetic
<400> 335
   tgtggcacat aaaatgatt 19
<210> 336
   <211> 19
   <212> DNA
   <213> Artificial Sequence
<220>
   <223> Description of Artificial Sequence: synthetic
<400> 336
   tctactggtg tttgtctta 19
<210> 337
   <211> 19
   <212> DNA
   <213> Artificial Sequence
<220>
   <223> Description of Artificial Sequence: synthetic
<220>
   <223> r=g or a
<400> 337
   tctactggtr tttgtctta 19
<210> 338
   <211> 19
   <212> DNA
   <213> Artificial Sequence
<220>
   <223> Description of Artificial Sequence: synthetic
<400> 338
   taagacaaac accagtaga 19
<210> 339
   <211> 19
   <212> DNA
   <213> Artificial Sequence
<220>
   <223> Description of Artificial Sequence: synthetic
<220>
   <223> y=c or t
<400> 339
   taagacaaay accagtaga 19
<210> 340
   <211> 19
   <212> DNA
   <213> Artificial Sequence
<220>
   <223> Description of Artificial Sequence: synthetic
<400> 340
   tctactggta tttgtctta 19
<210> 341
   <211> 19
   <212> DNA
   <213> Artificial Sequence
<220>
   <223> Description of Artificial Sequence: synthetic
<400> 341
   taagacaaat acccgtaga 19
<210> 342
   <211> 19
   <212> DNA
   <213> Artificial Sequence
<220>
   <223> Description of Artificial Sequence: synthetic
<400> 342
   ttaattggcc attttggac 19
<210> 343
   <211> 19
   <212> DNA
   <213> Artificial Sequence
<220>
   <223> Description of Artificial Sequence: synthetic
<220>
   <223> y=c or t
<400> 343
   ttaattggcy attttggac 19
<210> 344
   <211> 19
   <212> DNA
   <213> Artificial Sequence
<220>
   <223> Description of Artificial Sequence: synthetic
<400> 344
   gtccaaaatg gccaattaa 19
<210> 345
   <211> 19
   <212> DNA
   <213> Artificial Sequence
<220>
   <223> Description of Artificial Sequence: synthetic
<220>
   <223> r=g or a
<400> 345
   gtccaaaatr gccaattaa 19
<210> 346
   <211> 19
   <212> DNA
   <213> Artificial Sequence
<220>
   <223> Description of Artificial Sequence: synthetic
<400> 346
   ttaattggct attttggac 19
<210> 347
   <211> 19
   <212> DNA
   <213> Artificial Sequence
<220>
   <223> Description of Artificial Sequence: synthetic
<400> 347
   gtccaaaata gccaattaa 19
<210> 348
   <211> 19
   <212> DNA
   <213> Artificial Sequence
<220>
   <223> Description of Artificial Sequence: synthetic
<400> 348
   aattttctcc ttacgggtg 19
<210> 349
   <211> 19
   <212> DNA
   <213> Artificial Sequence
<220>
   <223> Description of Artificial Sequence: synthetic
<220>
   <223> m=c or a
<400> 349
   aattttctcm ttacgggtg 19
<210> 350
   <211> 19
   <212> DNA
   <213> Artificial Sequence
<220>
   <223> Description of Artificial Sequence: synthetic
<400> 350
   cacccgtaag gagaaaatt 19
<210> 351
   <211> 19
   <212> DNA
   <213> Artificial Sequence
<220>
   <223> Description of Artificial Sequence: synthetic
<220>
   <223> k=g or t
<400> 351
   cacccgtaak gagaaaatt 19
<210> 352
   <211> 19
   <212> DNA
   <213> Artificial Sequence
<220>
   <223> Description of Artificial Sequence: synthetic
<400> 352
   aattttctca ttacgggtg 19
<210> 353
   <211> 19
   <212> DNA
   <213> Artificial Sequence
<220>
   <223> Description of Artificial Sequence: synthetic
<400> 353
   cacccgtaat gagaaaatt 19
<210> 354
   <211> 19
   <212> DNA
   <213> Artificial Sequence
<220>
   <223> Description of Artificial Sequence: synthetic
<400> 354
   ttctccttac gggtgttag 19
<210> 355
   <211> 19
   <212> DNA
   <213> Artificial Sequence
<220>
   <223> Description of Artificial Sequence: synthetic
<220>
   <223> y=c or t
<400> 355
   ttctccttay gggtgttag 19
<210> 356
   <211> 19
   <212> DNA
   <213> Artificial Sequence
<220>
   <223> Description of Artificial Sequence: synthetic
<400> 356
   ctaacacccg taaggagaa 19
<210> 357
   <211> 19
   <212> DNA
   <213> Artificial Sequence
<220>
   <223> Description of Artificial Sequence: synthetic
<220>
   <223> r=g or a
<400> 357
   ctaacaccck taaggagaa 19
<210> 358
   <211> 19
   <212> DNA
   <213> Artificial Sequence
<220>
   <223> Description of Artificial Sequence: synthetic
<400> 358
   ttctccttat gggtgttag 19
<210> 359
   <211> 19
   <212> DNA
   <213> Artificial Sequence
<220>
   <223> Description of Artificial Sequence: synthetic
<400> 359
   ctaacaccca taaggagaa 19
<210> 360
   <211> 19
   <212> DNA
   <213> Artificial Sequence
<220>
   <223> Description of Artificial Sequence: synthetic
<400> 360
   tgaatgttca gtggctccg 19
<210> 361
   <211> 19
   <212> DNA
   <213> Artificial Sequence
<220>
   <223> Description of Artificial Sequence: synthetic
<220>
   <223> m=a or c
<400> 361
   tgaatgttcm gtggctccg 19
<210> 362
   <211> 19
   <212> DNA
   <213> Artificial Sequence
<220>
   <223> Description of Artificial Sequence: synthetic
<400> 362
   cggagccact gaacattca 19
<210> 363
   <211> 19
   <212> DNA
   <213> Artificial Sequence
<220>
   <223> Description of Artificial Sequence: synthetic
<220>
   <223> k=t or g
<400> 363
   cggagccack gaacattca 19
<210> 364
   <211> 19
   <212> DNA
   <213> Artificial Sequence
<220>
   <223> Description of Artificial Sequence: synthetic
<400> 364
   tgaatgttcc gtggctccg 19
<210> 365
   <211> 19
   <212> DNA
   <213> Artificial Sequence
<220>
   <223> Description of Artificial Sequence: synthetic
<400> 365
   cggagccacg gaacattca 19
<210> 366
   <211> 19
   <212> DNA
   <213> Artificial Sequence
<220>
   <223> Description of Artificial Sequence: synthetic
<400> 366
   cgggtggtgt cacaggaag 19
<210> 367
   <211> 19
   <212> DNA
   <213> Artificial Sequence
<220>
   <223> Description of Artificial Sequence: synthetic
<220>
   <223> w=t or a
<400> 367
   cgggtggtgw cacaggaag 19
<210> 368
   <211> 19
   <212> DNA
   <213> Artificial Sequence
<220>
   <223> Description of Artificial Sequence: synthetic
<400> 368
   cttcctgtga caccacccg 19
<210> 369
   <211> 19
   <212> DNA
   <213> Artificial Sequence
<220>
   <223> Description of Artificial Sequence: synthetic
<220>
   <223> w=a or t
<400> 369
   cttcctgtgw caccacccg 19
<210> 370
   <211> 19
   <212> DNA
   <213> Artificial Sequence
<220>
   <223> Description of Artificial Sequence: synthetic
<400> 370
   cgggtggtga cacaggaag 19
<210> 371
   <211> 19
   <212> DNA
   <213> Artificial Sequence
<220>
   <223> Description of Artificial Sequence: synthetic
<400> 371
   cttcctgtgt caccacccg 19
<210> 372
   <211> 6
   <212> PRT
   <213> Artificial Sequence
   <223> Description of Artificial Sequence: synthetic
<400> 372
   Lys Leu Asn Asp Lys Ser
     1               5
<210> 373
   <211> 7
   <212> PRT
   <213> Artificial Sequence
   <223> Description of Artificial Sequence: synthetic
<400> 373
   Asp Thr Gly Ser Phe Met Asn
   1 5
<210> 374
   <211> 5
   <212> PRT
   <213> Artificial Sequence
   <223> Description of Artificial Sequence: synthetic
<400> 374
   His Phe Asn Tyr Pro
   1 5
<210> 375
   <211> 6
   <212> PRT
   <213> Artificial Sequence
   <223> Description of Artificial Sequence: synthetic
<400> 375
   Asp Thr Gly Leu Phe Met
   1 5
<210> 376
   <211> 20
   <212> DNA
   <213> Artificial Sequence
<220>
   <223> Description of Artificial Sequence: synthetic
<400> 376
   aaactgaacg ataaaaggta 20

## Description

### Field of the invention

The present invention relates generally to uses and methods of diagnosing, the phenotypic spectrum as well as the overlapping clinical characteristics with several forms of inherited abnormal expression and/or function of the Multi Drug Resistance-1 (MDR-1) gene. In particular, the present invention relates to a method of diagnosing acquired multidrug resistance or sensitivity related to the presence of a molecular variant of the MDR-1 gene or to the use of an oligo- or polynucleotide for such a diagnosis. Accordingly, the present invention provides a method of diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c); wherein the presence of said polynucleotide is considered indicative of said acquired multidrug resistance or sensitivity in said method.

In addition, the present invention provides a method of diagnosing a disorder related to the presence of a molecular variant of the MDR-1 gene or susceptibility to such a disorder, comprising determining the presence of a polynucleotide as defined herein in a sample from a subject. It, moreover, provides the use of an antibody specifically binding to the gene product of an MDR-1 gene for the detection of expression of a molecular variant MDR-1 gene comprising a polynucleotide as defined herein and/or for distinguishing MDR-1 alleles comprising a polynucleotide as defined herein. Furthermore, it provides the use of the MDR-1 gene single nucleotide polymorphism C3435T in exon 26 as defined in claim 1 as a pharmacogenetic factor for the preparation of a diagnostic composition for the prediction of blood levels of a MDR-1 substrate and/or inducer for improvement of drug safety and efficacy, to predict and prevent side effects and drug interactions and/or to increase patient compliance. Moreover, the present invention relates to methods for identifying and obtaining drug candidates and inhibitors for therapy of disorders related to the malfunction of the MDR-1 gene as well as to methods of diagnosing the status of such disorders. The present invention furthermore provides a diagnostic composition comprising the molecular variant MDR-1 gene as described herein or the primer or probe as described herein. Said diagnostic composition is particularly useful for diagnosing various diseases with drugs that are substrates; inhibitors or modulators of the MDR-1 gene or its product.

### Background of the invention

The human MDR-1 gene encodes an integral membrane protein whose function is the energy dependent transport of different substances from the inside of cells and cell membranes to the outside of the cell. While the normal physiological function of MDR-1 is most likely the protection of cells from toxic substances, it is also known that many substrates of the MDR-1 transporter are drugs that have been developed for the treatment of human diseases. Because of that, the degree of expression and the functionality of the MDR-1 gene product can directly affect the effectiveness of any drug that serves as a substrate of MDR-1. For example, it is well known that the expression levels, and hence the degree of the function of MDR-1, directly affects the effectiveness of anti-tumor drugs in cancer therapy. In fact, the gene name "MDR" stands for Multi-Drug-Resistance, reflecting the observance that the protein encoded by this gene causes cancer cells to become refractory to the treatment with many drugs, all of which are substrates of the MDR-1 transporter.
The MDR-1 gene is expressed not only on certain cancer cells where it may directly affect the therapeutic effectiveness of drugs by providing a protective barrier against drug entry, but also on different non-malignant cells in various organs, e.g. in the colon and at the blood brain barrier. Also in these cells MDR-1 can affect the activity and availability of drugs. For example, MDR-1 in colon can control or modulate the degree of drug uptake from the colon following oral drug intake. MDR-1 at the blood-brain barrier may also influence or control the degree to which MDR-1 substrates can be taken up into the brain. Here, elevated MDR-1 activity may prevent the uptake of sufficient amounts of desired brain-drugs into the brain, or vice versa, MDR-1. variants with reduced activity towards certain drugs might lead to abnormally increased accumulation in the brain, leading to undesired or even dangerous drug side effects.
The common factor that controls MDR-1 dependent transport in malignant as well as normal cells and tissues is the activity of MDR-1. The MDR-1 activity in turn is dependent (i) on the levels of expression of the MDR-1 gene which determines the amount of MDR-1 protein that is synthesized in the cells, and (ii) on the functionality of the synthesized MDR-1 protein, i.e. which substrates are recognized and transported out of the cell with which effectiveness. The first of these parameter, the level of expression of MDR-1, has been intensively analyzed, particularly because the sensitivity of tumor cells towards cancer chemotherapy often correlates inversely with upregulation of MDR-expression: high MDR-1 expression correlates often with unsufficient effectiveness of cancer chemotherapy. Although the observed MDR-1 overexpression can partially be attributed to MDR-1 gene amplifications, it is known that other so far undetermined reasons must also exist, among them possibly allelic differences. Small differences in the MDR-1 gene sequences in individuals may be causative for different levels of MDR-1 gene expression. Target regions in the human genome where sequence differences might exist that directly influence MDR-1 gene expression would be the control regions of gene expression: the promoter and enhancer regions of MDR-1 and regions that influence the mode or efficacy of splicing of MDR-1 pre-mRNAs. In addition, expression levels may be influenced by structural changes in the genome, such as methylation, general chromatin alterations and other factors that are linked to MDR-1, in the region directly at or surrounding the MDR-1 gene. It is very difficult to directly find such linked factors or sequences and prove their mechanism of gene activation or repression. However, the linear structure of the human genome on defined chromosomes opens the possibility to utilize identified polymorphisms, which by themselves are not directly influencing expression levels of genes, as marker for other so far unidentified changes in and around the MDR-1 gene that affect the expression levels. This effect is known as linkage: defined alleles and base variations can serve as a marker for an important phenotype even if these changes by themselves are not causative for that phenotype.

The second parameter, the functionality of the synthesized MDR-1 protein, i.e. which substrates are recognized and transported out of the cell with which effectiveness, is predominantly determined by the amino acid sequence of the protein that is encoded by the MDR-1 allele. It is well known that amino acid changes may alter the functionality of proteins. Examples for naturally occurring variations, i.e. different alleles that have a direct impact on the actions of various drugs are, e.g., cytochrome P450 polymorphisms, or polymorphisms in TPMT, APOE, and a variety of other genes. Also, tumor related variations, e.g., in the p53 gene are known to mediate such phenotypes. So far only some polymorphism in the MDR-1 gene have been described, and been correlated with clinical effects (Mickley, Blood 91 (1998), 1749-1756). A major question remains in this field whether more of such polymorphisms exist and, if so, whether these can be correlated with drug activity and/or drug side effects. Experiments with artificially introduced mutations in the MDR-1 gene show unambiguously that MDR-1 reacts quite sensitive to amino acid exchanges. It has been shown that artificial mutations in the MDR-1 gene that translate into protein changes can alter the substrate spectrum, effectiveness of substrate transport, control of transport, and also the sensitivity of MDR-1 towards inhibition with specific inhibitory substances. It is clear that naturally occurring mutations, if they exist can have similar effects. It is unknown, however, how many of such variations exist, and with what frequency and at what positions in the human MDR-1 gene.
Accordingly, means and methods for diagnosing and treating a variety of forms of multidrug resistance which result from MDR-1 gene polymorphisms, and sensitivity interfering, e.g., with chemotherapeutic treatment of diseases, in particular cancer, was hitherto not available but are nevertheless highly desirable.

Thus, the technical problem of the present invention is to comply with the needs described above.
The solution to this technical problem is achieved by providing the embodiments
characterized in the claims.

### Summary of the Invention

The present invention is based on the finding of a novel, so far unknown variation in the nucleotide sequences of the human MDR-1 (Multi Drug Resistance) gene and the population distribution of these alleles. Based upon the knowledge of this novel sequence and MDR-1 gene base deviations, diagnostic tests and reagents for such tests were designed for the specific detection and genotyping of MDR-1 alleles in humans, including homozygous as well as heterozygous, frequent as well as rare alleles of the MDR-1 gene. The determination of the MDR-1 gene allele status of humans with such tests is useful for the therapy of various diseases with drugs that are substrates, inhibitors or modulators of the MDR-1 gene product.
In a first embodiment, the invention provides a method of diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c); wherein the presence of said polynucleotide is considered indicative of said acquired multidrug resistance or sensitivity in said method.
In yet another embodiment, the invention provides the a method of diagnosing a disorder related to the presence of a molecular variant of the MDR-1 gene or susceptibility to such a disorder, comprising determining the presence of a polynucleotide as defined herein in a sample from a subject.
In a further embodiment, the invention provides the use of an oligo- or polynucleotide for diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c).
In a further embodiment the invention provides a primer or probe for the aforementioned diagnostic use as well as a diagnostic composition comprising the molecular variant of the MDR-1 gene described herein, the primer or probe or the antibody described herein. Furthermore, the present invention provides the use of the MDR-1 gene single nucleotide polymorphism C3435T in exon 26 as defined in claim 1 as a pharmacogenetic factor for the preparation of a diagnostic composition for the prediction of blood levels of a MDR-1 substrate and/or inducer for improvement of drug safety and efficacy, to predict and prevent side effects and drug interactions and/or to increase patient compliance.

The pharmaceutical and diagnostic compositions, methods and uses of the invention are useful for the diagnosis and treatment of inherited drug resistance in tumors and other diseases the therapy of which is dependent on drug treatment. The novel variant forms of MDR-1 genes according to the invention provide the potential for the development of a pharmacodynamic profile of drugs and prodrugs for a given patient.

### Description of the invention

The finding and characterization of a variation in the MDR-1 gene, and diagnostic tests for the discrimination of different MDR-1 alleles in human individuals provide a very potent tool for improving the therapy of diseases with drugs that are targets of the MDR-1 gene product, and whose cellular uptake is therefore dependent on MDR-1. The diagnosis of the individual allelic MDR-1 status permits a more focused therapy, e.g., by opening the possibility to apply individual dose regimens of drugs. It may also be useful as prognostic tool for therapy outcome, certainly an improved approach over the use of general MDR-expression as prognostic maker. Furthermore, diagnostic tests to genotype MDR-1, and novel MDR-1 variants, will not only improve therapy established drugs and help to correlate genotypes with drug activity or side effects. These tests and sequences also provide reagents for the development of novel inhibitors that specifically modulate the activity of the individual types of MDR-1. The feasibility to use specific inhibitors of individual (artificially created) MDR-variants, and their potential therapeutic application, has, for example, recently been demonstrated in a model system (Moscow J. A. et al., Blood 94 (1999), 52-61; Dey S. et al., Biochemistry 38 (1999), 6630-6639).

Thus, the present invention provides a novel way to exploit molecular biology and pharmalogical research for drug therapy while bypassing their potential detrimental effects which are due to expression of variant MDR-1 genes.

Accordingly, the invention relates to a method of diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c); wherein the presence of said polynucleotide is considered indicative of said acquired multidrug resistance or sensitivity in said method.

In the context of the present invention the term "molecular variant" MDR-1 gene or protein as used herein means that said MDR-1 gene or protein differs from the wild type MDR-1 gene or protein as described herein (Genomic sequences of the MDR-1 gene are described, for examples, for exons 1-7: Accession number AC002457; for exon 8: Accession number M29429, J05168, AC005068; for exon 9: Accession number M29430, J05168, AC005068; for exon 10: Accession number M29431, J05168, AC005068; for exon 11 to 13: Accession number M29432, J05168 and AC005068; for exon 14: Accession number M29433, J05168, AC005068; for exon 15: Accession number M29434, J05168, AC005068; for exon 16: Accession number M29435, J05168, AC005068; for exon 17: Accession number M29436, J05168, AC005068; for exon 18: Accession number M29437, J05168, AC005068; for exon 19: Accession number M29438, J05168, AC005068; for exon 20: Accession number M29439, J05168, AC005068; for exon 21: Accession number M29440, J05168, AC005068; for exon 22: Accession number M29441, J05168, AC005068; for exon 23: Accession number M29442, J05168, AC005068; for exon 24: Accession number M29443, J05168, AC005068; for exon 25: Accession number M29444, J05168, AC005068; for exon 26: Accession number M29445, J05168, AF016535, AC005068; for exon 27: Accession number M29446, J05168, AC005068; for exon 28: Accession number M29447, J05168, AC005068) by way of nucleotide substitution(s), addition(s) and/or deletion(s). Preferably, said nucleotide substitution(s) result(s) in a corresponding change in the amino acid sequence of the MDR-1 protein.

The term "corresponding" as used herein means that a position is not only determined by the number of the preceding nucleotides and amino acids, respectively. The position of a given nucleotide or amino acid in accordance with the present invention which may be deleted, substituted or comprise one or more additional nucleotide(s) may vary due to deletions or additional nucleotides or amino acids elsewhere in the gene or the polypeptide. Thus, under a "corresponding position" in accordance with the present invention it is to be understood that nucleotides or amino acids may differ in the indicated number but may still have similar neighboring nucleotides or amino acids. Said nucleotides or amino acids which may be exchanged, deleted or comprise additional nucleotides or amino acids are also comprised by the term "corresponding position". Said nucleotides or amino acids may for instance together with their neighbors form sequences which may be involved in the regulation of gene expression, stability of the corresponding RNA or RNA editing, as well as encode functional domains or motifs of the protein of the invention.

In accordance with the present invention, the mode and population distribution of novel so far unidentified genetic variation in the MDR-1 gene has been analyzed by sequence analysis of relevant regions of the human MDR-1 gene from many different individuals. It is a well known fact that genomic DNA of individuals, which harbor the individual genetic makeup of all genes, including MDR-1 can easily be purified from individual blood samples. These individual DNA samples are then used for the analysis of the sequence composition of the MDR-1 gene alleles that are present in the individual which provided the blood sample. The sequence analysis was carried out by PCR amplification of relevant regions of the MDR-1 gene, subsequent purification of the PCR products, followed by automated DNA sequencing with established methods (ABI dyeterminator cycle sequencing).
One important parameter that had to be considered in the attempt to determine the individual MDR-1 genotype and identify novel MDR-1 variants by direct DNA-sequencing of PCR-products from human blood genomic DNA is the fact that each human harbors (usually, with very few abnormal exceptions) two gene copies of each autosomal gene (diploidy). Because of that, great care had to be taken in the evaluation of the sequences to be able to identify unambiguously not only homozygous sequence variations but also heterozygous variations. The details of the different steps in the identification and characterization of novel MDR-1 gene polymorphisms (homozygous and heterozygous) are described in the examples 1 and 2 below.

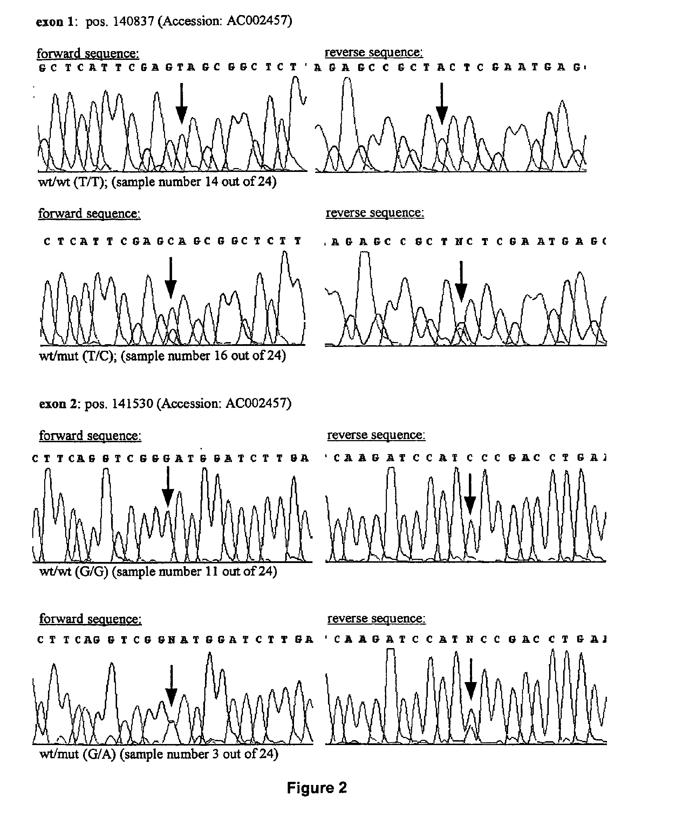

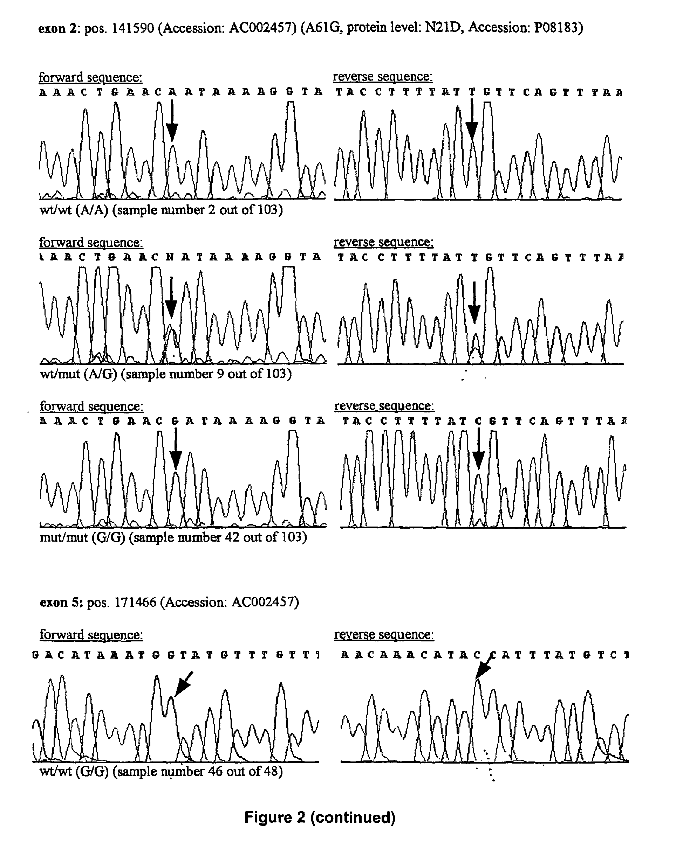

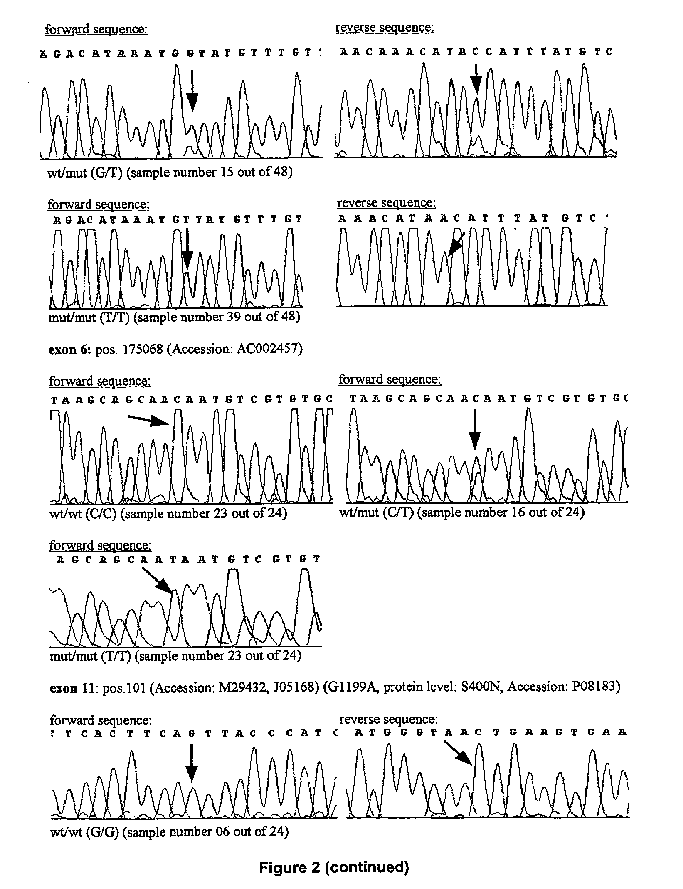

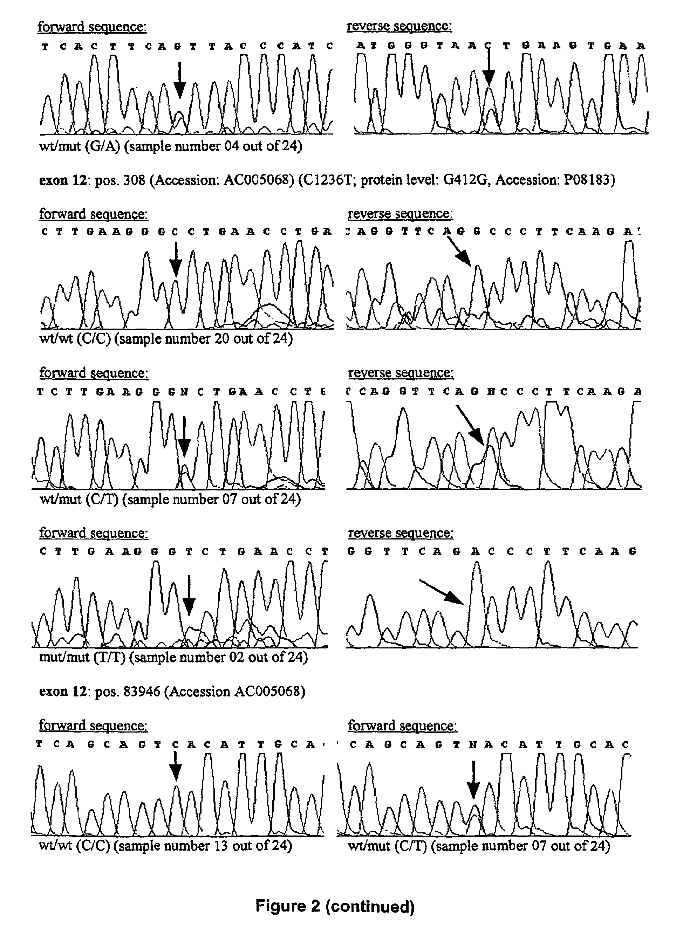

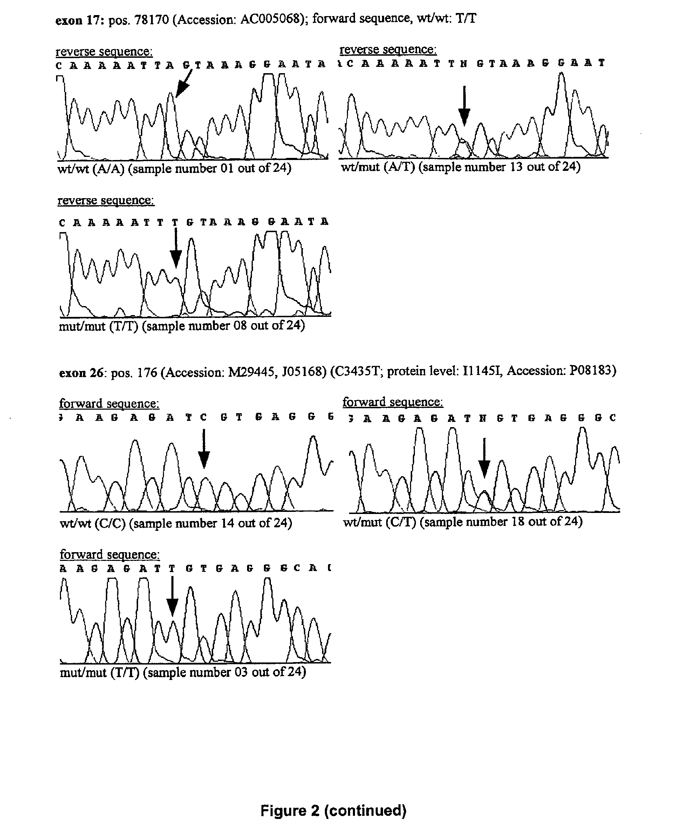

The mutations in the MDR-1 gene detected in accordance with the present invention are illustrated in Figure 2 (indicated by an arrow). The methods of the mutation analysis followed standard protocols and are described in detail in the examples. In general such methods to be used in accordance with the present invention for evaluating the phenotypic spectrum as well as the overlapping clinical characteristics with other forms of multidrug resistance and altered tolerance to drugs in patients with mutations in the MDR-1 gene encompass for example haplotype analysis, single-strand conformation polymorphism analysis (SSCA), PCR and direct sequencing; see also Mickley (1998), and references cited therein. On the basis of thorough clinical characterization of many patients the phenotypes can then be correlated to these mutations as well as to mutations that had been described earlier.

As is evident to the person skilled in the art this new molecular genetic knowledge can now be used to exactly characterize the genotype of the index patient where a given drug takes an unusual effect and of his family.

Over the past 20 years, genetic heterogeneity has been increasingly recognized as a significant source of variation in drug response. Many scientific communications (Meyer, Ann. Rev. Pharmacol. Toxicol. 37 (1997), 269-296 and West, J. Clin. Pharmacol. 37 (1997), 635-648) have clearly shown that some drugs work better or may even be highly toxic in some patients than in others and that these variations in patient's responses to drugs can be related to molecular basis. This "pharmacogenomic" concept spots correlations between responses to drugs and genetic profiles of patient's (Marshall, Nature Biotechnology, 15 (1997), 954-957; Marshall, Nature Biotechnology, 15 (1997), 1249-1252).

In this context of population variability with regard to drug therapy, pharmacogenomics has been proposed as a tool useful in the identification and selection of patients which can respond to a particular drug without side effects. This identification/selection can be based upon molecular diagnosis of genetic polymorphisms by genotyping DNA from leukocytes in the blood of patient, for example, and characterization of disease (Bertz, Clin. Pharmacokinet. 32 (1997), 210-256; Engel, J. Chromatogra. B. Biomed. Appl. 678 (1996), 93-103). For the founders of health care, such as health maintenance organizations in the US and government public health services in many European countries, this pharmacogenomics approach can represent a way of both improving health care and reducing overheads because there is a large cost to unnecessary drugs, ineffective drugs and drugs with side effects.

The mutations in the variant MDR-1 genes sometime result in amino acid deletion(s), insertion(s) and in particular in substitution(s) either alone or in combination. It is of course also possible to genetically engineer such mutations in wild type genes or other mutant forms. Methods for introducing such modifications in the DNA sequence of MDR-1 gene are well known to the person skilled in the art; see, e.g., Sambrook, Molecular Cloning A Laboratory Manual, Cold Spring Harbor Laboratory (1989) N.Y.

For the investigation of the nature of the alterations in the amino acid sequence of the MDR-1 protein computer programs may be used such as BRASMOL that are obtainable from the internet. Furthermore, folding simulations and computer redesign of structural motifs can be performed using other appropriate computer programs (Olszewski, Proteins 25 (1996), 286-299; Hoffman, Comput. Appl. Biosci. 11 (1995), 675-679). Computers can be used for the conformational and energetic analysis of detailed protein models (Monge, J. Mol. Biol. 247 (1995), 995-1012; Renouf, Adv. Exp. Med. Biol. 376 (1995); 37-45). These analysis can be used for the identification of the influence of a particular mutation on binding and/or transport of drugs.

Usually, said amino acid deletion, addition or substitution in the amino acid sequence of the protein encoded by the polynucleotide of the invention is due to one or more nucleotide substitution, insertion or deletion, or any combinations thereof.

The polynucleotide of the invention used in the methods or uses of the present invention as described herein may further comprise at least one nucleotide and optionally amino acid deletion, addition and/or substitution other than those specified hereinabove, for example those described in the prior art; e.g., Mickley (1998). This allows the study of synergistic effects of the mutations in the MDR-1 gene on the pharmalogical profile of drugs in patients who bear such mutant forms of the gene or similar mutant forms that can be mimicked by the above described proteins. It is expected that the analysis of said synergistic effects provides deeper insights into the onset of multidrug resistant phenotypes of certain forms of cancer. From said deeper insight the development of diagnostic and pharmaceutical compositions related to cancer will greatly benefit.

Thus, in a preferred embodiment of the method of diagnosing acquired multidrug resistance or sensitivity related to the presence of the molecular variant of the MDR-1 gene as described herein the nucleotide substitution result in altered expression of the variant MDR-1 gene compared to the corresponding wild type gene.

The polynucleotide of the invention employed in the methods or uses of the present invention may be, e.g., DNA, cDNA, genomic DNA, RNA or synthetically produced DNA or RNA or a recombinantly produced chimeric nucleic acid molecule comprising any of those polynucleotides either alone or in combination.

With the methods and uses of the invention, it is now possible to study *in vivo* and *in vitro* the efficiency of drugs in relation to particular mutations in the MDR-1 gene of a patient and the affected phenotype. Furthermore, the methods and uses of the invention can be used to determine the pharmacological profile of drugs, and for the identification and preparation of further drugs which may be more effective for the treatment of, e.g., cancer, in particular for the amelioration of certain phenotypes caused by the respective mutations such as those described above.

Thus, the present application describes drug/pro-drug selection and formulation of pharmaceutical compositions for the treatment of diseases which are amenable to chemotherapy taking into account the polymorphism of the variant form of the MDR-1 gene that cosegregates with the affected phenotype of the patient to be treated. This allows the safe and economic application of drugs which for example were hitherto considered not appropriate for therapy of, e.g., cancer due to either their side effects in some patients and/or their unreliable pharmalogical profile with respect to the same or different phenotype(s) of the disease. The means and methods and uses described herein can be used, for example, to improve dosing recommendations and allows the prescriber to anticipate necessary dose adjustments depending on the considered patient group.

In a further embodiment the present invention relates to a method of identifying and obtaining an MDR-1 inhibitor capable of modulating the activity of a molecular variant of the MDR-1 gene or its gene product comprising the steps of
(a) contacting a cell expressing a molecular variant MDR-1 gene comprising the polynucleotide of the invention in the presence of components capable of providing a detectable signal in response to drug transport, with a compound to be screened under conditions to permit MDR-1 mediated drug transport, and
(b) detecting the presence or absence of a signal or increase of a signal generated from the drug transport, wherein the presence or increase of the signal is indicative for a putative inhibitor.

The term "compound" in a method of the invention includes a single substance or a plurality of substances which may or may not be identical.
Said compound(s) may be chemically synthesized or produced via microbial fermentation but can also be comprised in, for example, samples, e.g., cell extracts from, e.g., plants, animals or microorganisms. Furthermore, said compounds may be known in the art but hitherto not known to be useful as an inhibitor, respectively. The plurality of compounds may be, e.g., added to the culture medium or injected into a cell or non-human animal of the invention.
If a sample containing (a) compound(s) is identified in the method of the invention, then it is either possible to isolate the compound from the original sample identified as containing the compound, in question or one can further subdivide the original sample, for example, if it consists of a plurality of different compounds, so as to reduce the number of different substances per sample and repeat the method with the subdivisions of the original sample. It can then be determined whether said sample or compound displays the desired properties, for example, by the methods described herein or in the literature (Spector et al., Cells manual; see supra). Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art or by using and modifying the methods as described herein. Furthermore, the person skilled in the art will readily recognize which further compounds and/or enzymes may be used in order to perform the methods of the invention, for example, enzymes, if necessary, that convert a certain compound into the precursor which in turn represents a substrate for the MDR-1 protein. Such adaptation of the method of the invention is well within the skill of the person skilled in the art and can be performed without undue experimentation.

Compounds which can be used in accordance with the present invention include peptides, proteins, nucleic acids, antibodies, small organic compounds, ligands, peptidomimetics, PNAs and the like. Said compounds can also be functional derivatives or analogues of known drugs such as verapamil or cyclosporin. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described. Furthermore, peptide mimetics and/or computer aided design of appropriate drug derivatives and analogues can be used, for example, according to the methods described below. Such analogs comprise molecules having as the basis structure of known MDR-substrates and/or inhibitors and/or modulators; see infra.
Appropriate computer programs can be used for the identification of interactive sites of a putative inhibitor and the MDR-1 protein of the invention by computer assistant searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known inhibitors. Appropriate peptidomimetics and other inhibitors can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Domer, Bioorg. Med. Chem. 4 (1996), 709-715. Furthermore, the three-dimensional and/or crystallographic structure of inhibitors and the MDR-1 protein of the invention can be used for the design of peptidomimetic drugs (Rose, Biochemistry 35 (1996), 12933-12944; Rutenber, Bioorg. Med. Chem. 4 (1996), 1545-1558).

In summary, the aforementioned methods for identifying and obtaining compounds may provide compounds which may be used in specific doses for the treatment of specific forms of diseases, e.g., cancer the chemotherapy of which is complicated by malfunctions of the MDR-1 gene often resulting in a drug resistant or sensitive phenotype.

In a still further embodiment the present invention relates to a method of diagnosing a disorder related to the presence of a molecular variant MDR-1 gene or susceptibility to such a disorder comprising
(a) determining the presence of a polynucleotide of the invention in a sample from a subject; and/or
(b) determining the presence of a variant form of MDR-1 protein, for example, with the antibody of the invention.

In accordance with this embodiment of the present invention, the method of testing the status of a disorder or susceptibility to such a disorder can be effected by using a polynucleotide or a nucleic acid molecule of the invention, e.g., in the form of a Southern or Northern blot or *in situ* analysis. Said nucleic acid sequence may hybridize to a coding region of either of the genes or to a non-coding region, e.g. intron. In the case that a complementary sequence is employed in the method of the invention, said nucleic acid molecule can again be used in Northern blots. Additionally, said testing can be done in conjunction with an actual blocking, e.g., of the transcription of the gene and thus is expected to have therapeutic relevance. Furthermore, a primer or oligonucleotide can also be used for hybridizing to one of the above-mentioned MDR-1 genes or corresponding mRNAs. The nucleic acids used for hybridization can, of course, be conveniently labeled by incorporating or attaching, e.g., a radioactive or other marker. Such markers are well known in the art. The labeling of said nucleic acid molecules can be effected by conventional methods. Additionally, the presence or expression of variant MDR-1 genes can be monitored by using a primer pair that specifically hybridizes to either of the corresponding nucleic acid sequences and by carrying out a PCR reaction according to standard procedures: Specific hybridization of the above mentioned probes or primers preferably occurs at stringent hybridization conditions. The term "stringent hybridization conditions" is well known in the art; see, for example, Sambrook et al., "Molecular Cloning, A Laboratory Manual" second ed., CSH Press, Cold Spring Harbor, 1989; "Nucleic Acid Hybridisation, A Practical Approach", Hames and Higgins eds., IRL Press, Oxford, 1985. Furthermore, the mRNA, cRNA, cDNA or genomic DNA obtained from the subject may be sequenced to identify mutations which may be characteristic fingerprints of mutations the MDR-1 gene. The present invention further comprises methods wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments as described above.
Further modifications of the above-mentioned embodiment of the invention can be easily devised by the person skilled in the art, without any undue experimentation from this disclosure; see, e.g., the examples. An additional embodiment of the present invention relates to a method wherein said determination is effected by employing an antibody of the invention or fragment thereof. The antibody used in the method of the invention may be labeled with detectable tags such as a histidine flags or a biotin molecule.

In a preferred embodiment of the present invention, the above described methods comprise PCR, ligase chain reaction, restriction digestion, direct sequencing, nucleic acid amplification techniques, hybridization techniques or immunoassays (Sambrook et al., loc. cit. CSH cloning, Harlow and Lane loc. cit. CSH antibodies).

In a preferred embodiment of the method of the present invention said disorder is cancer.

In this context and as used throughout this specification, "functional" MDR-1 gene means a gene wherein the encoded protein having part or all of the primary structural conformation of the wild type MDR-1 protein, i.e. possessing the biological property of mediating the drug transport through the membrane. Detection of the expression of a variant MDR-1 gene would allow the conclusion that said expression is interrelated to the generation or maintenance of a corresponding phenotype of the disease. Furthermore, the present invention relates to the use of an oligo- or polynucleotide for diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c).

In a particular preferred embodiment said oligonucleotide to be employed in the diagnostic use is about 10 to 100, more preferably 15 to 50 nucleotides in length and comprises the nucleotide sequence of SEQ ID NO: 122 or a wild type ("wt")- or mutated ("mut")-sequence of the promoter or of an exon of the MDR-1 gene depicted in Table 8 or a complementary sequence of any one of those.

Hence, in a still further embodiment, the present invention relates to a primer or probe consisting of an oligonucleotide as defined above to be employed in the diagnostic use. In this context, the term "consisting of" means that the nucleotide sequence described above and employed for the primer or probe of the invention does not have any further nucleotide sequences of the MDR-1 gene immediately adjacent at its 5' and/or 3' end. However, other moieties such as labels, e.g., biotin molecules, histidin flags, antibody fragments, colloidal gold, etc. as well as nucleotide sequences which do not correspond to the MDR-1 gene may be present in the primer and probes of the present invention. Furthermore, it is also possible to use the above described particular nucleotide sequences and to combine them with other nucleotide sequences derived from the MDR-1 gene wherein these additional nucleotide sequences are interspersed with moieties other than nucleic acids or wherein the nucleic acid does not correspond to nucleotide sequences of the MDR-1 gene.

In addition, the present invention relates to the use of an antibody binding specifically to the gene product of an MDR-1 gene for the detection of the expression of a molecular variant MDR-1 gene comprising a polynucleotide of the invention and/or for distinguishing MDR-1 alleles comprising a polynucleotide of the invention.

Moreover, the present invention relates to a diagnostic composition comprising the polynucleotide, the primer or probe or the antibody as described herein.

Antibodies against the variant MDR-1 protein of the invention can be prepared by well known methods using a purified protein according to the invention or a (synthetic) fragment derived therefrom as an antigen. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals. The antibodies can be monoclonal antibodies, polyclonal antibodies or synthetic antibodies as well as fragments of antibodies, such as Fab, Fv or scFv fragments etc. Furthermore, antibodies or fragments thereof to the aforementioned polypeptides can be obtained by using methods which are described, e.g., in Harlow and Lane "Antibodies, A Laboratory Manual", CSH Press, Cold Spring Harbor, 1988. These antibodies can be used, for example, for the immunoprecipitation and immunolocalization of the variant MDR-1 proteins of the invention as well as for the monitoring of the presence of such variant MDR-1 proteins, for example, in recombinant organisms, and for the identification of compounds interacting with the proteins according to the invention. For example, surface plasmon resonance as employed in the BIAcore system can be used to increase the efficiency of phage antibodies which bind to an epitope of the protein of the invention (Schier, Human Antibodies Hybridomas 7 (1996), 97-105; Malmborg, J. Immunol. Methods 183 (1995), 7-13).

As shown in Examples 6 and 8 the polymorphisms identified in accordance with the present invention, especially the single nucleotide polymorphism (SNP) C3435T in exon 26 of the MDR-1 gene are useful as a pharmacogenetic factor that enables the prediction of blood levels of diverse MDR-1 substrates and inducers for improvement of drug safety and efficacy, i.e. to predict and prevent side effects and drug interactions and to increase patient compliance. Such substrates and inducers are, for example, anticonvulsant/antiepileptic drugs, like Phenytoin; cardiac glycosides, like Digoxin; immunosuppressive drugs like Cyclosporin A and FK506; macrolid-antibiotics, like Clarithromycin and Erythromycin; and macrocyclic-antibiotics, like Rifampin. Thus, the present invention also relates to the use of the above described SNPs as a pharmacogenetic factor in accordance with the above. Preferably, the polymorphism is the MDR-1 exon 26 (C3435T) SNP either alone or in conjunction with any other SNP such as those described above.

Further applications of the polymorphisms identified in accordance with the present invention and means and methods that may be used in accordance with the above described embodiments can be found in the prior art, for example, as described in US-A-5,856,104, wherein the there described means and methods for forensics, Paternity testing, correlation of polymorphisms with phenotypic traits, genetic mapping of phenotypic traits, etc. can be equally applied in accordance with the present invention.

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

### Brief description of the figures

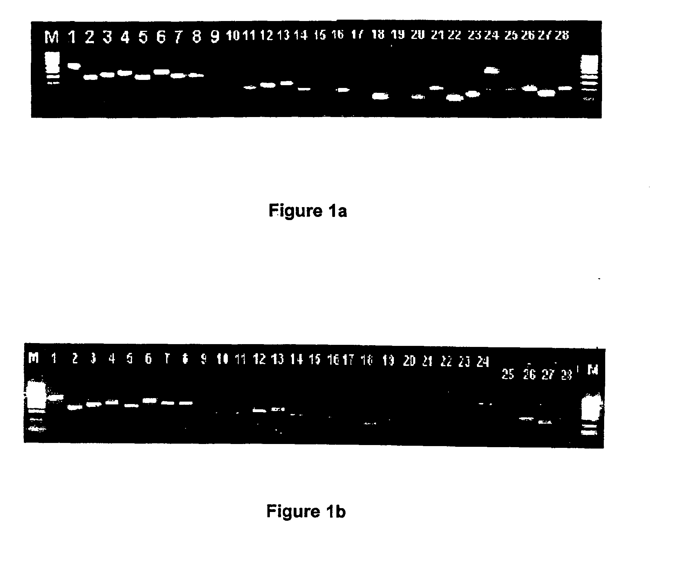

- **Figure 1**:: Gel of selected PCR fragments, before and after purification. Agarose (Appli Chem, Darmstadt) gel electrophoresis (1.5% Agarose gel) of MDR-1 PCR fragments before (A) and after (B) the purification step. M: molecular weight markers, 1-28: PCR fragments containing the sequences of exons 1-28 of the human MDR-1 gene, including relevant sequences that are flanking these exons.
- **Figure 2:**: Examples for homozygous and heterozygous MDR-1 alleles. The sequences of PCR fragments containing the sequences of exons 1-28 of the human MDR-1 gene, including relevant sequences that are flanking these exons, were determined by automated sequencing using the ABI Dyeterminator techniques. Heterozygous and homozygous deviations from the published MDR-1 sequence can be detected directly in the DNA sequence profiles.

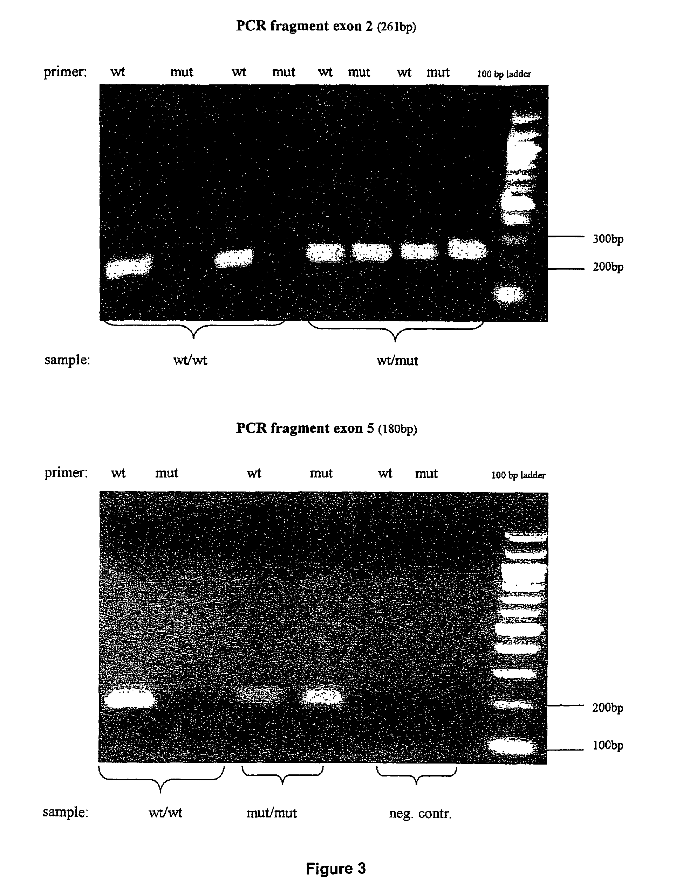

- **Figure 3:**: Examples for diagnosis of homozygous and heterozygous MDR-1 alleles. Agarose. (AppliChem, Darmstadt) gelelectrophoresis (1.5% Agarose gel) of the allele specific PCR fragments of exon 2 (261 bp) and exon 5 (180 bp).

The invention will now be described by reference to the following biological examples which are merely illustrative and are not to be construed as a limitation of the scope of the present invention.

### Examples

### Example 1: Isolation of genomic DNA from human blood, generation and purification of MDR-1 gene fragments

Genomic DNA was obtained by standard ion exchange chromatography techniques (Quiagen kits for isolation of genomic DNA from blood). Blood from all the individuals that were tested (volunteers from the department of Pharmacology at the Charitee Berlin) was obtained under consideration of all legal, ethical and medical and bureaucratical requirement of the Charitee Clinicum in Berlin, Germany.

Specific oligonucleotide primers, 2 for each fragment, were applied to obtain by polymerase chain reaction (PCR) defined DNA fragments containing specific parts of the human MDR-1 gene. These specific oligonucleotide primers were designed to bind to sequences upstream and downstream of the various exons of the MDR-1 gene. The resulting DNA fragments were to encode not only exon sequences, but also some intron sequences at the exon-intron boundaries. Such intronic sequences close to the exons are known to be important for correct processing and subsequent expression of the protein encoding mRNA, a process known as "splicing". Oligonucleotide primer pairs that were optimized for each of the 28 exons of the human MDR-1 gene, synthesized and purified by affinity chromatography (OPC cartridges). The sequence for each of primer is listed in Table 1.

Polymerase chain reactions were performed under conditions that were optimized for each of the fragments that cover the 28 exons of the human MDR-1 gene as well as the core promoter and enhancer region. PCRs were carried out for all exons in a reaction volume of 25µl. 50ng DNA template was added to standard PCR buffer containing 1,5mM MgCl₂ (Quiagen, Hilden), 50*µ*M dNTP's (Quiagen, Hilden), 25 pMol each primer (Metabion, Munich) and 0,625 U Taq polymerase (Quiagen, Hilden). All PCRs were performed on a Perkin Elmer thermocycler (model 9700) with an initial denaturation step of 2 min at 94°C and 36 amplification cycles of denaturation 94°C for 45 sec, primer annealing depending on the primer's melting temperature (PCR conditions: A-H) for 45 sec, and 45 sec for 72°C followed by a final extension of 72°C for 5 min. For the single PCR conditions A-H the following annealing temperatures were applied: A: 53°C; B: 56°C; C: 55°C D: 57,5°C; E: 58°C; F: 59°C; G: 54°C; H: 60°C.
PCRs were carried out for all fragments (promoter and enhancer) in a reaction volume of 50*µ*l. 50ng DNA template (exceptions: 100ng for promoter fragments 1-3) was added to standard PCR buffer containing 1,5mM MgCl2 (Quiagen, Hilden), 200*µ*M dNTP's (Quiagen, Hilden), 30 pMol each primer (Metabion, Munich; exception: 20 pMol for enhancer fragment 1) and 1 U Taq polymerase (Quiagen, Hilden). All PCRs were performed on a Perkin Elmer thermocycler (model 9700) with an initial denaturation step of 3 min at 94°C and different amplification cycles (30 for promoter fragment 2 + 4 and enhancer fragment 1; 31 for promoter fragment 3; 32 for promoter fragment 1 and enhancer fragment 2) of denaturation 94°C for 30 sec, primer annealing depending on the primer's melting temperature (PCR conditions: A and B) for 30 sec, and 30 sec for 72°C followed by a final extension of 72°C for 2 min. For the single PCR conditions **A** and **B** the following annealing temperatures were applied: **A**: 58°C; **B**: 56°C
The optimized PCR-conditions and the resulting size of the desired and obtained fragments are listed in Table 1. Examples of the resulting MDR-1 gene fragments that were used for further analysis of the individual genotype are presented in Figure **1.**

The defined DNA fragments containing specific parts of the human MDR-1 gene, exon sequences as well as some intron sequences at the exon-intron boundaries were processed to remove nonincorporated nucleotides and buffer components that otherwise might interfere with the subsequent determination of the individual MDR-1 genotype by direct DNA sequencing. For this purification, standard ion exchange chromatography techniques were used (Quiagen kits for PCR fragment purification). For all of the fragments, sufficient yields of purified fragments, suitable for direct DNA sequence analyses, were obtained. Examples of purified MDR-1 gene fragments that were used for direct sequence analysis of the individual MDR-1 genotype are presented in Figure 1.

### Example 2: Identification of different MDR-1 gene alleles by sequence determination in various individuals

For the sequence analysis of relevant regions of the human MDR-1 gene from many different individuals, PCR amplification of the relevant regions of the MDR-1 gene were carried out (see Tab.1) and the purified PCR products subsequently sequenced with established methods (ABI dyeterminator cycle sequencing). A very important parameter that was needed to consider using this approach was that each normal human individual harbors two MDR-1 gene copies. Because of this diploidy (of autosomal genes, and MDR-1 is autosomally encoded), great care had to be taken in the evaluation of the sequences to be able to identify unambiguously not only homozygous sequence variations but also heterozygous variations. Because of that, it was never relied on only one determined sequence, but always obtained at least two sequences from each defined MDR-1 gene fragment from each individual, by sequencing both opposite DNA strands.

For the initial evaluation of MDR-1 variations in the human population, sequence analysis of the relevant regions, including all exons, of the human MDR-1 gene was carried out from the genomic DNA from 24 different individuals. This number of individual samples was then extended for selected MDR-1 gene fragments, some of which have been analyzed from 127 individuals. The sequences were manually inspected for the occurrence of DNA sequences that were deviant from the published MDR-1 sequences, which are considered as "wildtype" sequences in all of this work. Because population genetics enables a calculation of the expected frequency of homozygous vs. heterozygous alleles of a defined gene (Hardy Weinberg formula, p e2 + 2pq + q e2 = 1), it was also possible to confirm the predicted (with that formula) distribution of homozygous vs. heterozygous alleles and deviations with the experimental findings. This serves as internal control and confirmation that a detected sequence deviation indeed represents a novel allele.

Several novel MDR-1 sequence variations were discovered and experimentally confirmed using this approach which are shown in Figure 2. 8 polymorphisms appear in intron sequences close flanking the exons 5, 6, 12 and 17 (SEQ ID NOs: 91, 154 and 160 for exon 5), (SEQ ID NOs: 101 and 166 for exon 6), (SEQ ID NO: 116 for exon 12) and (SEQ ID NOs: 119 and 172 for exon 17). 7 polymorphisms were found in the coding region, 2 in the exons 2 and 26, and one each in exons 5, 11 and 12 and one in noncoding exon 1 (SEQ ID NOs: 79 and 85 (for exon 2), 122 and 178 (for exon 26), 97 (for exon 5), 106 (for exon 11), 112 (for exon 12) and 73 (for exon 1), respectively). 3 variations result in changes in the amino acid sequence of the MDR-1 protein (SEQ ID NOs: 85 (for N21D), 97 (for F103S) and 106 (for S400N), respectively). Their changes will alter the MDR protein. One change that does not alter the protein is located directly before the ATG translational start codon (SEQ ID NO: 79). It is well known that this position is very important for the levels of expression of proteins by controlling the effectiveness of translation. Further polymorphisms do not change the amino acid composition of MDR-1, but they still are useful tools for MDR-1 genotyping because each of these variations define a novel MDR-1 allele. It is known that the expression of MDR-1 varies greatly between different individuals, and one very likely explanation for this variability in expression levels is allelic differences in the region directly in and surrounding the MDR-1 gene. Thus all novel and defined MDR-1 alleles serve as markers for the determination of the MDR-1 gene status in patients The importance of this MDR-1 genotyping for the diagnosis and therapy of diseases is well known to experts in the field, and it has also been explained in detail above in the introductory chapter.

The exact positions and further details of the novel MDR-1 alleles, including the exact novel sequence and sequence deviation, and the homozygous vs. heterozygous distribution of the allele in the population are listed in Table 2. The expected frequency for homozygotes of the variant allele were calculated on the basis of the Hardy-Weinberg distribution. The deviant base in the sequence is bold and underlined. Figure 2 shows examples of the discovery and appearance of novel variants in DNA samples from homozygous or heterozygous individuals.

### Example 3: Methods for specific detection and diagnosis of MDR-1 alleles

Methods to detect the various MDR-1 alleles that have been identified utilize the principle that specific sequence differences can be translated into reagents for allele differentiation. These reagents provide the necessary backbone for the development of diagnostic tests. Examples for such reagents include- but are not limited to - oligonucleotides that deviate from the wildtype MDR-1 sequence in the newly identified base substitution. Frequently, the principles of diagnostic tests for the determination of the individual MDR-1 gene status include - but are not limited to-differences in the hybridization efficiencies of such reagents to the various MDR-1 alleles. In addition, differences in the efficacy of such reagents in, or as different substrates for, enzymatic reactions, e.g. ligases or polymerases or restriction enzymes can be applied. The principles of these tests are well known to experts in the field. Examples are PCR- and LCR techniques, Chip-hybridizations or MALDI-TOF analyses. Such techniques are described in the prior art, e.g., PCR technique: Newton, (1994) PCR, BIOS Scientific Publishers, Oxford; LCR-technique: Shimer, Ligase chain reaction. Methods Mol. Biol. 46 (1995), 269-278; Chip hybridization: Ramsay, DNA chips: State-of-the art. Natrue Biotechnology 16 (1998), 40-44; and MALDI-TOF analysis: Ross, High level multiplex genotyping by MALDI-TOF mass spectrometry, Nature Biotechnology 16 (1998), 1347-1351. Other test principles are based on the application of reagents that specifically recognize the MDR-1 variant as translated expressed protein. Examples are allele-specific antibodies, peptides, substrate analogs, inhibitors, or other substances which bind to (and in some instances may also modify the action of) the various MDR-1 protein forms that are encoded by the new MDR-1 alleles. The examples that are presented here, to demonstrate the principles of diagnostic tests with reagents derived from the novel nucleotide substitutions defined in this application, are based on PCR-methods. It is obvious that, applying the described specific reagents, any of the other methods will also work for the differentiation of MDR-1 alleles.

### Example 4: Diagnosis of MDR-1 Alleles by specific PCR

Allele-specific PCR is a technique well known to experts in the field that allows the differentiation of alleles of genes by the application of the polymerase chain reaction with reagents (primer combinations) that are specifically designed for the detection of single allele sequences. The main component of such tests, and the only reagent that provides the specificity of such tests, are oligonucleotides that are designed to contain sequences that specifically distinguish different alleles of genes.

In this example, specific oligonucleotides were designed that can distinguish different MDR-1 alleles because of their differential hybridization efficacy to different alleles and because of their varying ability to serve as substrates for enzymatic reactions (the enzyme in this example being a thermostable polymerase). The reagents that were specifically designed and able to detect the presence and/or absence of the newly defined mdr-1 alleles in individual humans are listed as specific primer combinations for each new allele in Table 3. The design of these reagents bases on the newly discovered nucleotide sequences and base substitutions in the human MDR-1 gene, which are presented in example 2 and listed in Table 2 and Figure 2. In addition to the design of specific reagents, diagnostic test that are based upon the principle of polymerase chain reaction needs optimization of test conditions, i.e. optimized PCR-conditions. The result of test is in this case given as presence or absence of specific DNA fragments obtained using genomic DNA from individual humans as testable ingredient (template). The preparation of the genomic DNA from the blood of individuals is described in example 1.
PCRs were carried out for all fragments in a reaction volume of 20µl. 50ng DNA template was added to standard PCR buffer (Qiagen, Hilden) containing 1,5mM MgCl₂, 250µM dNTP's (Qiagen, Hilden), 1 x Q-solution (Qiagen, Hilden), 20 pMol each primer (Metabion, Munich; specific wt primer + common primer and specific mut primer + common primer) and 1 U Taq polymerase (Qiagen, Hilden). All PCRs were performed on a Perkin Elmer thermocycler (model 9700) with an initial denaturation step of 3 min at 95°C and 30 amplification cycles of denaturation 94°C for 30 sec, primer annealing depending on the primer's melting temperature (PCR conditions: A-E) for 30 sec, and 30 sec for 72°C followed by a final extension of 72°C for 8 min. For the single PCR conditions A-E the following annealing temperatures were applied: A: 54°C; B: 58°C; C: 50°C; D: 61 °C; E: 53°C.
The deviant base in the respective specific primer sequence is underlined and in a bold style. The presence or absence of specific DNA fragments in this assay translates in presence or absence of the tested allele.

Examples for such readouts, as results for the MDR-1 allele detection diagnosis, are shown Figure 3. It is obvious from these examples (Tab.3, Fig.3), that these tests are suitable to differentiate the presence of the analyzed MDR-1 alleles in humans. Homozygous as well as heterozygous, frequent as well as rare alleles of the MDR-1 gene can be detected. The specificity of these tests relies solely; and totally depends, on the specific oligonucleotide reagents that were applied. The design of these reagents in turn was dependent on the sequence information of the discovered MDR-1 variants and novel alleles, that are presented in example 2 and Table 2.

### Example 5: Diagnosis and correlation of different MDR-1 polymorphisms with expression levels and in vivo activity of MDR-1 in patients

To identify potential direct correlations of MDR-1 polymorphisms with clinical relevant phenotypes in humans, probands from a study at the Dr. Margarete Fischer-Bosch-Institut for Clinical Pharmacology in Stuttgart, were subjected to the determination of MDR-1 polymorphisms as described in examples 2-4. The expression levels of MDR-1 in the colon and liver of these patients was also estimated by established immunohistochemical detection of the MDR-1 protein. In the proband population, in addition to measurements of the expression levels of MDR-1 in the colon, measurements of MDR-1 upon induction of the gene by rifampicine were performed. Also, the in vivo activity of MDR-1 under noninduced and rifampicine induced conditions was determined by measuring the blood concentrations of orally administered digoxin (1mg), which is a known MDR-1 substrate and whose blood concentration also depends on the MDR-1 activity in the colon.
The results of the MDR-1 measurements, rifampicine induction experiments and digoxin-experiments, as well as results from the MDR-1 polymorphism detection analysis in the proband population show correlations between MDR-1 gene expression and MDR-1 in vivo activity with certain polymorphisms.

### MDR-1 protein levels:

As shown in table 4, a T/C polymorphism at position 176 in Acc.#M29445/J05168 in exon 26 correlates with the expression levels of MDR-1. Presence of the T allele at this position indicates weaker MDR-1 expression levels compared to samples which have only the corresponding homozygous C-allele. The mean of the rifampicin-induced MDR-1 levels of the C-allele population is much higher as that of the T-population (924 vs 587 relative units). In total agreement with that, a proband homozygous for the T-allele had the lowest detectable uninduced and induced MDR-1 level while a proband homozygous for the C allele displayed the highest level of all probands tested. The difference of induced MDR-1 expression levels between these individuals was 9-fold.

### MDR-1 in vivo activity:

Table 5 shows the results of the measurements of the in vivo activity of MDR-1 under noninduced and rifampicine induced conditions. This was done by measuring the blood concentrations of orally administered digoxin which is a known MDR-1 substrate and whose blood concentration also depends on the MDR-1 activity in the colon. Consistant with the observation that the polymorphism at position 176 in Acc.#M29445/J05168 in exon 26 T/C correlates with the expression levels of MDR-1, a correlation of this polymorphism was observed with digoxin blood levels, which in turn reflects the MDR-1 protein activity in vivo. The probands that harbor the T allele (correlates with weaker MDR-1 expression, see Tab. 4), contain higher blood levels of digoxin compared to samples which have only the corresponding homozygous C-allele. The reason for that is that the uptake of MDR-1 substrates such as digoxin from the colon to the blood appears to be more effective in humans with lower MDR-1 expression. This is totally consistent with the function of MDR-1 in the colon, i.e. re-transport and elimination of substrates from the uptaking cells into the lumen of the colon. The mean of the non-induced as well as rifampicin-induced digoxin concentration in the blood (correlates invers to MDR-1 activity) of the C-allele population are consistantly lower than those of the T-popuiation (63.9 vs. 44.9 and 45 vs. 28.6 Dig AUC induced). In total agreement with that, a proband with the homozygous T allele had the highest detectable digoxin concentration in the blood after rifampicine induction (57.3 Dig.AUC) and a proband homozygous for the C-allele displayed the lowest level of all probands (12.3 Dig.AUC). The difference of the digoxin blood levels between these individuals was more than 4-fold.

### MDR-1 in a patient population:

The results of our analysis of the correlation of MDR-1 expression levels, MDR-1 protein activity and MDR-1 polymorphism detection analysis are further corrobated by an analysis of the MDR-1 expression and MDR-1 genotyping of various patients from the Dr. Margarete Fischer-Bosch-Institut for Clinical Pharmacology in Stuttgart. Immunohistology was performed on the various patient tissue samples, particularly colon and liver, and they were compared to each other to allow a relative comparison of the MDR-protein between these samples. Within each set of experiments, patient samples were ranked according to their MDR-1 staining intensity, i.e. 1^{st} rank equals highest MDR-intensity and last rank lowest MDR-1 intensity.
The correlation of this ranking analysis with the MDR-1 genotype shows that the T allele at the polymorphism at position 176 in Acc.#M29445/J05168 in exon 26 correlates with lower expression of the MDR-1 gene when compared to patients which carry homozygous the C allele at this position. In this analysis, two other polymorphisms showed some correlation with MDR-1 expression: A homozygous T genotype at position 171466 in AC002457 (intron 4) may correlate with high expression and a polymorphism (GA) at position 101 in exon 11 (M29432/J05168) may correlate with low expression.

### Example 6: Validation of the genotype/phenotype correlation of the exon 26 (C3435T) polymorphism with extended sample numbers

To further validate the correlation of the single nucleotide polymorphism (SNP) T/C at position 176 in ACC.# M29445/J05168 described in the previous examples and now also referred to as MDR-1 exon 26 SNP C3435T, (position correspond to MDR-1 cDNA GenBank accession no. AF016535, Codon TTC exon 10, F335, is missing in that sequence), with the first base of the ATG start codon set to 1) with the levels of intestinal MDR-1 expression (first results shown in Example 5), additional volunteers of a further experimental study at the Dr. Margarete Fischer-Bosch-Institute for Clinical Pharmacology in Stuttgart were analysed. The expression levels of MDR-1 in the intestine of these volunteers and patients had been determined by quantitative immunohistochemistry and Western blots of biopsies and enterocyte preparations of the duodenum. To assure that this analysis reflect the specific PGP expression in intestinal enterocytes, an additional marker protein that is expressed in enterocytes, villin, was simultanously analyzed. The results of this analysis are shown in **Figure 4.** The T/T genotype is associated with significant lower MDR-1 expression levels compared to the C/C genotype. Individuals with a C/T genotype show an intermediate phenotype.
For a further validation of the correlation of MDR-1 genotype with intestinal digoxin uptake, additional volunteers of another clinical study at the University Medical Center, Charite in Berlin that addresses blood levels of digoxin after oral application (without rifampin induction and PGP protein determination, Johne et al. (1999), Clin. Pharmacol. Thr. 66, 338-345) were evaluated for their MDR-1 genotype in exon 26. In this study, maximum plasma concentrations (Cₘₐₓ) were evaluated during steady state conditions of digoxin. This pharmacokinetic parameter especially reveals differences in the absorption of digoxin between the different groups. Figure 5 shows a comparison of digoxin Cₘₐₓ of 7 volunteers that carried homozygously the T/T allele and 7 volunteers with the homozygous C/C genotype in exon 26. Volunteers homozygous for the T-allele show significant higher levels of digoxin compared to volunteers with a C/C genotype. The mean difference of 38% in digoxin Cₘₐₓ between the groups is statistically significant (p=0.006, Mann Whitney U 2 sample test) and reflects the impact of this polymorphism on digoxin pharmacokinetics.

Figure 4: Correlation of the exon 26 SNP with MDR-1 expression under non-induced conditions. The MDR-phenotype (expression and activity) of 21 volunteers and patients was determined by Western blot analyses. The box plot shows the distribution of MDR-1 expression clustered according to the MDR-1 genotype at the relevant exon 26 SNP. The genotype-phenotype correlation has a significance of p=0.056 (N=21).

**Figure 5**: Correlation of MDR-1 genotype and digoxin uptake in vivo. The MDR-1 genotype in exon 26 was analyzed in 14 healthy volunteers who participated in a clinical study that addresses blood levels of digoxin during steady state conditions (Johne et al. (1999), Clin. Pharmacol. Thr. 66, 338-345). A statistically significant difference (p=0.006; Mann Whitney U 2 sample test) was found in the comparison of maximum concentrations (Cₘₐₓ) of digoxin between two groups of 7 healthy volunteers harboring either T/T or C/C genotype. The mean difference of 38% in Cₘₐₓ may reflect the importance of genotype on the absorption of digoxin after oral application. A 0.25 mg dose was applied upon steady-state of digoxin.

### Example 7: Identification of new MDR-1 polymorphisms by sequence analysis of a large collection of various individuals

An extended search for SNPs in the human MDR-1 gene revealed in addition to the different novel MDR-1 polymorphisms numerous further new polymorphisms in the MDR-1 gene which are listed in Table 8. Within the new screen the number of individual samples was extended for all MDR-1 exons, as well as for the MDR-1 promoter fragments, some of which have been analyzed from 236 individuals.

It is possible that in addition to the MDR-1 exon 26 (C3435T) SNP that can be used to predict PGP expression, other more rare polymorphisms in regions of the MDR-1 gene have also some affect on expression. E.g. promoter polymorphisms and protein changing SNPs are very likely to have an additional effect on MDR-1 expression and activity. Futhermore, al these novel polymorphisms can be utilized to generate an exact individual MDR-1 genotype -i.e. allele composition- which may be unique for individuals and thus very useful to predict individual MDR-1 dependent drug response.
The more polymorphisms are known in the human MDR-1 gene, the more complete and thus more useful will an individual MDR-1 genotype description be. The identification of these 32 new MDR-1 polymorphisms is an further important step towards achieving the goal of establishing many different MDR-1 genotypes that predict outcome and side effects of drug therapy.

### Example 8: Determination of the MDR-1 exon 26 (C3435T) polymorphism as a pharmacogenetic factor that influences drug levels in combination with other pharmacogenetic factors

The anticonvulsant drug Phenytoin is commonly used in the therapy of epilepsia, acute and chronic suppression of ventricular arrhythmias and in digitalis intoxication. The narrow therapeutic range with a number of severe side effects in combination with a nonlinear pharmacokinetic (i.e. overproportional increase of plasma levels in response to dosage elevation) make Phenytoin treatment challenging and suitable parameters to predict plasma levels from a given dose highly desirable in order to improve therapeutic outcome and to prevent side effects.
It is known that the polymorphic enzymes 2C9 and 2C19 have an effect on the metabolism of Phenytoin (Mamiya et al. 1998, Epilepsia Dec;39(12):1317-23), and it has been shown that 2C9 defects can lead to abnormal blood levels that may cause side effects or drug inefficacy (Aynacioglu et al. 1999, Br J Clin Pharmacol. Sep;48(3):409-15). However, it is also clear that 2C9 genotyping does not permit to make exact bloodlevel predictions from given dose. Even in 2C9 genotyped individuals, compensated for the respective enzyme genotype, blood levels vary significantly.

**Table 6** shows that MDR-1 exon 26 (C3435T) SNP plays - in addition to 2C9- a clear role in phenytoin blood levels, and MDR-1 genotyping for this SNP permits a more accurate correlation between phenytoin dose, genotype and blood levels.
Within 2C9/19 enzyme genotyped groups, variation of levels can be explained by the MDR-1 genotype, particularly in the groups of 2C9/C19 poor metabolizers, which already show increased blood levels. Here MDR-1 genotyping is able to identify a subgroup of patients who is at increased risk to exhibit extraordinary high phenytoin blood levels: Poor metabolizers which have the MDR-1 T/T genotype. These patients have an increased risk to encounter overdose related adverse drug effects. For example, within a group of 100 patients which received phenytoin, a 2C9 deficient patient with the low PGP (T/T) genotype showed the highest blood concentration, which was about twofold increased compared to the "normal" population. The correlation between Cyp 2C9 genotype and Phenytoin plasma levels is statistically significant, but the significance increases by taking into account the MDR-1 T/T genotype as a covariate (p< 0.001, ANCOVA).

**Table 6: Dependence of Phenytoin levels on pharmacogenetic components**

| MDR-1 genotype | CC and CT | TT |
|---|---|---|
| intestinal PGP | high/medium | low |

| Phenytoin blood levels: | | |
|---|---|---|
| 2C9 metabolizers | normal | normal |
| 2C9 weak and/or deficient metabolizers | high | **VERY HIGH** |

**Table 1:**

| PCR fragment name | PCR primer position | Primer sequence | PCR conditions | fragment size |
|---|---|---|---|---|
| Exons: | for exons 1-7: (Accession: AC002457) | | | |
| | | | | |
| ex.1 | 140510-140529 | "Forward: 5' CCC,TTA, ACT, ACG, TCC, TGT, AG 3' " | E | 572bp |
| | 141081-141062 | "Reverse: 5' GAG,GAC,TTC,ACA,CTA,TCC,AC 3' " | | |
| | | | | |
| ex.2 | 141423-141442 | "Forward: 5' TCT, TAC, TGC, TCT, CTG, GGC, TTC 3' " | C | 347bp |
| | 141770-141751 | "Reverse: 5' CTC,AGC,CAA,CAA,ACT,TCT,GC 3' " | | |
| | | | | |
| ex.3 | 145681-145700 | "Forward: 5' CAC,TCA, GTG, ATA, ACC, ACG, TA 3' " | D | 385bp |
| | 146066-146047 | "Reverse: 5' GCA, TCT, CCA, TTA, ACA, TAC, CC 3' " | | |
| | | | | |
| ex.4 | 155899-155918 | "Forward: 5' GGG, TGT, CTT, GGA, CTA, GGT, TG 3' " | D | 422bp |
| | 156320-156301 | "Reverse: 5' TGC, CTC, CTA, CAG, GAC, TAA, AC 3' " | | |
| | | | | |
| ex.5 | 171308-171327 | "Forward: 5' CAC, ACA, GTC, AGC, AGA, GAA, GT 3' " | G | 353bp |
| | 171660-171639 | "Reverse: 5' ACT, ATC,AAG,AGT,ATT,GTT,CTC,C 3' " | | |
| | | | | |
| ex.6 | 174661-174680 | "Forward: 5' GGA,ATG,AGT,GGT,CTC,TTT,GG 3' " | E | 442bp |
| | 175102-175083 | "Reverse: 5' AAG,GCA,CTG,GGA,ACA,AAA,GG 3' " | | |
| | | | | |
| ex.7 | 175322-175342 | "Forward: 5' TCC,TAG,TAG,AAA,CTT,CTA,CCC 3' " | E | 390bp |
| | 175711-175693. | "Reverse: 5' TTC,CGT,AGG,GTG,AGA,GCA,G 3' " | | |
| | | | | |

| | for exons 8-28: (Accession: AC005068) | | | |
|---|---|---|---|---|
| | | | | |
| ex.8 | 95327-95307 | "Forward: 5' CAG, ATT, TTG, CTC, TAC, ACA, TGC 3' " | G | 391bp |
| | 94937-94959 | "Reverse: 5' ATT, AGT, TAT, GCT, GTA, ATA, CAT, CC 3' " | | |
| | | | | |
| ex.9 | 87858-87837 | "Forward: 5' CAT,GTA,TAT,CAC,AGG,ACT,GAA,C 3' " | A | 406 bp |
| | 87453-87474 | "Reverse: 5' CTA,GTA,GTG,CAT,ATG,TCT,GTA,G 3' " | | |
| | | | | |
| ex.10 | 84881-84861 | "Forward: 5' GTG,ACA,GAA,TGA,GAA,CCT,GTC 3' " | B | 422 bp |
| | 84460-84480 | "Reverse: 5' TGG,AGA,GCT,GGA,TAA,AGT,GAC 3' " | | |
| | | | | |
| ex.11 | 84415-84390 | "Forward: 5' AAA,TTG,ATC,TGT,TAG,AAG,CCA,AG 3' " | A | 228bp |
| | 84184-84206 | "Reverse: 5' ACT, AGG, TTT, AAA, TAT, ACA, TGC, AC 3' " | | |
| ex.12 | 84183-84163 | "Forward: 5' GAA,CAG,TCA,GTT,CCT,ATA,TCC 3' " | D | 260bp |
| | 83919-83939 | "Reverse: 5' GGG, CAA, CAT, CAG, AAA, GAT, GTG 3' " | | |
| | | | | |
| ex.13 | 83939-83919 | "Forward: 5' CAC,ATC,TTT,CTG,ATG,TTG,CCC 3' " | H | 295bp |
| | 83644-83662 | "Reverse: 5' AAG,GGC,AAA,GGG,CAA,GGA,C 3' " | | |
| | | | | |
| ex.14 | 83354-83334 | "Forward: 5' TGG,GTT,TTC,TGT,GGT,AGA,AAT 3 " | A | 214bp |
| | 83141-83160. | "Reverse: 5' GTT,GGT,TTG,AAC,TAA,GCC,TC 3' " | | |
| | | | | |
| ex.15 | 79861-79891 | "Forward: 5' AAA,TTT,CTC,TCT,CTT,TAG,GCC 3' " | G | 199bp |
| | 79663-79684 | "Reverse: 5' TTC, TGA, AGT, TAA, ACT, ATA, CCT, G 3' " | | |
| | | | | |
| ex.16 | 78834-78812 | "Forward: 5' TTC, TTA, TTT, ATT, TTA, GAC, AGC, AG 3' " | B | 209bp |
| | 78626-78644 | "Reverse: 5' GCA,TCT,CCC,TTC,ATA,CCA,G 3' " | | |
| | | | | |
| ex.17 | 78207-78185 | "Forward: 5' CAT,TGA,TAA,GGA,ATA,AGG,ATA,GG 3' " | B | 427bp |
| | 77781-77800 | "Reverse: 5' CCT,ATC,ACA,AAC,CAG,ACT,GC 3' " | | |
| | | | | |
| ex.18 | 75902-75382 | "Forward: 5' ATT,TCC,AGC,GTA,CTA,AGG,CTC 3' " | A | 358 bp |
| | 75045-75065 | "Reverse: 5' ACT,TGG,CTG,TTA,GTA,GCC,ATG 3' " | | |
| | | | | |
| ex.19 | 73301-73281 | "Forward: 5' GTC, ACA, GAA, ACA, TAG, CAA, GCC 3' " | B | 329bp |
| | 72973-72993 | "Reverse: 5' CAA, CCA, ATA, TAG, GAG, CTA, GGC 3' " | | |
| | | | | |
| ex.20 | 70482-70461 | "Forward: 5' ACA,TCC,TGA,GTA,CTA,AAT,GCA,G 3' " | C | 351 bp |
| | 70132-70154 | "Reverse: 5' AAA, GCA, TGT, GAT, ATA, TTC,GTA, GG 3' | | |
| | | | | |
| ex.21 | 65342-65319 | "Forward: 5' TTT, CTC, TAA, TTT, GTT, TTG, TTT, TGC 3' " | B | 245bp |
| | 65098-65118 | "Reverse: 5' TTT, AGT, TTG, ACT, CAC, CTT, CCC 3'" | | |
| | | | | |
| ex.22 | 54877-54857 | "Forward: 5' CCT, TCC, AGA, TCC, ATA, ACT, CAG 3' " | C | 372 bp |
| | 54506-54527 | "Reverse: 5' TAC,CTT,CTA,GCC,AAA,GTA,ATC,C 3' " | | |
| | | | | |
| ex. 23 | 53436-53415 | "Forward: 5' AAG, TGT, AAA, GTG, AGG, ACC, AAA, C 3' " | B | 391 bp |
| | 53047-53067 | "Reverse: 5' TTC, ATG, AGG, CTT, CAC, AGT, AGG 3'" | | |
| | | | | |
| ex.24 | 50544-50523 | "Forward: 5' TCA,GCT,TTC,TAG;CAT,TGT,GAT,G 3' " | F | 354 bp |
| | 50191-50213 | "Reverse: 5' TTG,AAA,GGA,ATC,TAT,GAT,CTA,GG 3' " | | |
| | | | | |
| ex.25 | 49262-49242 | "Forward: 5' TTC,TTC,TCA,TTG,CAG,AAC,ACA 3' " | F | 230bp |
| | 49033-49052 | "Reverse: 5' TGT,CAA,AGT,GTG,CTC,ACC,AC 3' " | | |
| ex.26 | 43323-43302 | "Forward: 5' GAT, CTG, TGA, ACT, CTT, GTT, TTC, A 3' " | A | 244bp |
| | 43080-43100 | "Reverse: 5' GAA, GAG, AGA, CTT, ACA, TTA, GGC 3' " | | |
| | | | | |
| ex.27 | 39992-39971 | "Forward: 5' CAT,GAA,CCA,TTC,TTA,GCT,TCT,G 3' " | A | 451 bp |
| | 39543-39564 | "Reverse: 5' TTG, TGT, CAT, CTT, TAC, TCT, CCT, G 3' " | | |
| | | | | |
| ex.28 | 38285-38263 | "Forward: 5' ATG, TGA, TTA, TGG, AAT, AGG, TTG, TC 3' " | A | 247bp |
| | 38036-39058 | "Reverse: 5' TAT,TTA,ACA,TCT,CAT,ACA,GTC,AG 3' " | | |
| | | | | |

| | for promoter fragments: | | | |
|---|---|---|---|---|
| | (Accession: AC002457) | | | |
| Promoter: | | | | |
| | | | | |
| fragment 1 | 138856-138876 | "Prom 1f (f): 5' CCA, AGG, ACT, GTT, GAA, AGT, AGC 3' " | A | 487 bp |
| | 139342-139322 | "Prom 3r (r): 5' TTG, CAT, ATG, CAA, GTG, TAC, AGC 3' " | | |
| | | | | |
| fragment 2 | 139285-139304 | "Prom 2f (f): 5' CAC,AGG,GTT,GTT,GTT,AAG,CC 3' " | A | 574 bp |
| | 139858-139838 | "Prom 5r (r): 5' TCT,GAG,GAT,GTT,TCC,ACT,TTC 3' " | | |
| | | | | |
| fragment 3. | 139809-139831 " | Prom 4f (f): 5' TTA, TGG, CTT, TGA, AGT, ATG, AGT, TA 3' " | B | 570 bp |
| | 140378-140358 | "Prom 6r (r): 5' GCA, TGC, TTG, ACA, GTT, TCT, GAG 3' " | | |
| | | | | |
| fragment 4 | 140358-140378 | "Prom 6f (f) : 5' CTC, AGA, AAC, TGT, CAA, GCA, TGC 3' " | A | 592 bp |
| | 140949-140931 | "Prom 7r (r): 5' TTG,GAA,CGG,CCA,CCA,AGA,C 3' " | | |
| | | | | |

| | for enhancer fragments: | | | |
|---|---|---|---|---|
| Enhancer: | "(Accession: M57451, J05674) " | | | |
| | | | | |
| fragment 1 | pos. 1-19 | "Enh 1f (f): 5' CCC, TTC, TAA, CCA, TGG, CCA, G 3' " | A | 338 bp |
| | 338-320 | "Enh 3r (r) : 5', GTG, CCT, CCT, GTC, AAT, GGT, G 3' " | | |
| | | | | |
| fragment 2 | 279-297 | "Enh 2f (f): 5' CTA,CTG,AAA,CCG,CAG,CAT,G 3' " | A | 416 bp |
| | 694-676 | "Enh 4r (r): 5' TTG, GAG, ACA, GTG, ACT, CAC, C 3" | | |

**Table 2:**

| PCR fragment name | Position of the variation | frequency: heterozygotes | frequency: homozygotes mutant | frequency: homozygotes mutant "(expected Hardy-Weinberg)" | wt-sequence | wt/mut- and/or mut-sequence |
|---|---|---|---|---|---|---|
| | | | | | | |

| | for exons 1-7: (Accession: AC002457) | | | | | |
|---|---|---|---|---|---|---|
| | | | | | | wt/mut: |
| ex.1 | 140837 | 4.17% | . <4% | 0.04% | f: GCTCATTCGAGTAGCGGCTCT | f: CTCATTCGAGT/CAGCGGCTCTT |
| | | | | | r: AGAGCCGCTACTCGAATGAG | r: AGAGCCGCTA/GCTCGAATGAG |
| | | | | | | mut: |
| | | | | | | f:CTCATTCGAGCAGCGGCTCTT |
| | | | | | | r: AGAGCCGCTGCTCGAATGAG |
| | | | | | | |
| | | | | | | wt/mut: |
| ex.2 | 141530 | 12.60% | <4% | 0.50% | f: CTTCAGGTCGGGATGGATCTTGA | f: CTTCAGGTCGGG/AATGGATCTTGA |
| | | | | | r: CAAGATCCATCCCGACCTGA | r: CAAGATCCATC/TCCGACCTGA |
| | | | | | | *mut*: |
| | | | | | | f:CTTCAGGTCGGAATGGATCTTGA |
| | | | | | | r:CAAGATCCATTCCGACCTGA |
| | | | | | | |
| | | | | | | wt/mut: |
| ex.2 | 141590 | 15.50% | 0.97% | 1.20% | f: AAACTGAACAATAAAAGGTA | f: AAACTGAACA/GATAAAAGGTA |
| | | | | | r. TACCTTTTAGTTCAGTTTAA | r. TACCTTTTATT/CGTTCAGTTTAA |
| | | | | | | mut: |
| | | | | | | f:AAACTGAACGATAAAAGGTA |
| | | | | | | r: TACCTTTTATCGTTCAGTTTAA |
| | | | | | | |
| ex.5 | 171466 | 26.08% | 4.34% | 2.90% | f: GACATAAATGGTATGTTTGTTT | wt/mut: f:AGACATAAATGG/TTATGTTTGT |
| | | | | | r:AACAAACATACCATTTATGTCT | r:AACAAACATAG/ACAMATGTC |
| | | | | | | mut: |
| | | | | | | f:AGACATAAATGTTATGTTTGT |
| | | | | | | r:AACAAACATAACATTTATGTC |
| | | | | | | |
| | | | | | | wt/mut: |
| ex.5 | 171512 | <2% | <2% | <2% | f: GATACAGGGTTCTTCATGAAT | f: GATACAGGGTT/CCTTCATGAAT |
| | | | | | r: ATTCATGAAGAACCCTGTATC | r. ATTCATGAAGA/GACCCTGTATC |
| | | | | | | mut: |
| | | | | | | f:GATACAGGGTCCTTCATGAAT |
| | | | | | | r:ATTCATGAAGGACCCTGTATC |
| | | | | | | wt/mut: |
| | | | | | | |
| ex.6 | 175068 | 36.36% | 18.18% | 12.90% | f: TAAGCAGCAACAATGTCGTGTGC | f: TAAGCAGCAAC/TAATGTCGTGTGC |
| | | | | | | mut: |
| | | | | | | f:AGCAGCAATAATGTCGTGT |

| | for exons 11 + 12: (Accession: "M29432,J05168)" | | | | | |
|---|---|---|---|---|---|---|
| | | | | | | |
| | | | | | | wt/mut: |
| ex.11 | 101 | 13.64% | <4% | 0.50% | f: TTCACTTCAGTTACCCATC | f: TCACTTCAG/ATTACCCATC |
| | | | | | r: ATGGGTAACTGAAGTGAA | r: ATGGGTAAC/TTGAAGTGAA |
| | | | | | | mut. |
| | | | | | | f: TCACTTCAATTACCCATC |
| | | | | | | r: ATGGGTAATTGAAGTGAA |
| | | | | | | |
| | | | | | | wt/mut: |
| ex.12 | 308 | 52.2% | 12.50% | 15.20% | f: CTTGAAGGGCCTGAACCTGA | f: TCTTGAAGGGC/TCTGAACCTG |
| | | | | | r. CAGGTTCAGGCCCTTCAAGA | r: TCAGGTTCAGG/ACCCTTCAAGA |
| | | | | | | |

| | for exon 12: (Accession: AC005068) | | | | | mut/mut: |
|---|---|---|---|---|---|---|
| | | | | | | f: CTTGAAGGGTCTGAACCT |
| | | | | | | r: GGTTCAGACCCTTCAAG |
| | | | | | | |
| | | | | | | wt/mut: |
| ex.12 | 83946 | 8.70% | < 4 % | 0.16% | f: TCAGCAGTCACATTGCA | f: CAGCAGTC/TACATTGCAC |
| | | | | | | mut: |
| | | | | | | f: CAGCAGTTACATTGCAC |

| | for exon 17: (Accession: AC005068) | | | | | |
|---|---|---|---|---|---|---|
| | | | | | | |
| | | | | | | wt/mut: |
| ex.17 | 78170 | 45.83% | 12.50% | 12.90% | r: CAAAAATTAGTAAAGGAATA | r: ACAAAAATTA/TGTAAAGGAAT |
| | | | | | | mut/mut: |
| | | | | | | r: CAAAAATTTGTAAAGGAATA |

| | for exon 26: (Accession: "M29445,J05168)" | | | | | |
|---|---|---|---|---|---|---|
| | | | | | | |
| | | | | | | wt/mut: |
| ex.26 | 176 | 54.16% | 25% | 27% | f: GAAGAGATCGTGAGGG | f: GAAGAGATC/TGTGAGGGC |
| | | | | | | mut/mut: |
| | | | | | | f:AAGAGATTGTGAGGGCA |

| **PCR fragment name** | **Position of the variation** | **frequency:** heterozygotes | **frequency:** homozygotes mutant | **frequency:** homozygotes mutant (expected, Hardy-Weinberg) | **wt-sequence** | **wt/mut- and/or mut-sequence** |
|---|---|---|---|---|---|---|
| | for exons 1-7: (Accession: AC002457) | | | | | |
| | | | | | | |
| | | | | | | **wt/mut:** |
| ex. 5 | 171456 | 8,33% | <4% | 0,20% | f: GACTAAAGAGACATAAATG | f: GACTAAAGAG/CACATAAATG |
| | | | | | r: CATTTATGTCTCTTTAGTC | r: CATTTATGTC/GTCTTTAGTC |
| | | | | | | **mut:** |
| | | | | | | f: GACTAAAGACACATAAATG |
| | | | | | | r:CATTTATGTGTCTTTAGTC |
| | | | | | | |
| | | | | | | **wt/mut:** |
| ex.5 | 171404 | 4,17% | <4% | 0,04% | f:ATCATTAAATGAAATGAGT | f: ATCATTAAAT/CGAAATGAGT |
| | | | | | r: ACTCATTTCATTTAATGAT | r:ACTCATTTCA/GTTTAATGAT |
| | | | | | | **mut:** |
| | | | | | | f: ATCATTAAACGAAATGAGT |
| | | | | | | r: ACTCATTTCGTTTAATGAT |
| | | | | | | |
| | | | | | | **wt/mut:** |
| ex.6 | 175074 | 8.33% | <4% | 0,20% | f: CAACAATGTCGTGTGCATC | f:CAACAATGTC/TGTGTGCATC |
| | | | | | r:GATGCACACGACATTGTTG | r: GATGCACACG/AACATTGTTG |
| | | | | | | **mut:** |
| | | | | | | f:CAACAATGTTGTGTGCATC |

| | **for exon 17:** (Accession: AC005068) | | | | | r: GATGCACACAACATTGTTG |
|---|---|---|---|---|---|---|
| | | | | | | |
| | | | | | | **wt/mut:** |
| **ex.17** | 77811 | 4,17% | <4% | 0,04% | f: GGCTTGAAGATGTAAGAAT | f: GGCTTGAAGA/GTGTAAGAAT |
| | | | | | r:ATTC7TACATCTTCAAGCC | r:ATTCTTACAT/CCTTCAAGCC |
| | | | | | | **mut:** |
| | | | | | | f:GGCTTGAAGGTGTAAGAAT |
| | | | | | | r:ATTCTTACACCTTCAAGCC |

| | **for exon 26:** (Accession: M29445,J05168) | | | | | |
|---|---|---|---|---|---|---|
| | | | | | | **wt/mut:** |
| ex.26 | 137 | 4,17% | <4% | 0,04% | f: GAACATTGCCTATGGAGAC | f:GAACATtGCCC/TTATGGAGA |
| | | | | | r. GTCTCCATAGGCAATGTTC | r: GTCTCCATAG/AGCAATGTT |
| | | | | | | **mut:** |
| | | | | | | f:GAACATTGCTTATGGAGAC |
| | | | | | | r:GTCTCCATAAGCAATGTTC |

**Table 3:**

| PCR fragment with a variation | Position of the variation | specific wildtype primer/ specific mutant primer | common primer | fragment size | PCR condition |
|---|---|---|---|---|---|
| | for exons 1-7: (Accession: AC002457) | | | | |
| | | | | | |
| ex.2 | 141530 | "Pos,-1Gf (wt): 5' GGT,TTC,TCT,TCA,GGT,CGG,G 3' " | "Exon 2r. 5' CTC,AGC,CAA,CAA,ACT,TCT,GC 3' " | 261 bp | A |
| | | "Pos.-1Af (mut): 5' CGG,TTT,CTC,TTC,AGG,TCG,GA 3' " | | | |
| | | | | | |
| ex.2 | 141590 | "Pos.61Ar (wt): 5' GAA,ACA,AGC,TAG,TTA,CCT,TTT,ATT 3' " | "Exon 2f: 5'TCT,TAC,TGC,TCT,CTG,GGC,TTC 3' " | 190 bp | A |
| | | "Pos.61Gr (mut): 5' AAA,CAA,GCT,AGT,TAC,CTT,TTA,TC 3' " | | | |
| | | | | | |
| ex.5 | 171466 | "Pos.-25Gr (wt): 5' GAC,CAC,CAC,AAA,ACA,AAC,ATA,G 3' " | "Exon 5f: 5' CAC,ACA,GTC,AGC,AGA,GAA,GT 3' " | 180 bp | B |
| | | "Pos.-25Tr (mut): 5' GAC,CAC,CAC,AAA,ACA,AAC,ATA,A 3' " | | | |
| | | | | | |
| ex:5 | 171512 | "Pos.308Tr (wt): 5' CTT,CCT,CCA,GAT,TCA,TGA,AGA 3' " | "Exon 5f: 5' CAC,ACA,GTC,AGC,AGA,GAA,GT 3' " | 225 bp | B |
| | | "Pos.308Cr (mut): 5' CTT,CCT,CCA,GAT,TCA,TGA,AGG 3' " | | | |
| ex.6 | 175068 | "Pos.+139Cr (wt):5' AAA,AGG,ATG,CAC,ACG,ACA,TTG 3' " | "Exon 6f: 5' GGA,ATG,AGT,GGT,CTC,TTT,GG 3' " | 428 bp | B |
| | | ''Pos.+139Tr (mut): 5' CAA,AAG,GAT,GCA,CAC,GAC,ATT,A 3' " | | | |
| | | | | | |

| | for exons 11 + 12: (Accession: "M29432,J05168)" | | | | |
|---|---|---|---|---|---|
| | | | | | |
| ex.11 | 101 | "Pos.1199Gf (wt): 5' GAA,TTC,AGA,AAT,GTT,CAC,TTC,AG 3' " | "Exon 11r:5' ACT,AGG,TTT,AAA,TAT,ACA,TGC,AC 3' " | 151 bp | C |
| | | "Pos.1199Af (mut): 5' GAA,TTC,AGA,AAT,GTT,CAC,TTC,AA 3' | | | |
| | | | | | |
| ex.12 | 308 | "Pos:1236Cf (wt): 5' CCT,GGT,AGA,TCT,TGA,AGG,GC 3' " | "Exon 12r: 5' GGG,CAA,CAT,CAG,AAA,GAT,GTG 3' " | 205 bp | D |
| | | "Pos.1236Tf (mut): 5' CCT,GGT,AGA,TCT,TGA,AGG,GT 3' " | | | |
| | | | | | |

| | for exon 17: (Accession: AC005068) | | | | |
|---|---|---|---|---|---|
| | | | | | |
| ex.17 | 78170 | "Pos.-76Tf (wt): 5' AGG,ATA,GGA,TAT,ATT,CCT,TTA,CT 3' " | "Exon 17r: 5' TGG,GCA,TCA,CAC,TTA,CCC,C 3' " | 261 bp | E |
| | | "Pos -76Af (mut): 5' AGG,ATA,GGA,TAT,ATT,CCT,TTA,CA 3'" | | | |
| | | | | | |

| | for exon 26: (Accession: "M29445,J05168)" | | | | |
|---|---|---|---|---|---|
| | | | | | |
| ex.26 | 176 | "Pos.3435Cr (wt): 5' CTC,CTT,TGC,TGC,CCT,CAC,G 3' " "Pos.3435Tr (mut): 5' CTC,CTT,TGC,TGC,CCT,CAC,A 3' " | "Exon 26f: 5' GAT,CTG,TGA,ACT,CTT,GTT,TTC,A 3' " | 195 bp | E |

**Table 4**

| samples | PGP concentration | MDR-1 genotype |
|---|---|---|
| not induced probands | 55 | T-allele present (T/T and T/C) at position 176 in Acc.#M29445/J05168 in exon 26 |
| | 39 | |
| | 276 | |
| | 376 | |
| | | |
| not induced probands, mean | 212 | |
| rifampicine-induced probands | 142 | |
| | 1085 | |
| | 520 | |
| | 601 | |
| | | |
| rifampicine-induced probands, mean | 587 | |
| not induced probands | 96 | T-allele absent (C/C only) at position 176 in Acc.#M29445/J05168 |
| | 302 | |
| | 291 | |
| | | |
| not induced probands, mean | 230 | |
| rifampicine-induced probands | 423 1264 | |
| | 1086 | |
| | | |
| rifampicine-induced probands, mean | 924 | |
| lowest rif-induced activity | 142.1 | homozygous T/T at position 176 in Acc.#M29445/J05168 |
| highest rif-induced activity | 12649 | homozygous C/C at position 176 in Acc.#M29445/J05168 |

**Table 5**

| samples | digoxin concentration in blood | MDR-1 genotype |
|---|---|---|
| not induced probands | 63.6 | T-allele present (T/T and T/C) at position 176 in Acc.#M29445/J05168 |
| | 64.1 | |
| | 73.2 | |
| | 54.7 | |
| | | |
| not induced probands, mean | 63.9 | |
| rifampicine-induced probands | 57.3 | |
| | 39 | |
| | 45.8 | |
| | 37.7 | |
| | | |
| rifampicine-induced probands; mean | 45 | |
| not induced probands | 55.6 | T-allele absent (C/C only) at position 176 in Acc.#M29445/J05168 |
| | 30.8 | |
| | 48.3 | |
| | | |
| not induced probands, mean | 44.9 | |
| rifampicine-induced probands | 39.6 | |
| | 12.3 | |
| | 33.9 | |
| | | |
| rifampicine-induced probands, mean | 28.6 | |
| highest rif-induced dig blood level | 57.3 | homozygous T/T at position 176 in Acc.#M29445/J05168 |
| lowest rif-induced dig blood level | 12.3 | homozygous C/C at position 176 in Acc.#M29445/J05168 |

**Table 8: NEW MDR-1 SNP's**

| **PCR fragment name** | **Position of the variation** | **frequency:** heterozygotes | **frequency:** omozygotes mutant | **frequency:** omozygotes mutant (expected, Hardy-Weinberg) | **wt-sequence** | **wt/mut- and/or mut-sequence** |
|---|---|---|---|---|---|---|
| | | | | | | |
| | **for promoter** **fragments 1-4 and for exons 3-6** (Accession: AC002457) | | | | | |
| | | | | | | |
| | | | | | | **wt/mut:** |
| **promoter fragment 1** | 139015 | 3% * | <1% * | 0.02%* | f: AACTTACTTATATCTTTGA | f: AACTTACTTA/GTATCTTTGA |
| | *: frequencies in Africans-Americans | | | | r: TCAAAGATATAAGTAAGTT | r: TCAAAGATAT/CAAGTAAGTT |
| | | | | | | **mut:** |
| | | | | | | f: AACTTACTTGTATCTTTGA |
| | | | | | | r: TCAAAGATACAAGTAAGTT |
| | | | | | | |
| | | | | | | **wt/mut:** |
| **promoter fragment 1** | 139064 | 1.5% | <1% | 0.01% | f: AGAAATAGTATAATCAACA | f: AGAAATAGTA/TTAATCAACA |
| | | | | | r: TGTTGATTATACTATTTCT | r: TGTTGATTAT/AACTATTTCT |
| | | | | | | **mut:** |
| | | | | | | f: AGAAATAGTTTAATCAACA |
| | | | | | | r: TGTTGATTAAACTATTTCT |
| | | | | | | |
| | | | | | | **wt/mut:** |
| **promoter fragment 1** | 139119 | 24.2% * | 3%* | 2.3%* | f:TAGGGAGGGTTTAAGGCCA | f: TAGGGAGGGT/CTTAAGGCCA |
| | | *: frequencies in Africans-Americans | | | r: TGGCCTTAAACCCTCCCTA | r: TGGCCTTAAA/GCCCTCCCTA |
| | | | | | | **mut:** |
| | | | | | | f: TAGGGAGGGCTTAAGGCCA |
| | | | | | | r:TGGCCTTAAGCCCTCCCTA |
| | | | | | | |
| | | | | | | **wt/mut:** |
| **promoter fragment 1** | 139177 | 1.5% | 1.5% | 0.05% | f: GAAAGGTGAGATAAAGCAA | f: GAAAGGTGAG/AATAAAGCAA |
| | | | | | r:TTGCTTTATCTCACCTTTC | r:TTGCTTTATC/TTCACCTTTC |
| | | | | | | |
| | | | | | | **mut:** |
| | | | | | | f:GAAAGGTGAAATAAAGCAA |
| | | | | | | r:TTGCTTTATTTCACCTTTC |
| | | | | | | |
| | | | | | | **wt/mut:** |
| **promoter fragment 1** | 139276 | 6.7% * | <1% * | 0.1% * | f: CATTTACCCCAGATGGACC | f: CATTTACCCC/TAGATGGACC |
| | | *: frequencies in Africans-Americans | | | r: GGTCCATCTGGGGTAAATG | r: GGTCCATCTG/AGGGTAAATG |
| | | | | | | **mut:** |
| | | | | | | f: CATTTACCCTAGATGGACC |
| | | | | | | r: GGTCCATCTAGGGTAAATG |
| | | | | | | |
| | | | | | | **wt/mut:** |
| **promoter fragment 2** | 139479 | 9.7% | <1% | 0.2% | f: GAGGCGGGCGGATCACGAG | f: GAGGCGGGCG/AGATCACGAG |
| | | | | | r: CTCGTGATCCGCCCGCCTC | r: CTCGTGATCC/TGCCCGCCTC |
| | | | | | | **mut:** |
| | | | | | | f: GAGGCGGGCAGATCACGAG |
| | | | | | | r: CTCGTGATCTGCCCGCCTC |
| | | | | | | |
| | | | | | | **wt/mut:** |
| **promoter fragment 2** | 139619 | 12.1% | <1 | 0.4 | f:GGAGAATGGTGTGAACCCG | f: GGAGAATGGT/CGTGAACCCG |
| | | | | | r: CGGGTTCACACCATTCTCC | r: CGGGTTCACA/GCCATTCTCC |
| | | | | | | **mut:** |
| | | | | | | f: GGAGAATGGCGTGAACCCG |
| | | | | | | r: CGGGTTCACGCCATTCTCC |
| | | | | | | |
| | | | | | | **wt/mut:** |
| **promoter fragment 3** | 140118 | 1.5% | <1% | 0.01% | f: ATATGGAAGGAAATTACAA | f: ATATGGAAGG/AAAATTACAA |
| | | | | | r: TTGTAATTTCCTTCCATAT | r:TTGTAATTTC/TCTTCCATAT |
| | | | | | | **mut:** |
| | | | | | | f:ATATGGAAGAAAATTACAA |
| | | | | | | r:TTGTAATTTTCTTCCATAT |
| | | | | | | |
| | | | | | | **wt/mut:** |
| **promoter fragment 3** | 140216 | 3.1 % * | <1% ***** | 0.03% * | f: AACACGGGCATTGATCTGA | f: AACACGGGCA/GTTGATCTGA |
| | | *: frequencies in Africans-Americans | | | TCAGATCAATGCCCGTGTT | r: TCAGATCAAT/CGCCCGTGTT |
| | | | | | | **mut:** |
| | | | | | | f: AACACGGGCGTTGATCTGA |
| | | | | | | r:TCAGATCAACGCCCGTGTT |
| | | | | | | |
| | | | | | | **wt/mut:** |
| **promoter fragment 4** | 140490 | 5.9%* | <1%* | 0.08%* | f: TGTATTAAATGCGAATCCC | f: TGTATTAAAT/CGCGAATCCC |
| | | *: frequencies in Africans-Americans | | | r: GGGATTCGCATTTAATACA | r: GGGATTCGCA/GTTTAATACA |
| | | | | | | **mut:** |
| | | | | | | f: TGTATTAAACGCGAATCCC |
| | | | | | | r: GGGATTCGCGTTTAATACA |
| | | | | | | |
| | | | | | | **wt/mut:** |
| **promoter fragment 4** | 140568 | 2.9%* | <1%* | 0.02%* | f: TTGAAAGACGTGTCTACAT | f: TTGAAAGACG/ATGTCTACAT |
| | | *: frequencies in Africans-Americans | | | r: ATGTAGACACGTCTTTCAA | r: ATGTAGACAC/TGTCTTTCAA |
| | | | | | | **mut:** |
| | | | | | | f: TTGAAAGACATGTCTACAT |
| | | | | | | r: ATGTAGACATGTCTTTCAA |
| | | | | | | |
| | | | | | | **wt/mut**: |
| **promoter fragment 4** | 140576 | 10.2% * | <1%* | 0.3% * | f: CGTGTCTACATAAGTTGAA | f: CGTGTCTACA/TTAAGTTGAA |
| | | *: frequencies in Africans-Americans | | | r: TTCAACTTATGTAGACACG | r: TTCAACTTAT/AGTAGACACG |
| | | | | | | **mut:** |
| | | | | | | f: CGTGTCTACTTAAGTTGAA |
| | | | | | | r: TTCAACTTAAGTAGACACG |
| | | | | | | |
| | | | | | | **wt/mut:** |
| **promoter fragment 4** | 140595 | 5% * | <1 % * | 0.06% * | f: ATGTCCCCAATGATTCAGC | f: ATGTCCCCAA/GTGATTCAGC |
| | | *: frequencies | in Africans-Americans | | r: GCTGAATCATTGGGGACAT | r. GCTGAATCAT/CTGGGGACAT |
| | | | | | | **mut:** |
| | | | | | | f: ATGTCCCCAGTGATTCAGC |
| | | | | | | r: GCTGAATCACTGGGGACAT |
| | | | | | | |
| | | | | | | **wt/mut:** |
| **promoter fragment 4** | 140727 | 3.1%* | <1%* | 0.03%* | f: CCGGGCCGGGAGCAGTCAT | f: CCGGGCCGGG/AAGCAGTCAT |
| | | *: frequencies in Africans-Americans | | | r: ATGACTGCTCCCGGCCCGG | r: ATGACTGCTC/TCCGGCCCGG |
| | | | | | | **mut:** |
| | | | | | | f: CCGGGCCGGAAGCAGTCAT |
| | | | | | | r: ATGACTGCTTCCGGCCCGG |

| | **for promoter fragments 1-4 and for exons 3-6:** (Accession: AC002457) | | | | | |
|---|---|---|---|---|---|---|
| | | | | | | |
| | | | | | | **wt/mut:** |
| **ex.3** | 145984 | 8.3% | <1 % | 0.2% | f: AAAATACTTCGGAAATTTG | f: AAAATACTTC/TGGAAATTTG |
| | | | | | r:CAAATTTCCGAAGTATTTT | r:CAAATTTCCG/AAAGTATTTT |
| | | | | | | **mut:** |
| | | | | | | f: AAAATACTTTGGAAATTTG |
| | | | | | | CAAATTTCCAAAGTATTTT |
| | | | | | | |
| | | | | | | **wt/mut:** |
| **ex.5** | 171511 | 1.5% | <1% | 0.01% | f: GATACAGGGTTCTTCATGA | f: GATACAGGGT/CTCTTCATGA |
| | | | | | r:TCATGAAGAACCCTGTATC | r:TCATGAAGAA/GCCCTGTATC |
| | | | | | | **mut:** |
| | | | | | | f: GATACAGGGCTCTTCATGA |
| | | | | | | r. TCATGAAGAGCCCTGTATC |
| | | | | | | |
| | | | | | | **wt/mut:** |
| **ex.6** | 174901 | 1.1% | <1% | 0.003% | f: GTGCACGATGTTGGGGAGC | f: GTGCACGATG/ATTGGGGAGC |
| | | | | | r: GCTCCCCAACATCGTGCAC | r: GCTCCCCAAC/TATCGTGCAC |
| | | | | | | **mut:** |
| | | | | | | f: GTGCACGATATTGGGGAGC |
| | | | | | | r:GCTCCCCAATATCGTGCAC |
| | | | | | | |
| | | | | | | **wt/mut:** |
| **ex.6** | 175142 | 16.3% | <1% | 0.7% | f: CATTAAATGAAGGACTGGG | f: CATTAAATGA/GAGGACTGGG |
| | | | | | r. CCCAGTCCTTCATTTAATG | r. CCCAGTCCTT/CCATTTAATG |
| | | | | | | **mut:** |
| | | | | | | f: CATTAAATGGAGGACTGGG |
| | | | | | | r: CCCAGTCCTCCATTTAATG |
| | | | | | | |
| | | | | | | **wt/mut:** |
| **ex.6** | 175180 | 46.9% | 20.3% | 19.4% | f: TCCTCTGAGAATGTGCAGT | f:TCCTCTGAGA/GATGTGCAGT |
| | | | | | r: ACTGCACATTCTCAGAGGA | r: ACTGCACATT/CCTCAGAGGA |
| | | | | | | **mut**: |
| | | | | | | f:TCCTCTGAGGATGTGCAGT |
| | | | | | | r: ACTGCACATCCTCAGAGGA |

| | **for exons 10-26:** (Accession: AC005068) | | | | | |
|---|---|---|---|---|---|---|
| | | | | | | |
| | | | | | | **wt/mut:** |
| **ex.10** | 84701 | 45.8% | 25% | 23% | t: AAAATTGCTGTCACTATCT | f: AAAATTGCTG/ATCACTATCT |
| | | | | | r: AGATAGTGACAGCAATTTT | r: AGATAGTGAC/TAGCAATTTT |
| | | | | | | **mut:** |
| | | | | | | f:AAAATTGCTATCACTATCT |
| | | | | | | r:AGATAGTGATAGCAATTTT |
| | | | | | | |
| | | | | | | **wt/mut:** |
| **ex.12** | 84032 | 0.5% | <1% | 0.001% | f:GAGCACAACAGTCCAGCTG | f:GAGCACAACA/GGTCCAGCTG. |
| | | | | | r:CAGCTGGACTGTTGTGCTC | r:CAGCTGGACT/CGTTGTGCTC. |
| | | | | | | **mut:** |
| | | | | | | f:GAGCACAACGGTCCAGCTG |
| | | | | | | r:CAGCTGGACCGTTGTGCTC |
| | | | | | | |
| | | | | | | **wt/mut:** |
| **ex.12** | 84074 | 3.4% * | <1%* * | 0.03% * | f:TGGGCAGACGGTGGCCCTG | f:TGGGCAGACG/AGTGGCCCTG |
| | | *: frequencies in Africans-Americans | | | r. CAGGGCCACCGTCTGCCCA | r. CAGGGCCACC/TGTCTGCCCA |
| | | | | | | **mut:** |
| | | | | | | f:TGGGCAGACAGTGGCCCTG |
| | | | | | | r:CAGGGCCACTGTCTGCCCA |
| | | | | | | |
| | | | | | | **wt/mut:** |
| **ex.12** | 84119 | 3.4% * | <1%* | 0.03% * | f: CTCGTCCTGGTAGATCTTG | f: CTCGTCCTGG/ATAGATCTTG |
| | | *: frequencies in Africans-Americans | | | r: CAAGATCTACCAGGACGAG | r: CAAGATCTAC/TCAGGACGAG |
| | | | | | | **mut:** |
| | | | | | | f:CTCGTCCTGATAGATCTTG |
| | | | | | | r:CAAGATCTATCAGGACGAG |
| | | | | | | |
| | | | | | | **wt/mut**: |
| **ex.12** | 83973 | 3.4% * | <1% *. | 0.03% * | f:GACCCATGCGAGCTAGACC | f:GACCCATGCG/AAGCTAGACC |
| | | *: frequencies in Africans-Americans | | | r:GGTCTAGCTCGCATGGGTC | r:GGTCTAGCTC/TGCATGGGTC |
| | | | | | | **mut:** |
| | | | | | | f:GACCCATGCAAGCTAGACC |
| | | | | | | r:GGTCTAGCTTGCATGGGTC |
| | | | | | | |
| | | | | | | **wt/mut:** |
| ex.19 | 73252 | 8.3% | <1% | 0.2% | f: ACMGTCTAATCTCCTGC | f: ACTTTGTCTA/GATCTCCTGC |
| | | | | | r: GCAGGAGATTAGACAAAGT. | r: GCAGGAGATT/CAGACAAAGT |
| | | | | | | mut: |
| | | | | | | f: ACTTTGTCTGATCTCCTGC |
| | | | | | | r: GCAGGAGATCAGACAAAGT |
| | | | | | | |
| | | | | | | wt/mut: |
| ex.20 | 70371 | 3.8% * | <1%* | 0.04%* | f: AATCATTTTCTGTGCCACA | f: AATCATTTTC/ATGTGCCACA |
| | | *: frequencies in Africans-Americans | | | r: TGTGGCACAGAAAATGATT | r: TGTGGCACAG/TAAAATGATT |
| | | | | | | mut: |
| | | | | | | f: AATCATTTTATGTGCCACA |
| | | | | | | r:TGTGGCACATAAAATGATT |
| | | | | | | wt/mut: |
| | | | | | | |
| ex.20 . | 70253 | 22.2% | <1 % | 1.2% | f:TCTACTGGTGTTTGTCTTA | f: TCTACTGGTG/ATTTGTCTTA |
| | | | | | r:TAAGACAAACACCAGTAGA | r:TAAGACAAAC/TACCAGTAGA |
| | | | | | | mut: |
| | | | | | | f:TCTACTGGTATTTGTCTTA |
| | | | | | | r: TAAGACAAATACCAGTAGA |
| | | | | | | |
| | | | | | | wt/mut: |
| ex.20 | 70237 | 16.7% | <1% | 0.6% | f:TTAATIGGCCATTTTGGAC | f: TTAATTGGCC/TATTTTGGAC |
| | | | | | r: GTCCAAAATGGCCAATTAA | r: GTCCAAAATG/AGCCAATTAA |
| | | | | | | **mut:** |
| | | | | | | f: TTAATTGGCTATTTTGGAC |
| | | | | | | r: GTCCAAAATAGCCAATTAA |
| | | | | | | |
| | | | | | | **wt/mut:** |
| ex.20 | 70204 | 16.7% | <1% | 0.6% | f: AATTTTCTCCTTACGGGTG | f: AATTTTCTCC/ATTACGGGTG |
| | | | | | r: CACCCGTAAGGAGAAAATT | r: CACCCGTAAG/TGAGAAAATT |
| | | | | | | **mut:** |
| | | | | | | f: AATTTTCTCATTACGGGTG |
| | | | | | | r: CACCCGTAATGAGAAAATT |
| | | | | | | |
| | | | | | | **wt/mut:** |
| **ex.20** | 70200 | 4.2% | <1% | 0.04% | f: TTCTCCTTACGGGTGTTAG | f: TTCTCCTTAC/TGGGTGTTAG |
| | | | | | r:CTAACACCCGTAAGGAGAA | r:CTAACACCCG/ATAAGGAGAA |
| | | | | | | **mut:** |
| | | | | | | f: TTCTCCTTATGGGTGTTAG |
| | | | | | | r: CTAACACCCATAAGGAGAA |
| | | | | | | |
| | | | | | | **wt/mut:** |
| **ex.26** | 43263 | 0.5% | <1% | 0.001% | f: TGAATGTTCAGTGGCTCCG | f:TGAATGTTCA/CGTGGCTCCG |
| | | | | | r:CGGAGCCACTGAACATTCA | r:CGGAGCCACT/GGAACATTCA |
| | | | | | | **mut:** |
| | | | | | | f:TGAATGTTCCGTGGCTCCG |
| | | | | | | r:CGGAGCCACGGAACATTCA |
| | | | | | | |
| | | | | | | **wt/mut:** |
| **ex.26** | 43162 | 0.5% | <1% | 0.001% | f: CGGGTGGTGTCACAGGAAG | f:CGGGTGGTGT/ACACAGGAAG |
| | | | | | r:CTTCCTGTGACACCACCCG | r:CTTCCTGTGA/TCACCACCCG |
| | | | | | | **mut:** |
| | | | | | | f:CGGGTGGTGAGACAGGAAG |
| | | | | | | r:CTTCCTGTGTCACCACCCG |

| | | | | | | |
|---|---|---|---|---|---|---|
| Legend for table 8: New MDR-1 SNP's MDR1 variations and the respective wildtype and mutant alleles: The expected frequency for homozygotes for the mutant allele were calculated on the basis of the Hardy-Weinberg distribution. In the sequence section the deviant bases are underlined and in a bold style. | | | | | | |

## Claims

1. A method of diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c);
wherein the presence of said polynucleotide is considered indicative of said acquired multidrug resistance or sensitivity in said method.

2. The method of claim 1, wherein the nucleotide substitution results in altered expression of the variant MDR-1 gene compared to the corresponding wild type gene.

3. The method of claim 1 or 2, wherein said acquired multidrug resistance is in tumors and other diseases, the therapy of which is dependent on drug treatment.

4. A method of diagnosing a disorder related to the presence of a molecular variant of the MDR-1 gene or susceptibility to such a disorder, comprising determining the presence of a polynucleotide as defined in claim 1 in a sample from a subject.

5. The method of claim 4, wherein said disorder is cancer.

6. The method of any one of claims 1 to 5, comprising PCR, ligase chain reaction, restriction digestion, direct sequencing, nucleic acid amplification techniques, hybridization techniques or immunoassays.

7. In vitro use of an oligo- or polynucleotide for diagnosing acquired multidrug resistance or sensitivity related to the presence of a molecular variant of the MDR-1 gene comprising determining in a sample from a subject the presence of a polynucleotide selected from the group consisting of:
(a) a polynucleotide having the nucleic acid sequence of SEQ ID NO 122;
(b) a polynucleotide encoding a molecular variant Multi Drug Resistance (MDR)-1 polypeptide, wherein said polynucleotide is having at a position corresponding to position 176 of the MDR-1 gene (Accession No: M29445 or J05168) a nucleotide exchange;
(c) a polynucleotide encoding a molecular variant MDR-1 polypeptide, wherein said polynucleotide is having at a position corresponding to position 176 of the MDR-1 gene (Accession No: M29445 or J05168) a T; and
(d) a nucleic acid molecule complementary to the polynucleotide of (a) to (c); Wherein said oligo-or polynucleotide is suitable for said determination and
wherein the presence of said polynucleotide is considered indicative of said acquired multidrug resistance or sensitivity in said use.

8. A diagnostic composition comprising the polynucleotide as defined in claim 1 or the oligo- or polynucleotide of claim 7.

9. Use of the MDR-1 gene single nucleotide polymorphism C3435T in exon 26 as defined in claim 1(a) or (c) as a pharmacogenetic factor for the preparation of a diagnostic composition for the prediction of blood levels of a MDR-1 substrate and/or inducer for improvement of drug safety and efficacy, to predict and prevent side effects and drug interactions and/or to increase patient compliance.

10. The use of claim 9, wherein the substrate and/or inducer are selected from anticonvulsant/antiepileptic drugs, cardiac glycosides, immunosuppressive drugs, macrolid-antibiotics, or macrocyclic-antibiotics.

11. A method of identifying and obtaining an MDR-1 inhibitor capable of modulating the activity of a molecular variant of the MDR-1 gene comprising the steps of
(a) contacting a cell expressing a molecular variant MDR-1 gene comprising a polynucleotide as defined in claim 1 in the presence of components capable of providing a detectable signal in response to drug transport, with a compound to be screened under conditions to permit MDR-1 mediated drug transport, and
(b) detecting the presence or absence of a signal or increase of a signal generated from the drug transport, wherein the presence or increase of the signal is indicative for a putative inhibitor.

## Patentansprüche

1. Verfahren zur Diagnose einer erworbenen Multidrug-Resistenz oder -Sensitivität, die in Beziehung steht zu dem Vorhandensein einer molekularen Variante des MDR-1-Gens, umfassend die Bestimmung des Vorhandenseins eines Polynucleotids in einer Probe einer Testperson, wobei das Polynucleotid ausgewählt ist aus der Gruppe bestehend aus:
(a) einem Polynucleotid mit der Nucleinsäuresequenz von SEQ ID NO: 122;
(b) einem Polynucleotid, das eine molekulare Variante des Multidrug-Resistenz (MDR)-1-Polypeptids codiert, wobei das Polynucleotid einen Nucleotidaustausch an einer Position aufweist, die der Position 176 des MDR-1-Gens (Zugangsnr. M29445 oder J05168) entspricht;
(c) einem Polynucleotid, das eine molekulare Variante des MDR-1-Polypeptids codiert, wobei das Polynucleotid an einer Position ein T aufweist, die der Position 176 des MDR-1-Gens (Zugangsnr. M29445 oder J05168) entspricht; und
(d) einem Nucleinsäuremolekül, das komplementär zu dem Polynucleotid nach (a) bis (c) ist;
wobei das Vorhandensein des Polynucleotids als Hinweis auf die erworbene Multidrug-Resistenz oder -Sensitivität in diesem Verfahren angesehen wird.

2. Verfahren nach Anspruch 1, wobei die Nucleotidsubstitution zu einer geänderten Expression des varianten MDR-1-Gens im Vergleich zum entsprechenden Wildtyp-Gen führt.

3. Verfahren nach Anspruch 1 oder 2, wobei die erworbene Multidrug-Resistenz in Tumoren und anderen Krankheiten vorhanden ist, deren Therapie von einer Wirkstoffbehandlung abhängig ist.

4. Verfahren zur Diagnose einer Krankheit im Zusammenhang mit dem Vorhandensein einer molekularen Variante des MDR-1-Gens oder einer Anfälligkeit für eine solche Krankheit, umfassend die Bestimmung des Anfälligkeit für eine solche Krankheit, umfassend die Bestimmung des Vorhandenseins eines wie in Anspruch 1 definierten Polynucleotids in einer Probe einer Testperson.

5. Verfahren nach Anspruch 4, wobei die Krankheit Krebs ist.

6. Verfahren nach einem der Ansprüche 1 bis 5, umfassend PCR, Ligase-Kettenreaktion, Restriktionsverdau, direktes Sequenzieren, Nucleinsäureamplifikationstechniken, Hybridisierungstechniken oder immunoassays.

7. In vitro-Verwendung eines Oligo- oder Polynucleotids zur Diagnose erworbener Multidrug-Resistenz oder -Sensitivität, die im Bezug steht mit dem Vorhandensein einer molekularen Variante des MDR-1-Gens, umfassend die Bestimmung des Vorhandenseins eines Polynucleotids in einer Probe einer Testperson, wobei das Polynucleotid ausgewählt ist aus der Gruppe bestehend aus.
(a) einem Polynucleotid mit der Nucleinsäuresequenz von SEQ ID NO: 122;
(b) einem Polynucleotid, das eine molekulare Variante des Multidrug (MDR)-1-Polypeptids codiert, wobei das Polynucleotid einen Nucleotidaustausch an einer Position aufweist, die der Position 176 des MDR-1-Gens (Zugangsnr. M29445 oder J05168) entspricht;
(c) einem Polynucleotid das eine molekulare Variante des MDR-1-Polypeptids codiert, wobei das Polynucleotid an einer Position ein T aufweist, die der Position 176 des MDR-1-Gens (Zugangsnr. M29445 oder J05168) entspricht; und
(d) einem Nucleinsäuremolekül, das komplementär zu dem Polynucleotid nach (a) bis (c) ist;
wobei das Oligo- oder Polynucleotid für die Bestimmung geeignet ist, und wobei das Vorhandensein des Polynucleotids als Hinweis auf die erworbene Multidrug-Resistenz oder -Sensitivität in diesem Verfahren angesehen wird.

8. Diagnostisches Mittel, umfassend das wie in Anspruch 1 definierte Polynucleotid oder das Oligo- oder Polynucleotid nach Anspruch 7.

9. Verwendung des MDR-1-Gen-Einzelnucleotid-Polymorphismus C3435T in Exon 26, der wie in Anspruch 1(a) oder (c) definiert ist, als pharmakogenetischen Faktor zur Herstellung eines diagnostischen Mittels für die Vorhersage von Blutspiegeln eines MDR-1-Substrats und/oder -Induktors zur Verbessung von Wirkstoffsicherheit und -Effizienz, um Nebenwirkungen und Wirkstoffinteraktionen vorherzusagen und abzuwenden und/oder um Patienten-compliance zu erhöhen.

10. Verwendung nach Anspruch 9, wobei das Substrat und/oder der Induktor ausgewählt ist/sind aus krampflösenden/antiepileptischen Mitteln, Herzglykosiden, immunsuppressiven Wirkstoffen, Macrolidantibiotika oder macrocyclischen Antibiotika.

11. Verfahren zum Auffinden und Erhalten eines MDR-1-Inhibitors, der die Aktivität einer molekularen Variante des MDR-1-Gens modulieren kann, umfassend die Schritte:
(a) des Inkontaktbringens einer Zelle, die eine molekulare Variante des MDR-1-Gens exprimiert, das ein wie in Anspruch 1 definiertes Polynucleotid umfasst, in der Anwesenheit von Stoffen, die ein nachweisbares Signal als Antwort auf Wirkstofftransport liefern können, mit einer zu durchmusternden Verbindung unter Bedingungen, die MDR-1-vermittelten Wirkstofftransport erlauben, und
(b) des Nachweisens der Anwesenheit oder Abwesenheit eines Signals oder einer Zunahme eines Signals, das von dem Wirkstofftransport erzeugt wurde, wobei die Anwesenheit oder Zunahme des Signals auf einen möglichen Inhibitor hinweist.

## Revendications

1. Méthode pour diagnostiquer une résistance ou une sensibilité multi-drogues acquise liée à la présence d'un variant moléculaire du gène MDR-1 comprenant la détermination dans un échantillon du sujet de la présence d'un polynucléotide sélectionné parmi le groupe consistant en :
(a) un polynucléotide ayant la séquence d'acide nucléique de la SEQ ID No 122 ;
(b) un polynucléotide codant un polypeptide variant moléculaire de Résistance Multi-Drogues (MRD)-1, ledit polynucléotide présentant un échange de nucléotide à une position correspondant à la position 176 du gène MDR-1 (N° d'Accession : M29445 ou J05168) ;
(c) un polynucléotide codant un polypeptide variant moléculaire de MDR-1, ledit polynucléotide ayant un T à une position correspondant à la position 176 du gène MDR-1 (N° d'Accession : M29445 ou J05168) ;
(d) une molécule d'acide nucléique complémentaire à un polynucléotide de (a) à (c) ;
dans laquelle la présence dudit polynucléotide est considérée comme indicative de ladite résistance ou sensibilité multi-drogues acquise dans ladite méthode.

2. Méthode selon la revendication 1, dans laquelle la substitution du nucléotide résulte en une expression altérée du gène MDR-1 variant comparée au gène correspondant sauvage.

3. Méthode selon la revendication 1 ou 2, dans laquelle ladite résistance multi-drogues acquise est dans des tumeurs et d'autres maladies, dont la thérapie est dépendante du traitement par la drogue.

4. Méthode pour diagnostiquer un trouble lié à la présence d'un variant moléculaire du gène MDR-1 ou une susceptibilité à un tel trouble, comprenant la détermination de la présence d'un polynucléotide tel que défini dans la revendication 1 dans un échantillon d'un sujet.

5. Méthode selon la revendication 4, dans laquelle ledit trouble est le cancer.

6. Méthode selon l'une quelconque des revendications 1 à 5, comprenant une PCR, une réaction de ligation en chaine, une digestion par des enzymes de restriction, le séquençage direct, des techniques d'amplification d'acide nucléique, des techniques d'hybridation, ou des immunoessais.

7. Utilisation *in vitro* d'un oligo- ou polynucléotide pour diagnostiquer une résistance ou une sensibilité multi-drogues acquise liée à la présence d'un variant moléculaire du gène MDR-1 comprenant la détermination dans un échantillon du sujet de la présence d'un polynucléotide sélectionné parmi le groupe consistant en :
(a) un polynucléotide ayant la séquence d'acide nucléique de la SEQ ID No 122 ;
(b) un polynucléotide codant un polypeptide variant moléculaire de Résistance Multi-Drogues (MRD)-1, ledit polynucléotide présentant un échange de nucléotide à une position correspondant à la position 176 du gène MDR-1 (N° d'Accession : M29445 ou J05168) ;
(c) un polynucléotide codant un polypeptide variant moléculaire de MDR-1, ledit polynucléotide ayant un T à une position correspondant à la position 176 du gène MDR-1 (N° d'Accession : M29445 ou J05168) ;
(d) une molécule d'acide nucléique complémentaire à un polynucléotide de (a) à (c) ;
dans laquelle ledit oligo- ou polynucléotide est adapté pour ladite détermination et
dans laquelle la présence dudit polynucléotide est considérée comme indicative de ladite résistance ou sensibilité multi-drogues acquise dans ladite méthode.

8. Composition diagnostique comprenant le polynucléotide tel que défini dans la revendication 1 ou l'oligo- ou polynucléotide de la revendication 7.

9. Utilisation du polymorphisme nucléotidique (SNP) du gène MDR-1 C3435T dans l'exon 26 tel que défini dans la revendication 1(a) ou (c) comme facteur pharmacogénétique pour la préparation d'une composition diagnostique pour la prédiction des niveaux sanguins d'un substrat et/ou inducteur de MDR-1 pour l'amélioration de l'innocuité et de l'efficacité d'un médicament, pour prédire et prévenir les effets secondaires et les interactions entre médicaments et/ou pour améliorer le respect du traitement.

10. Utilisation selon la revendication 9, dans laquelle le substrat et/ou inducteur est sélectionné parmi les drogues anticonvulsivantes/antiépileptiques, des glucosides cardiaques, des drogues immunosuppressives, les antibiotiques macrolides ou les antibiotiques macrocycliques.

11. Méthode pour identifier et obtenir un inhibiteur de MDR-1 capable de moduler l'activité d'un variant moléculaire du gène MDR-1 comprenant les étapes consistant à :
(a) mettre en contact une cellule exprimant un variant moléculaire du gène MDR-1 comprenant un polynucléotide tel que défini dans la revendication 1 en présence de composants capable de fournir un signal détectable en réponse au transport d'une drogue, avec un composé à cribler dans des conditions permettant le transport de drogue médié par MDR-1 ; et
(b) détecter la présence ou l'absence d'un signal ou l'augmentation du signal généré par le transport de la drogue, dans laquelle la présence ou l'augmentation du signal est indicative d'un inhibiteur potentiel.
